(19)

**Europäisches Patentamt**
**European Patent Office**
**Office européen des brevets**

(11) **EP 4 775 665 A2**

(12) **EUROPEAN PATENT APPLICATION**

(43) Date of publication:
**15.07.2026 Bulletin 2026/29**

(51) International Patent Classification (IPC):
**C12N 3/00** *(2006.01)*

(21) Application number: **26165288.7**

(22) Date of filing: **18.12.2020**

(52) Cooperative Patent Classification (CPC):
**C12N 1/36; A61K 35/74; C07K 14/33; C12N 3/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2019 EP 19217672**
**18.12.2019 US 201962949480 P**

(62) Document number(s) of the earlier application(s) in accordance with Art. 76 EPC:
**20848793.4 / 4 077 359**

(71) Applicant: **LivingMed Biotech S.R.L.**
**4000 Liège (BE)**

(72) Inventor: **KUBIAK, Aleksandra Maria**
**6226DR Maastricht (NL)**

(74) Representative: **Grund, Martin et al**
**Grund Intellectual Property Group**
**Patentanwälte und Solicitor PartG mbB**
**Steinsdorfstraße 2**
**80538 München (DE)**

Remarks:
•The complete document including Reference Table(s) and the Sequence Listing(s) can be downloaded from the EPO website
•This application was filed on 17.03.2026 as a divisional application to the application mentioned under INID code 62.
•Claims filed after the date of filing of the application/ after the date of receipt of the divisional application (Rule 68(4) EPC).

(54) **GENETICALLY MODIFIED CLOSTRIDIUM STRAINS AND USES THEREOF**

(57) The present invention relates to genetically modified *Clostridia* strains that provide an improved combination of replication and physiological characteristics useful for a variety of clinical and industrial applications. In particular, clinical grade spores generated from the *Clostridia* strains described herein can be efficiently stored, formulated, and advantageously used in the prevention and/or treatment of medical conditions, such as cancer.

FIG. 1

Selecting an appropriate clostridium strain (CLOSTRA)

Modifying CLOSTRA by modifying and/or deleting genetic element(s) controlling sporulation gene(s) (obtaining CLOSTRA-S)

Modifying CLOSTRA by including and/or deleting genetic element(s) functional for medical or industrial uses (obtaining CLOSTRA-T, CLOSTRA-A, or CLOSTRA-AT)

Culturing, selecting, and optimizing CLOSTRA that combine genetic and functional features that have been modified in CLOSTRA-T, CLOSTRA-A, or CLOSTRA-AT with those that have been modified in CLOSTRA-S (obtaining CLOSTRA-ST, CLOSTRA-SA, or CLOSTRA-SAT)

Producing corresponding spore preparations (CLOSTRA-STS, CLOSTRA-SAS, OR CLOSTRA-SATS)

Formulating corresponding spore (or cell) preparations for industrial or medical uses (CLOSTRA-STF, CLOSTRA-SAF, OR CLOSTRA-SATF)

EP 4 775 665 A2

**Description**

**FIELD OF THE INVENTION**

The present invention relates to novel *Clostridium* strains and spores, their method of manufacture, and uses in various industrial and clinical applications.

**BACKGROUND OF THE INVENTION**

Clostridia are Gram-positive, anaerobic, endospore-forming bacteria that are grouped under the genus *Clostridium,* comprising approximately 180 species known to produce different types of acids and other chemical compounds through the degradation of sugars, alcohols, amino acids, purines, pyrimidines, and polymers, such as starch and cellulose. The sporulation process in *Clostridia* for survival and reproduction purposes commonly follows exposure to unfavorable conditions in the local environment (such as pH, heating, freezing, radiation, or oxygen conditions). This process is controlled by a complex cascade of sporulation-specific proteins and other factors acting at the transcriptional or posttranslational level. For example, *spo0A, spoIIAA, spoIIE, sigH, sigF, sigE, sigG,* and *sigK* genes are those mainly responsible for organizing and modulating the expression of spore morphogenetic components, generating different complex phenotypes (Shen S et al., 2019; Al-Hinai M et *al.,* 2015).

The replication and other biological properties of the species making up the Clostridia genus have been studied for various objectives, which can be categorized in three main areas: control of pathogenic *Clostridium* infections, exploitation as platform organisms for industrial use (in particular for biotransformation and for producing chemical commodities), and exploiting the cytotoxic properties of *Clostridium* species against undesirable cells and cellular events (for instance, inhibition of cancer cell growth and an induction of a response against cancer cells). On these fronts, several technologies have emerged, or have been adapted from other organisms, for the purposes of culturing, administering, selecting, or using Clostridia, in particular by modifying a *Clostridium* genome with regulatory or coding sequences that have been modified, cloned, added, disrupted, and/or placed under specific systems for the control of gene expression.

As recently reviewed (Joseph R *et al.,* 2018; McAllister KN, and Sorg JA, 2019; Kwon SW *et al.,* 2020), a series of improvements have emerged for engineering and selecting strains from the *Clostridium* species featuring a permanently, efficiently, and precisely modified genome, thereby permitting a better understanding of *Clostridium* sporulation and metabolic processes *in vitro,* but also for improving their uses, as cells or as spores. Mechanisms that exploit the natural homologous recombination pathways present in the *Clostridium* species have been applied with uneven success and efficacy. Allelic exchange systems, CRISPR-based genetic editing and gene expression control in *Clostridium* has led to the possibility of modifying this type of genome and then selecting those clones or variants most suitable for a particular contemplated use.

Several approaches have been developed, by making use of constructs providing *Clostridium* strains featuring a single functional, highly accurate genome modification(s) (including nucleotide substitution, gene deletion and cassette insertion) directed to biological or physiological features such as sporulation, additional or enhanced enzymatic activity, expression of codon-optimized, non-Clostridial genes, expression of a Clostridia gene under the control of external inducers, resistance to chemical compounds or conditions, virulence control, or basic cell labeling (Atmadjaja A *et al.* 2019; Banjeree A *et al.,* 2014; Bruder M et al,. 2016; Ingle P et *al.,* 2019; Canadas I *et al.,* 2019; Dembek M *et al.,* 2017Diallo M *et al.,* 2019; Dong H *et al.,* 2012; Ehsaan M et al, 2016; Foulquier C *et al.,* 2019; Huang H *et al.,* 2016; Pyne M *et al.,* 2016; Wang S *et al.,* 2018; Wasels F *et al.,* 2017; Wen Z *et al*., 2017; WO2003/074681; WO2017/064439; WO2010/005722; WO2015/075475; WO2017/190257).

The combination of cloned sequences and *Clostridium-specific* genetic modifications have mostly succeeded in generating single, specifically defective or repressed genes in a laboratory setting. The use of Clostridia in more demanding applications, such as in industrial and clinical development programs has been much more restrictive, since improved functional properties must necessarily be associated with both strict biocontainment and a tight control of gene expression and replication *in vivo.* For instance, since *Clostridium* species are known to have the capacity to specifically accumulate in the human body in hypoxic or anoxic environments, such as those present within solid tumors, the clinical use of *C*lostridia-based cancer therapies has been proposed and tested in various models as a means to elicit specific immunological responses, target cancer cells, deliver cytokines, and/or activate prodrugs in Clostridia-infected tumors (Ni G *et al.,* 2019; Torres W *et al*., 2018; Heap *J et al.,* 2014; Zhang YL *et al.* 2014 Mowday A *et al.* 2016; Kubiak AM and Minton NP, 2015; WO2007/149433; WO2016/095503; WO2009/111177; WO2003/045153; WO2013/159155).

*Clostridium*-based cancer therapies have been tested alone or in combination with other drugs, including those applications targeting the tumor microenvironment and cancer-specific immune responses (Agrawal S and Kandimalla E, 2019; Zhong S *et al.,* 2019; Xin Y *et al.,* 2019). However, these approaches have not yet been successfully translated into clinical practices, at least because the pre-clinical and clinical characterization of *Clostridium* spore-based formulations still requires substantial improvement for adequately exploiting and controlling their activities, including managing replication control, especially when existing as spores, specific localization upon administration, and significantly, interaction with cells present in the human organism, such that beneficial therapeutic strategies can be developed and successfully applied.

## SUMMARY OF THE INVENTION

The present invention relates to spore-forming *Clostridium* strains resulting from a combined approach of both targeting and modifying sequences in the *Clostridium* genome using non-Clostridial gene sequences. Genetically modified Clostridium strains can then be selected and advantageously used to prepare cell or spore preparations offering improved industrial and clinical utility associated with Clostridial gene replication control both in vivo and in vitro, as well as the use of such strains with, for example, suitable compounds and treatments. The approaches disclosed herein further allow establishing improved clinical uses of Clostridia cells and spores as a drug, including as a cancer therapeutic agent, not only in a monotherapy context, but also in optimized combination therapies using conventional treatment protocols (targeting, or not, the same tumors or other tissues where Clostridial cells or spores are generally administered and/or biologically active and replicating), using specific dosage regimens and/or in subpopulations of patients that would benefit from a combination therapy involving two or more therapeutic agents.

In one embodiment, the novel *Clostridium* strains present both an inducible or repressible sporulation phenotype that is combined with the expression of a heterologous gene and/or the inactivation (or at least the attenuation) of at least an additional *Clostridium* gene, in particular a gene unrelated to *Clostridium* replication but whose expression negatively affects the use of the *Clostridium* strain for industrial or clinical applications, such as undesired cytotoxic effects or metabolic use of specific nutrients for growth and replication. In particular, the inducible or repressible sporulation phenotype results from the deletion, substitution, or other genetic modification of the sequence that controls the expression of the one or more Clostridial genes involved in the sporulation process. The choice of the sporulation gene to be targeted is made preferably from among those genes that are at an early stage of this process, such as at the initiation of sporulation up to the establishing of an asymmetric septum (in some cases before the establishing of the prespore), but having a limited effect over normal strain replication and metabolism, in accordance with the literature (Shen S *et al.,* 2019; Al-Hinai M *et al.,* 2015).

Preferably, these novel *Clostridium* strains present both an inducible sporulation phenotype and the inactivation (or at least the attenuation) of at least an additional *Clostridium* gene, whereby such strains can be further modified by the introduction and the basal or inducible expression of a heterologous gene, within the location of a specifically inactivated *Clostridium* gene or any other location in a Clostridial genome. In particular, the heterologous gene may be isolated from the mammalian, preferably human, plant, or non-Clostridial bacterial genome and codes, modified or unmodified, for a protein having enzymatic activities, antigenic properties, antigen-binding properties, hormonal properties, or other observable effect on general human cellular or physiological functions such as signaling, replication, metabolism, immune response, cytotoxicity, necrosis, apoptosis, or differentiation.

The resulting *Clostridium* strains disclosed herein can be used for producing purified and/or concentrated cell preparations (or corresponding spore preparations) to be stored or used directly in a clinical context, for instance, applied at an effective therapeutic dose to a patient in need thereof. In particular, where the final use of the preparation is in connection with additional requirements, such preparation may be modified appropriately to produce the corresponding batch or ready-to-use formulations and may further comprise, for example, supplementary biological or chemical components, such as drugs, additives, salts, or excipients.

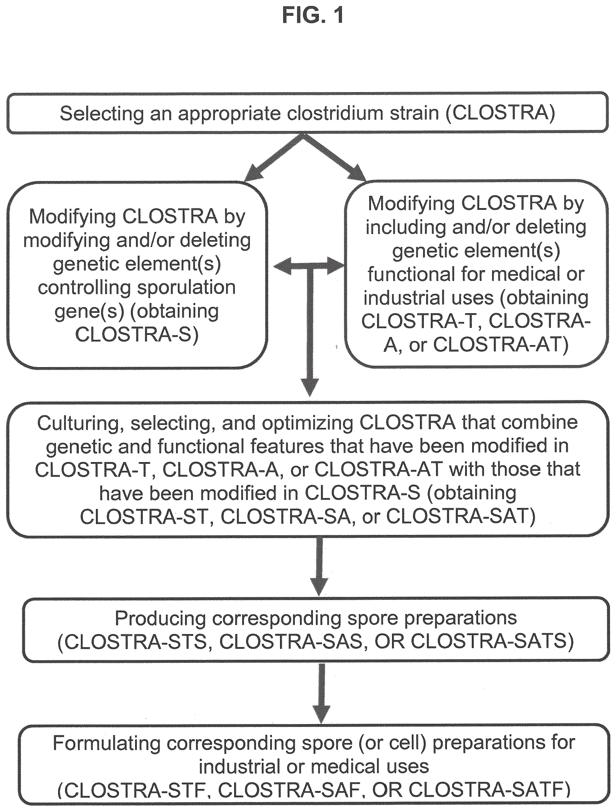

A general schematic representation of a strategy for generating the presently disclosed genetically modified *Clostridium* strains is shown in FIG. 1, including the nomenclature of various final or intermediate *Clostridium* strains originated from a commercially or otherwise available *Clostridium* strain (indicated as CLOSTRA) that is used throughout the instant disclosure. CLOSTRA is preferably any *Clostridium* species belonging to the *Clostridium* genus that presents sporulation and germination control in organisms, including *C. (Clostridium) butyricum, C. sporogenes, C. novyi, C. botulinum, C. difficile,* and other species known for their properties of clinical or industrial interest. In particular, a novel *Clostridium* strain that presents an inducible or repressible sporulation phenotype is identified herein as CLOSTRA-S. Such genetically modified sequence that controls the expression of one or more *Clostridium* genes involved in sporulation can be induced or repressed by a compound to which the vegetative Clostridia cells are exposed, preferably in cell culture conditions. A novel *Clostridium* strain that presents an inactivated (or at least attenuated) *Clostridium* gene is identified as CLOSTRA-A. A novel *Clostridium* strain that expresses a biologically functional, heterologous gene is identified herein as CLOSTRA-T. A particular CLOSTRA-S, CLOSTRA-A, and CLOSTRA-T strain may identify a strain where two or more modifications in the Clostridila genomes may be combined to obtain the desired properties, for instance, a CLOSTRA-A strain in which two independent, non-sporulation genes are inactivated, or at least attenuated, through the deletion of a Clostridial genomic sequence, with or without integrating an exogenous marker or functional sequences at this genomic location, providing non-toxigenic CLOSTRA-Derived Strains and CLOSTRA-Derived Products suitable for clinical or industrial applications.

Each of such *Clostridium* strains containing one or more of the genetic modifications disclosed above, can be further modified to include additional modification(s), thereby establishing additional genetically modified strains that present any of the preferred combination of phenotypes herein. These further strains are identified by combinations of the nomenclature disclosed above, for example, CLOSTRA-AT, CLOSTRA-SA, CLOSTRA-ST, and CLOSTRA-SAT. In particular, a CLOSTRA-SA or CLOSTRA-A2, as described in the Examples, wherein at least two endogenous Clostridial genes are modified, can be used as a reference "platform" strain that can be further optimized for a given industrial or clinical use by introducing one or more heterologous genes, thus generating a series of alternative, numbered CLOSTRA-SAT strains having specific properties and uses. Each of the CLOSTRA-AT, CLOSTRA-SA, CLOSTRA-ST, and CLOSTRA-SAT strains can be stored and used as such or as the corresponding spores (identified by the names CLOSTRA-ATS, CLOSTRA-SAS, CLOSTRA-STS, and CLOSTRA-SATS) and formulations of cells or spores (identified by the names CLOSTRA-ATF, CLOSTRA-SAF, CLOSTRA-STF, and CLOSTRA-SATF).

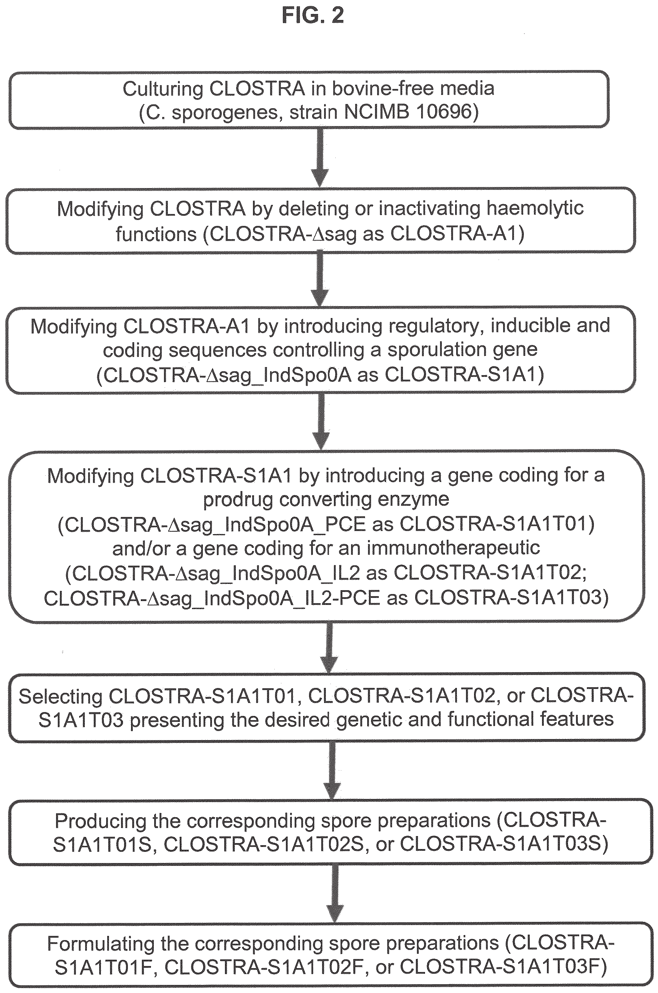

Exemplary schematic representations of strategies for generating the novel *Clostridium* strains disclosed herein for the clinical uses according to the invention are provided in FIG. 2, FIG. 12, and FIG. 16, wherein specific CLOSTRA-S, CLOSTRA-SA, CLOSTRA-SAT, CLOSTRA-SATS, and CLOSTRA-SATF strains are sequentially generated and characterized, in particular with respect to the instant novel *Clostridium* strains in which the sporulation can only be activated in specific cell culture conditions (*e.g.* by using a chemical compound), and any known undesirable effects caused by the *Clostridium* strains *in vivo* is eliminated (*e.g.* hemolysis in human subjects, use of specific molecules as a source of energy or biological building blocks, and/or growth and replication in specific tissues), or least substantially reduced. Such CLOSTRA strains can be modified to express therapeutically relevant proteins (*e.g.,* a cytokine, an antibody, an antigen, and/or a prodrug converting enzyme), and can be more efficiently and safely administered as cells (or spores) to be used in cell culture containers (for producing metabolites or proteins of industrial interest) or *in vivo* for a variety of medical uses, for instance, orally, systemically (*e.g.* intravenously), or injected within specific tissues (*e.g.* intratumorally), and formulated or not with a drug that may be activated by the novel *Clostridium* strains (or spores) *in vivo.*

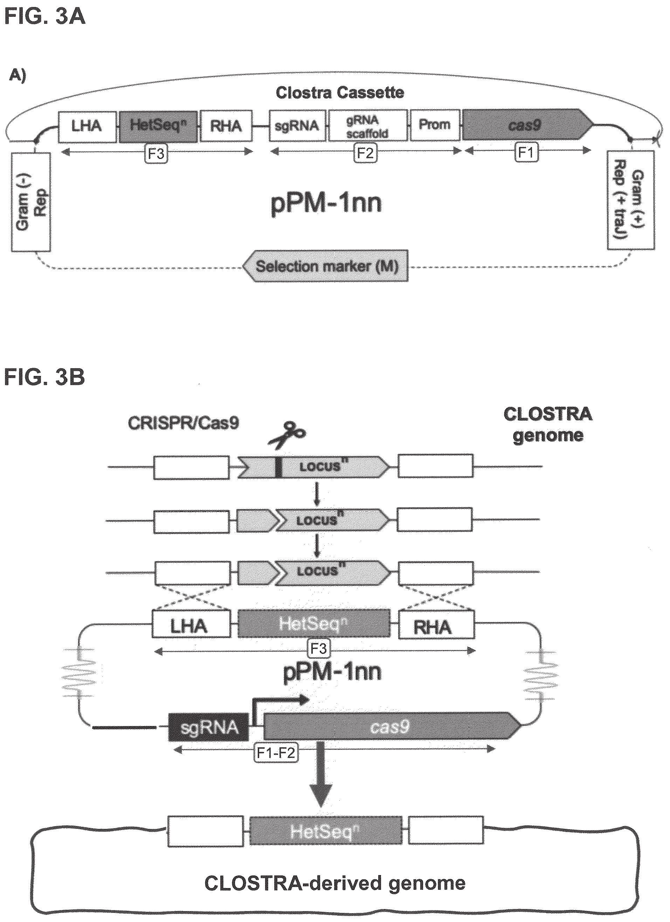

The novel *Clostridium* strains described herein can be produced using conventional techniques permitting the modification of a *Clostridium* genome in a specific and controlled manner, such as by means of plasmids and other constructs wherein the sequences to be integrated in the *Clostridium* genome are appropriately cloned, modified, oriented, and ordered in the genome, using standard recombinant DNA technologies applicable to *Clostridium* strains. The resulting CLOSTRA-S, CLOSTRA-A, and CLOSTRA-T strains (or other strains presenting the combinations listed above) feature the desired modification(s) and relevant phenotype(s) in a stable manner. In particular, such *Clostridium* strains can be modified by using plasmids allowing the integration of sequences in *Clostridium* genome through exploitation of the strains' own homologous recombination system, for example, by utilizing approaches based on CRISPR/Cas9 technologies. FIGs. 3A-3B, FIGs. 5A-5B, FIG. 12 and FIGs. 16A-16C provide an overview of plasmid features, plasmid cloning strategies and the resulting genomic modifications related to the use of CRISPR/Cas9 technologies for the purposes of generating specific CLOSTRA-S, CLOSTRA-A, CLOSTRA-T strains, and other combinations disclosed herein. Exemplary CLOSTRA strains present the desired combinations of features within the original CLOSTRA genome, such as tag sequences, marker sequences, inducible gene expression regulatory sequences, and sequences coding for enzymes (targeting specific drugs or carbon sources), therapeutic proteins such as cytokines (*e.g.* IL-2, IL-10, IL-12, IL-15), antigens raising an immunological response in humans (*e.g.* against a specific viral or bacterial proteins, leading to vaccination or immunization against such virus or bacteria), growth factors, toxins, or antibodies (*e.g.* anti-CTLA4, anti-PD-L1, or anti-PD-1 antibody).

Additional products and methods can be associated with the production and use of the novel *Clostridium* strains disclosed herein, in particular when the final use is related to a therapeutic context and/or for modes of administration relating to such uses (*e.g.,* as injectable formulations). Novel Clostridial cell culture media and conditions are also established to provide one or more of cells and spores that are compliant with regulatory and practical requirements including as GMP (Good Manufacturing Practices), clinical procedures, or environmental obligations normally applicable to genetically modified organisms, with an absolute control of sporulation when *Clostridium* cells are used for any application. Further embodiments according to the present invention relating to specific novel products, uses and methods are additionally disclosed in the following detailed description and in the Examples below.

## BRIEF DESCRIPTION OF THE DRAWINGS

**FIG. 1**: Schematic representation illustrating the manufacture and uses of the novel genetically modified *Clostridium* strains disclosed herein. Starting from a reference *Clostridium* strain (indicated as CLOSTRA), two distinct types of genetic modifications are specifically designed for either modifying the sporulation process of the strain (obtaining the reference strain indicated as CLOSTRA-S) or for modifying replication-unrelated, biological functions, in particular by integrating heterologous genes coding for an enzyme or other useful protein (obtaining the reference strain indicated as CLOSTRA-T), or by deleting fully or partially one or more *Clostridium* genes (obtaining the reference strain indicated as CLOSTRA-A). These changes can be pursued sequentially and/or in parallel, or separately, in any order, such that the genes are combined to create alternative reference strains (indicated as CLOSTRA-AT, CLOSTRA-ST, CLOSTRA-SA, and CLOSTRA-SAT). If more than one genetic modification is required for generating an appropriately modified CLOSTRA-Derived Strain for a given category, the genetic modifications can be introduced and identified separately (for instance, as CLOSTRA-T01, CLOSTRA-T02, etc. strains), and the genetic modification required for introducing other features (such as those found in CLOSTRA-S or CLOSTRA-A) can be performed in between those for generating CLOSTRA-A and CLOSTRA-T. These novel, genetically modified *Clostridium* strains (CLOSTRA-Derived Strains) can be used directly for industrial and medical purposes as cells, or, following generating the corresponding spore preparations, which may occur in the form of a liquid suspension or lyophilized preparation (indicated as CLOSTRA-STS, CLOSTRA-SAS, and CLOSTRA-SATS) that can be then appropriately formulated according to the contemplated specific use (indicated as CLOSTRA-STF, CLOSTRA-SAF, and CLOSTRA-SATF).

**FIG. 2**: Schematic representation of manufacture and uses of novel genetically modified *Clostridium* (CLOSTRA) strains in which the sporulation process, the haemolytic activity, and the expression of one or more non-Clostridial genes are simultaneously modified. A process for introducing three different modifications into the CLOSTRA genomes is indicated in one of the possible schemes, but preferably into CLOSTRA-SA in which two distinct gene modifications are performed separately (one for inserting a tag sequence and then inactivating an endogenous *Clostridium* gene presenting haemolytic activity in humans, and/or an external, inducible control of sporulation), is first generated (as exemplified by intermediate CLOSTRA-A1 and CLOSTRA S1A1). In the next stage, one or more genes coding for mammalian and/or bacterial proteins having biological, pharmacological, drug properties, or alternatively drug-converting properties when expressed in vivo, are inserted to generate the corresponding CLOSTRA-SAT strains. The resulting CLOSTRA-SAT strains can then be selected and screened for identifying those strains presenting the most appropriate genetic and functional features in terms of expression of exogenous, therapeutic genes, sporulation control, and/or replication in GMP culture systems so that the corresponding spores CLOSTRA-SAT can be manufactured, formulated, and used, in particular for medical uses that involve, and are compatible with, the administration of in vivo *Clostridium* spores (as exemplified by CLOSTRA-S1A1T01S, CLOSTRA-S1A1T02S, and CLOSTRA-S1A1T03S). The process for constructing CLOSTRA-SAT and then producing corresponding CLOSTRA-SATS and CLOSTRA-SATF, can be adapted by inactivating or deleting two or more *Clostridium* genes (in particular, relating to the degradation or metabolization of a chemical or biological substrate) for generating CLOSTRA-Derived Strains having a phenotype to be exploited during selection and/or later use. One example relates to the deletion of a PyrE gene, generating a CLOSTRA-Derived Strain in which uracil auxotroph mutant is a relevant phenotype (in addition to the absence of the hemolytic activity). Such alternative exemplary strain CLOSTRA-A2 can be used to incorporate additional elements, including those disclosed above, for example, a sequence for an external, inducible control of sporulation and/or coding for mammalian and/or bacterial proteins presenting biological, pharmacological, or drug-like properties, or alternatively, drug-converting properties (*i.e.,* generating the corresponding CLOSTRA-S1A2 strain and related CLOSTRA-SAT, CLOSTRA-SATS, and CLOSTRA-SATF). Additional details and examples of these embodiments are provided in Examples 3-4 and in the Figures of the instant disclosure.

**FIG 3**: Schematic representations showing generating CLOSTRA-A, CLOSTRA-S, CLOSTRA-T separately, or combining their respective features into CLOSTRA-AT, CLOSTRA- SA, CLOSTRA-ST, or CLOSTRA-SAT. FIG. 3A: Exemplary structure of the pPM-1nn template plasmid comprising, as a backbone, the genetic elements allowing the replication of a plasmid and its maintenance by selection in different microorganisms, in particular Gram-negative and Gram-positive replicons (Gram-(-) Rep and Gram-(+) Rep (the latter including a *TraJ* gene required for correct replication and conjugation in the *Clostridium* species), and a selection marker (M) that may be required during the cloning and selection stages. Such backbone is completed with the Clostra Cassette including three primary elements (F1, F2, and F3) for homologous recombination within the CLOSTRA genome using a CRISPR/Cas9 approach. A first element includes a heterologous sequence (HetSeq", referring to coding, regulatory, or tag sequences, as well as incomplete or complete genes), which is cloned between sequences used as Left Homology Arm and Right Homology Arm (referred to as LHA and RHA, respectively; F3). A second element includes a sgRNA module designed to specifically guide the CRISPR/Cas9 modification into the CLOSTRA genome (F2). A third element includes a Cas9 gene to be expressed into CLOSTRA (F1). All of these elements can be sourced from commercially available plasmids or alternatively, amplified from genomic, transcribed, or retro-transcribed nucleic acids, before being cloned within either a plasmid backbone or in the Clostra Cassette, in the order shown, or any other order sufficient to provide an effective expression and/or integration of the cloned nucleic acids. The size and orientation of genetic elements as shown is not representative for all plasmids derived from pPM-1 nn. FIG. 3B: General scheme for modifying a Clostridial genome (referred to as CLOSTRA genome) in a given position (Locus") using a HetSeq$^n$ and a pPM1-1nn plasmid (represented as a simple schematic structure, i.e. showing only the Clostra Cassette relevant for modifying the CLOSTRA genome). HetSeq" from F3 is flanked in the Clostra Cassette of pPM-1nn by sequences homologous to the genome up- and down-stream (left and right homology arms, LHA and RHA) of the gRNA target site. Following a double-stranded break of the CLOSTRA genome by Cas9 (due to the activity of coding and regulatory sequences in F1 and F2 of Clostra Cassette in pPM-1nn), cells that repair the break by the high-fidelity, homology-directed repair pathway, using pPM-1nn plasmid as a template for repair, will remove the gRNA target site and introduce HetSeq", thereby re-establishing the integrity of the CLOSTRA genome and thus allowing survival of cells comprising such CLOSTRA-derived genome.

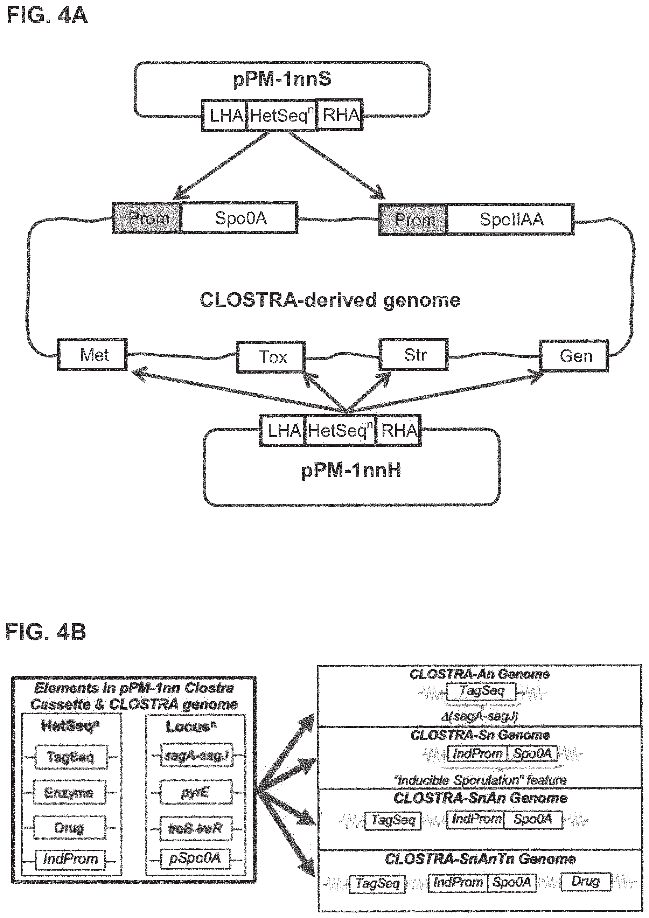

**FIG 4**: Schematic representation of exemplary types and combinations of HetSeq" that can be cloned into pPM-1 nn and of a Locus" that can be targeted within the CLOSTRA genome and exploited for separately or sequentially knocking-in or knocking-out full genes, coding sequences, and/or regulatory sequences in the CLOSTRA genome. FIG. 4A: An exemplary CLOSTRA-derived genome can be modified using a type of pPM-1nn (pPM-1nnS) in which the HetSeq" of a Clostra Cassette is a regulatory sequence that is targeted to the promoter of a sporulation gene as Locus" (as exemplified by *Spo0A* and *SpoIIAA*). After (or before) the modification in the regulation of a sporulation gene, such CLOSTRA-derived genome can be modified using a type of pPM-1nn (pPM-1nnH) in which HetSeq" of the Clostra Cassette is a marker, coding and/or regulatory sequence that is targeted to a different Locus", being, for example, a gene for a metabolic activity (Met), a toxic activity (Tox), a structural protein (Str), or any other coding or non-coding element (Gen). FIG. 4B: Four exemplary $HetSeq^n$ sequences such a tag sequence (TagSeq), a DNA coding for an enzyme active in CLOSTRA (Enzyme), a DNA coding for protein having therapeutic activity (Drug), or a promoter that can be induced in CLOSTRA (IndProm), are aligned with four exemplary $Locus^n$ sequences in CLOSTRA genome that can be targeted and modified by introducing a pPM-1nn plasmid (being a pPM-1nnS or pPM-1nnH plasmid) in CLOSTRA. Examples of modified CLOSTRA genomes resulting from the consecutive use of one, two, or three of such distinct pPM-1nn plasmids are shown by way of an exemplary positioning, orientation, and size of introduced HetSeq", generating the desired CLOSTRA-A, CLOSTRA- S, CLOSTRA-T, and combinations listed above, in particular where the inducible sporulation feature is present.

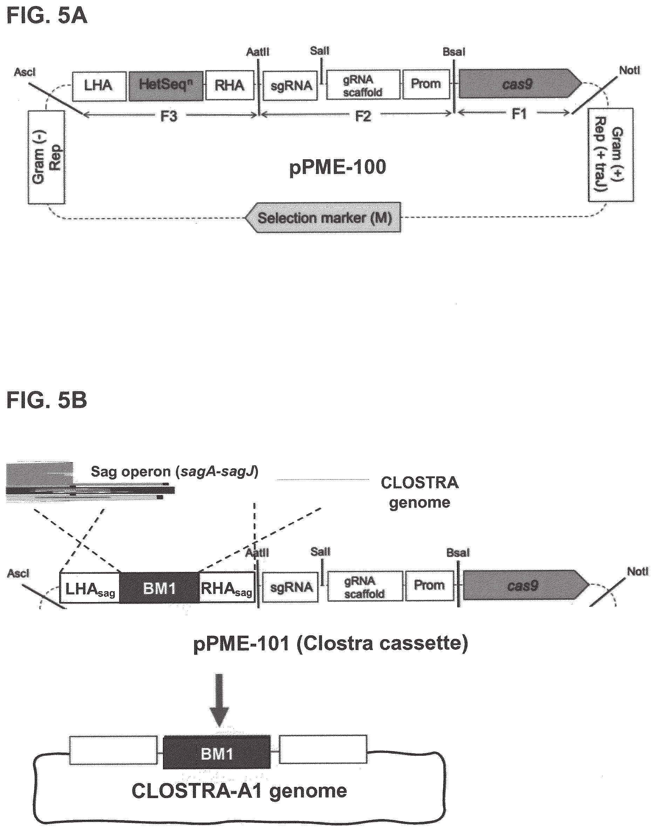

**FIG. 5**: Designing an exemplary CLOSTRA-A based on *Clostridium sporogenes* (chosen as a representative CLOSTRA), including a tagged sequence as $HetSeq^n$ instead of CLOSTRA gene sequences controlling haemolysin expression. FIG. 5A: The generic reference plasmid pPME-100 is based on the pPM-1nn structure and includes specific restriction sites for facilitating cloning process, as schematically represented with a Clostra Cassette cloned between *AscI* and *NotI* restriction sites and sequence element forming a Clostra Cassette referred to as F3 (including the HetSeq", cloned between the sequences for homologous recombination LHA and RHA). The two elements for performing CRISPR-Cas9 technology in *Clostridium* strains (F1 and F2), with further restriction sites available for cloning, are also shown (*AatII, SalI,* and *BsaI*). FIG. 5B: An exemplary pPM-1nnH plasmid named pPME-101 contains the PM-CB tag sequence (BM1) within the Clostra Cassette (represented here) that is inserted in the Streptolysin S ("Sag") operon (from *sagA* to *sagJ*), and subsequently generating CLOSTRA-A1 strains with a haemolysin negative phenotype. Left- and right homology arms ($LHA_{sag}$ and $RHA_{sag}$) flanking the PM-CB tag sequence (BM1) are selected and assembled in the F3 element of a Clostra Cassette so that F1 and F2 elements for performing CRISPR-Cas9 technology, also in the Clostra Cassette, can trigger homologous recombination between the sequences surrounding the Sag operon CLOSTRA genome and the pPME-101 plasmid, establishing the CLOSTRA-A1 genome.

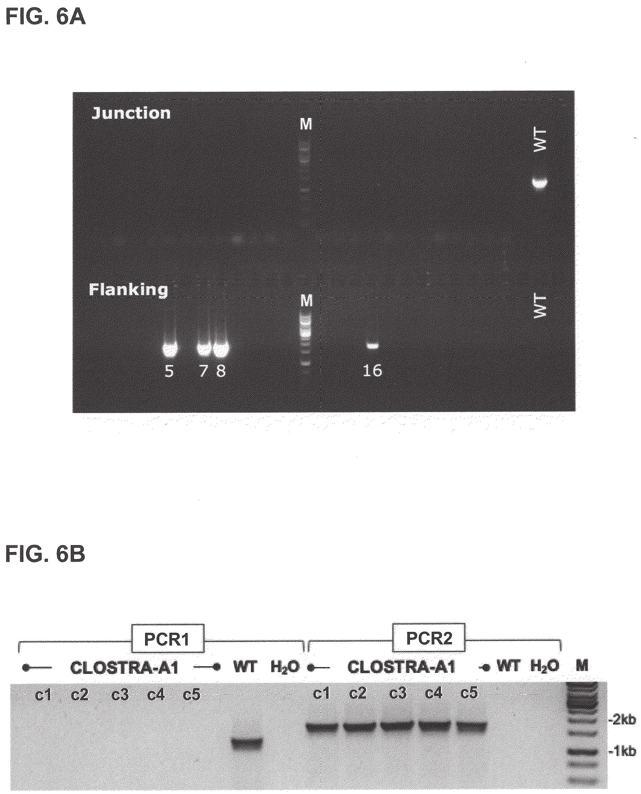

**FIG 6**: Validating an exemplary CLOSTRA-A based on *Clostridium sporogenes* (chosen as a representative CLOSTRA), including a tagged sequence as $HetSeq^n$ instead of the *C. sporogenes* gene sequences leading haemolysin expression and *in vivo* haemolytic activity. FIG. 6A: PCR screening for Streptolysin S operon deletion □□SagA-SagJ) among 24 CLOSTRA-Derived colonies potentially carrying a PM-CB tag sequence and featuring a Sag operon deletion. Colonies selected from a plate of trans-conjugants were PCR-screened using two primer pairs (see Materials & Methods described in Example 1 herein). Four of the 24 colonies (clones 5, 7, 8, and 16) produced a 1.8kb band characterizing CLOSTRA-A1 knockout genotype. FIG. 6B: PCR screening for a Streptolysin S operon deletion □□Sag) among 16 CLOSTRA-Derived colonies potentially carrying a BM1 tag sequence and featuring a Sag operon deletion (CLOSTRA-A1). Colonies selected from a plate of trans-conjugants (c1-c5) were PCR-screened using two distinct primer pairs. The first pair is used in a PCR reaction (PCR1) where a fragment of 1375 bp is amplified only in the original, unmodified CLOSTRA (WT). The second pair is used in a PCR reaction (PCR2) where a product of 1844bp is amplified only in the correctly modified CLOSTRA-A1 (in WT, the PCR2 amplification would lead to a fragment of more than 10kb, well beyond a standard PCR reaction). The DNA fragment markers are indicated in both panels as M. Sanger sequencing of PCR products confirmed the absence of sag operon and the presence of PM-CB tag sequence.

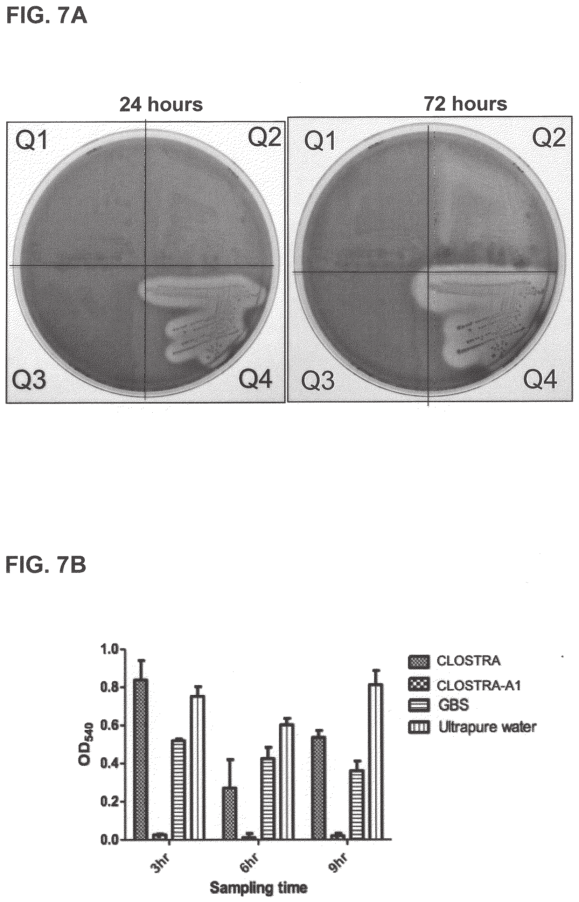

**FIG. 7**: *In vitro* analysis of CLOSTRA-A1 compared to control CLOSTRA cells. FIG. 7A: Analysis of red blood cell breakdown using Columbia Blood Agar plate (5% sheep blood, BD Biosciences) at 24 and 72 hours following initial colony streaking. Four cell types are tested at both time points: *C. sporogenes* sag knockout (CLOSTRA-A1; sag = Streptolysin S homologue; Q1), *C. sporogenes* wild-type, (CLOSTRA; Q2), *C. butyricum* (negative control; Q3), Group B Streptococcus (positive control; Q4). FIG. 7B: Quantitative analysis of human red blood cell breakdown by wild type and knockout strains of *C. sporogenes* in a liquid blood assay, in which hemoglobin release is measured at $OD_{540}$ for CLOSTRA, CLOSTRA-A1, Group B Streptococcus (GBS, positive control being an established beta-hemolytic species).

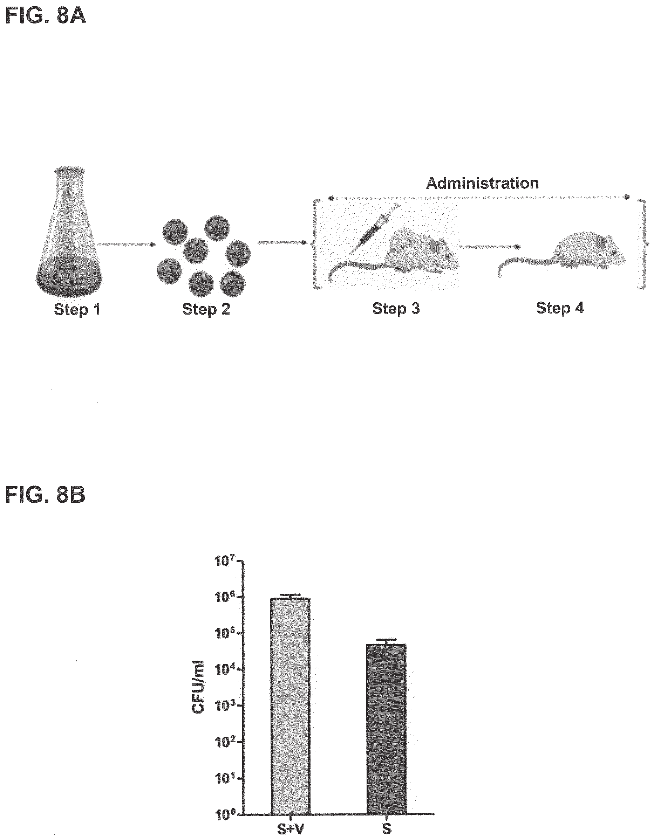

**FIG. 8**: Functional, *in vivo* analysis of CLOSTRA-A1 in the form of CLOSTRA-A1S spores. FIG. 8A: Schematic illustration of a CLOSTRA-A1S preparation starting from CLOSTRA-A1S cultures. (Step 1) The CLOSTRA-A1 strain was cultured in bovine free media (BFM) for sufficient spore formation. Resulting CLOSTRA-A1S pure preparations (Step 2) are formulated as CLOSTRA-A1F (approximately $10^6$ spores in 0.1 ml saline solution) for intravenous injection into 8 tumor-bearing animals in cancer mouse model (Step 3). Animals are subjected to a thorough physical examination every 24 hours, before being sacrificed on day 4, and tumors excised for colonization studies (Step 4). FIG. 8B: Tumor colonization study using the CLOSTRA-A1 strain. CLOSTRA-A1S (S) and vegetative CLOSTRA-A1 (V) are recovered from tumors of eight experimental mice expressed in colony forming units per ml of sample. S+V: spores and vegetative cells present in tumor samples before heat-treatment; S: spores only present in tumor samples following the heat-treatment procedure (80°C for 20 minutes). Samples were plated on pre-reduced plates with BFM medium and incubated for 24 hours under anaerobic conditions. The detection limit for colony counts was 50 CFU/ml.

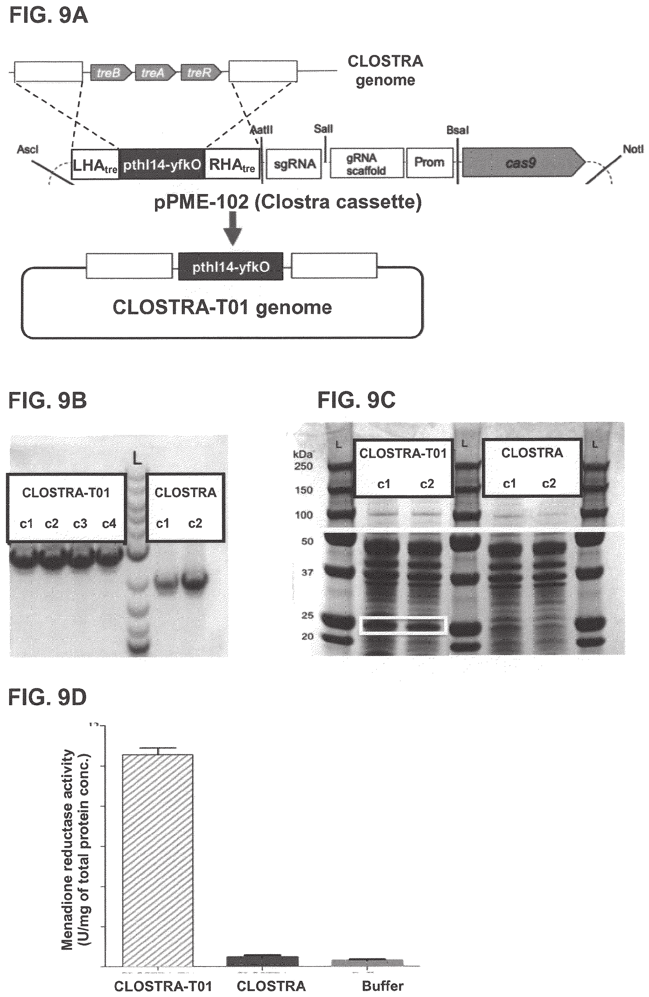

**FIG. 9**: Designing and validating an exemplary CLOSTRA-T expressing a prodrug converting enzyme as HetSeq$^n$ instead of a specific CLOSTRA genomic sequence. FIG. 9A: Plasmid pPME-102 (represented in a simplified format, *i.e.* showing only the exemplary Clostra Cassette relevant for modifying the CLOSTRA genome) containing the yfkO gene under the control of a Pthl14 promoter (Pthl14-yfkO) within the F3 element of Clostra Cassette to be inserted in a Trehalose operon (from treB to treR) and subsequently generating CLOSTRA-T01 with a Trehalose-negative phenotype. Left- and right homology arms ($LHA_{tre}$ and $RHA_{tre}$) flanking Pthl14-yfkO sequence are selected to trigger homologous recombination between the sequences surrounding the Trehalose operon within the CLOSTRA genome and pPME-102 plasmid. FIG. 9B: Confirmation of the presence of a YfkO sequence from pPME-102 plasmid in four CLOSTRA-T01 clones (c1-c4) when compared to two CLOSTRA wild type clones (c1-c2), as tested by PCR and visualized on 1.0% agarose gel (L = NEB 2-log DNA ladder). FIG. 9C: SDS PAGE gels on soluble fraction CLOSTRA-T01 clones and CLOSTRA clones after being cultured for 7 hours. Protein marker (L): Precision All Blue Protein Standard (Bio-Rad). SDS PAGE gels on standardized lysates of CLOSTRA-T01 and CLOSTRA (two clones for each). YfkO nitroreductase protein bands are indicated with a white box. Distinct bands of expected sizes for each NTR protein are visible and highlighted on the SDS PAGE gels. FIG. 9D: Menadione reductase activity of CLOSTRA-T01 (due to YfkO nitroreductase), CLOSTRA (as a negative control), or buffer only, as measured in 7-hour lysates that were prepared in triplicates using BugBuster reagents (Novagen Milipore, 70584) in accordance with manufacturer's recommendations (Novagen, User Protocol TB245 Rev. F 1108). Enzymatic activity was measured by the change in absorbance at $\lambda$ = 550 nm over 60 seconds. Error bars represent the standard error of the means. Menadione reduction is used as standard substrate to quantify the enzymatic activity of a PCE from the nitroreductase family.

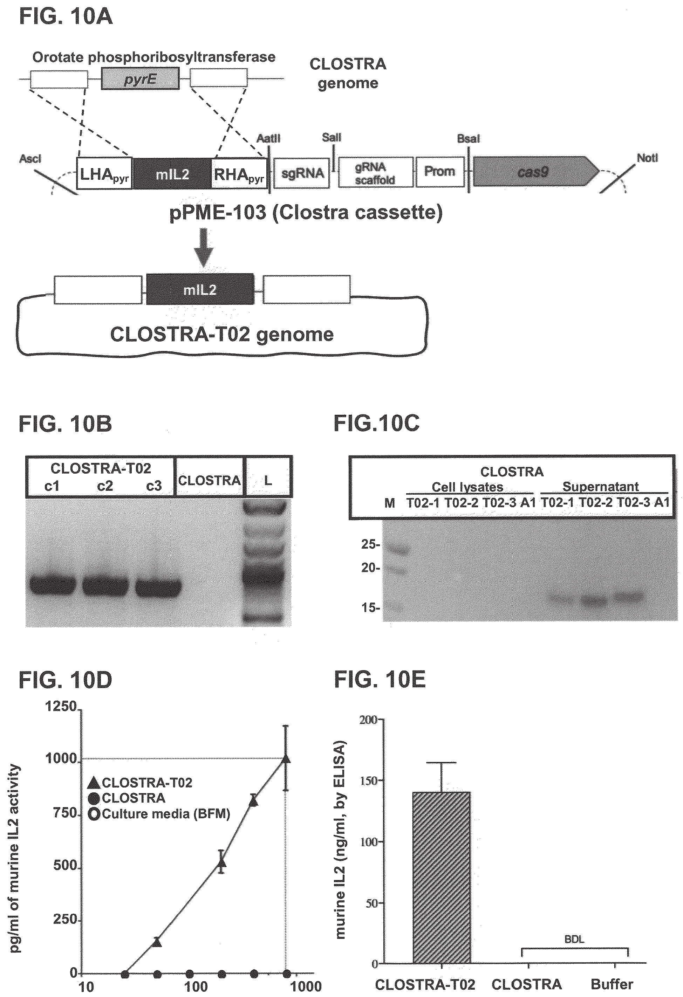

**FIG. 10:** Designing and validating an exemplary CLOSTRA-T expressing a cytokine as HetSeqn instead of CLOSTRA genomic sequences. FIG. 10A: When compared to the simplified representation of pPME-102 shown in FIG. 9A, F3 of the Clostra Cassette in Plasmid pPME-103, contains the murine interleukin 2 coding sequence that was placed under the control of a Pfdx promoter and nprM3 signal sequence within the Clostra Cassette (Pfdx- nprM3-IL2, the whole sequence represented as mIL2; full PfdxnprM3-mIL2-Flag, SEQ ID NO: 63), and then inserted at a pyrE locus within CLOSTRA genome, subsequently generating CLOSTRA-T02 with a pyrE negative phenotype (uracil auxotroph). Left- and right homology arms ($LHA_{pyr}$ and $RHA_{pyr}$) flanking the mIL2 gene are selected to trigger homologous recombination between the sequence surrounding pyrE (orotate phosphoribosyltransferase) gene in CLOSTRA genome and pPME-103 plasmid. FIG. 10B: Confirmation of the presence of mIL2 gene in three CLOSTRA-T02 clones (c1-c3) when compared to non-modified CLOSTRA clone, as tested by PCR and visualized on 1.0% agarose gel, with bands corresponding to the expected fragments (approximately 950 bp) not produced in original CLOSTRA. FIG. 10C: Visualisation of Western immunoblotting (Immunoblot analysis) performed on control CLOSTRA-A1 (A1) and three CLOSTRA-T02 clones (T02-1/-3). The cell lysates and supernatant fractions were separated by SDS-PAGE, transferred on a nitrocellulose membrane, and analysed using an anti-Flag-tag antibody. "M" identifies protein marker (Precision Plus Protein™ All Blue, Bio Rad). Flag-tagged murine IL-2 protein is visualised on the nitrocellulose membranes with TMB-Blotting 1-Step Solution (Thermo Scientific Pierce) and corresponds to a size of approximately 18.2 kDa. FIG. 10D: Biological activity of mIL2 secreted by a selected CLOSTRA-T02 clone measured by cellular proliferation of CTLL-2 cell line, with culturing media (BFM) and CLOSTRA as a negative control. FIG. 10E: Secreted murine IL2 is measured by ELISA for a CLOSTRA-T02 clone, non-modified CLOSTRA, and buffer only as a negative control (BDL: below detection limit).

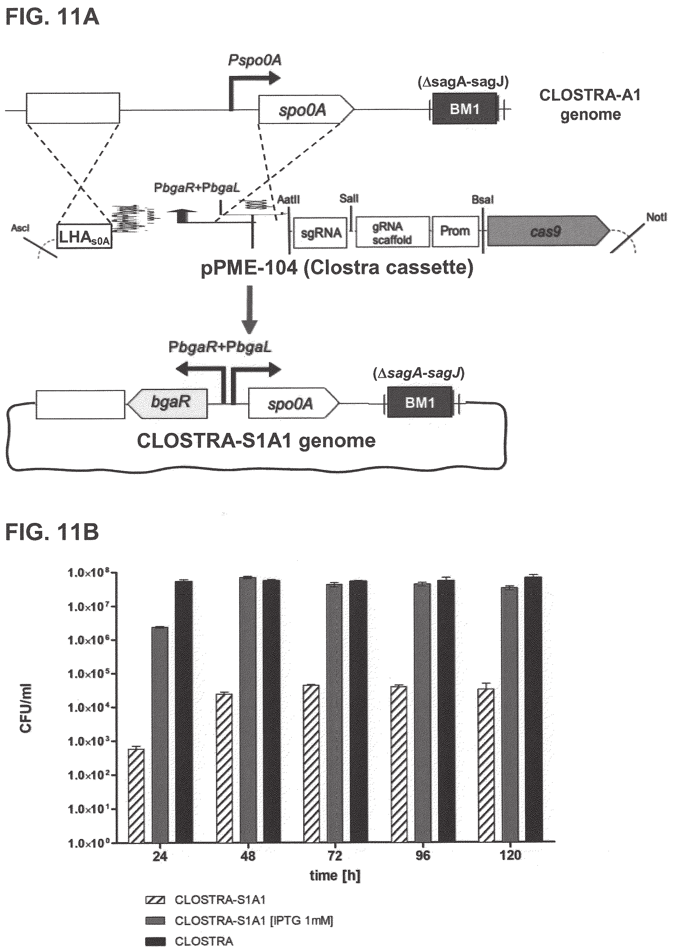

**FIG. 11:** Designing and validating an exemplary CLOSTRA-SA based on CLOSTRA-A already including a tagged sequence as HetSeq$^n$ instead of CLOSTRA genomic sequences controlling haemolysin expression and further, subsequently modified to present conditional sporulation features in vitro and in vivo. FIG. 11A: When compared to the simplified representation of pPME-102 in FIG. 9A, F3 of the Clostra Cassette in plasmid pPME-104, the heterologous sequence contains a bgaR transcription regulator that is placed under the control of a divergent promoter (PbgaR:PbgaL) containing two lactose operators, such as those described in the literature (Hartman AH *et al.,* 2011). Left- and right-side homology arms ($LHA_{s0A}$ and *spo0A* as RHA) flanking the spo0A promoter (*Pspo0A*) are selected to trigger homologous recombination between CLOSTRA-A1 genome and pPME-104 plasmid. Upon heterologous recombination, the native *spo0A* promoter in CLOSTRA-A1 is targeted and replaced by a lactose inducible system controlling *spo0A* and subsequently generating CLOSTRA-S1A1 with a haemolysin negative phenotype. An alternative CLOSTRA strain (CLOSTRA-S2A1) can be similarly generated by using, as left- and right-side homology arms (LHA and RHA), the sequences flanking the *spoIIAA* promoter (*Pspo0A*). FIG. 11B: Testing CLOSTRA-S1A1 inducible sporulation. A CLOSTRA-S1A1 strain was cultured in BMF media with and without the addition of a 1 mM IPTG solution. Heat-treated samples, taken at 24 hours intervals post-inoculation, for five subsequent days, were plated in anaerobic conditions in serial dilution on pre-reduced BFM agar plates. After a 24-48-hour incubation, formed colonies were evaluated. Bars represent the number of CFU (colony forming unit) per ml of listed CLOSTRA cultures. The data represent the average of three independent experiments and error bars indicate the standard errors of the means. The detection limit for colony counts was 50 CFU/ml.

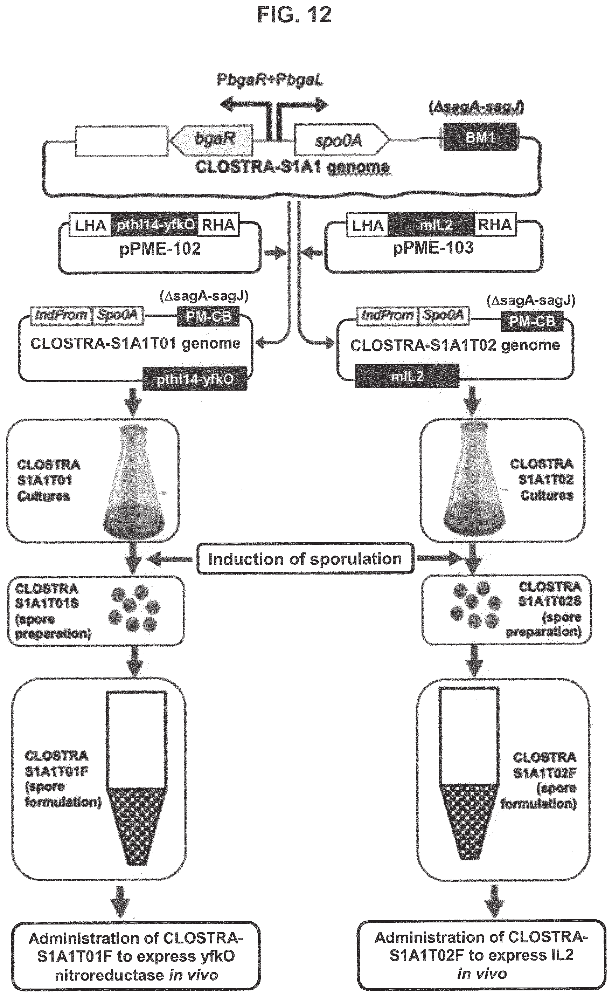

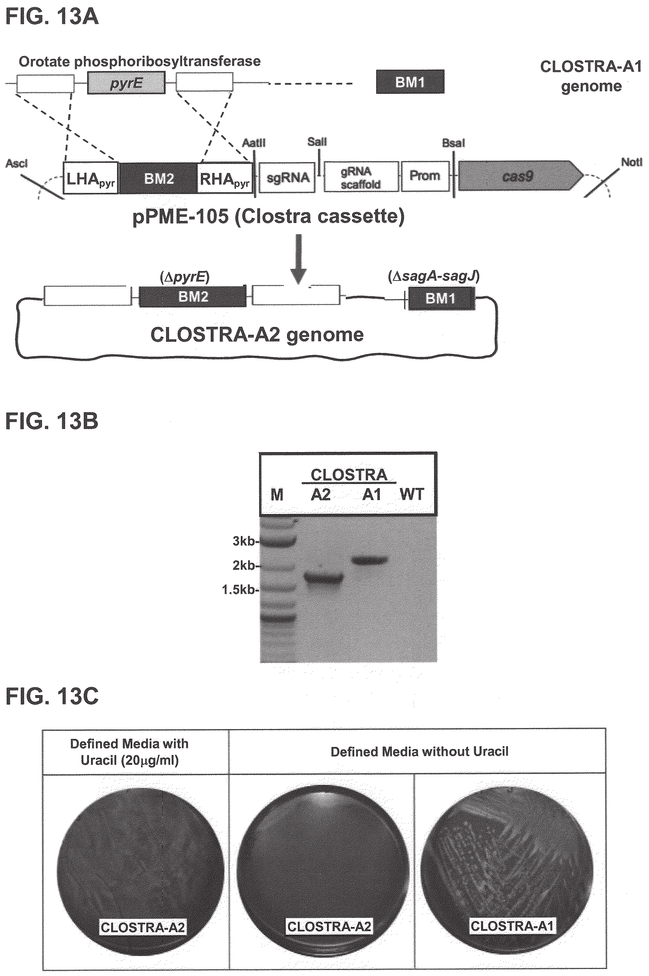

**FIG. 12**: Schematic representation of two approaches for generating exemplary, alternative CLOSTRA-SAT strains based on the same CLOSTRA-SA strain, whose genome can be modified using the disclosed pPME-102 and pPME-103 for modifying a CLOSTRA strain into CLOSTRA-T01 and CLOSTRA-T02 strains, respectively. Resulting CLOSTRA-S1A1T01 and CLOSTRA-S1A1T02 strains can be used to generate CLOSTRA-S1A1T01S, CLOSTRA-S1A1T01F, CLOSTRA-S1A1T02S, and CLOSTRA-S1A1T02F formulations (comprising either spores or cells), after inducing sporulation with an appropriate compound, isolating the spores, and formulating them in order to be administered *in vivo,* where the cells originated by such spores will express either YfkO nitroreductase or murine Interleukin 2 (to be substituted with a human coding sequence, in the case of clinical applications). The PM-CB tag sequence (or BM1) replaces the Sag operon in the indicated CLOSTRA-Derived Strains. Moreover, CLOSTRA-S1A1T01 can be further modified to integrate a therapeutic gene of interest within pPME-103, thereby generating CLOSTRA-S1A1T03 with a combination of two therapeutic genes, for instance, a cytokine and a prodrug converting enzyme. Further alternative CLOSTRA-SAT strains can be generated starting from alternative CLOSTRA-S and CLOSTRA-A strains (shown in FIGs. 13-16) used for producing the corresponding spores and formulations according to this FIG. 12.

**FIG. 13**: Designing and validating a CLOSTRA-A2 strain based on a CLOSTRA-A1 strain, including a BM2 tagged sequence as HetSeq[n] instead of pyrE (orotate phosphoribosyltransferase gene) in CLOSTRA-A1. FIG. 13A: When compared to the simplified representation of pPME-102 in FIG. 9A, F3 of the Clostra Cassette in the pPME-105 plasmid contains a BM2 tag sequence. Left- and right homology arms ($LHA_{pyr}$ and $RHA_{pyr}$) flanking the BM2 tag sequence are selected to trigger homologous recombination between the sequences surrounding the pyrE gene in the CLOSTRA-A1 genome and pPME-105 plasmid. FIG. 13B: Visualization of PCR products on an agarose gel confirming the deletion of pyrE gene (609bp deletion) from the CLOSTRA-A1 genome (A1) and selection of CLOSTRA-A2 strain containing a BM2 tag sequence. (CLOSTRA-A2), using water as a control (WT). M: 1kb plus DNA ladder. FIG. 13C: Validating CLOSTRA-A2 clones as uracil auxotroph mutants by cultivation on defined growth medium according to the literature (Lovitt *et al.,* 1987). Following a 48 hour-incubation in anaerobic conditions, CLOSTRA-A2 grows in defined medium only when supplemented with uracil (20□g/ml) and fails to grow in media lacking such supplementation. CLOSTRA-A1 strain has been used as a control to demonstrate the functionality of *pyrE* gene and subsequent ability to grow in defined medium without any external source of uracil.

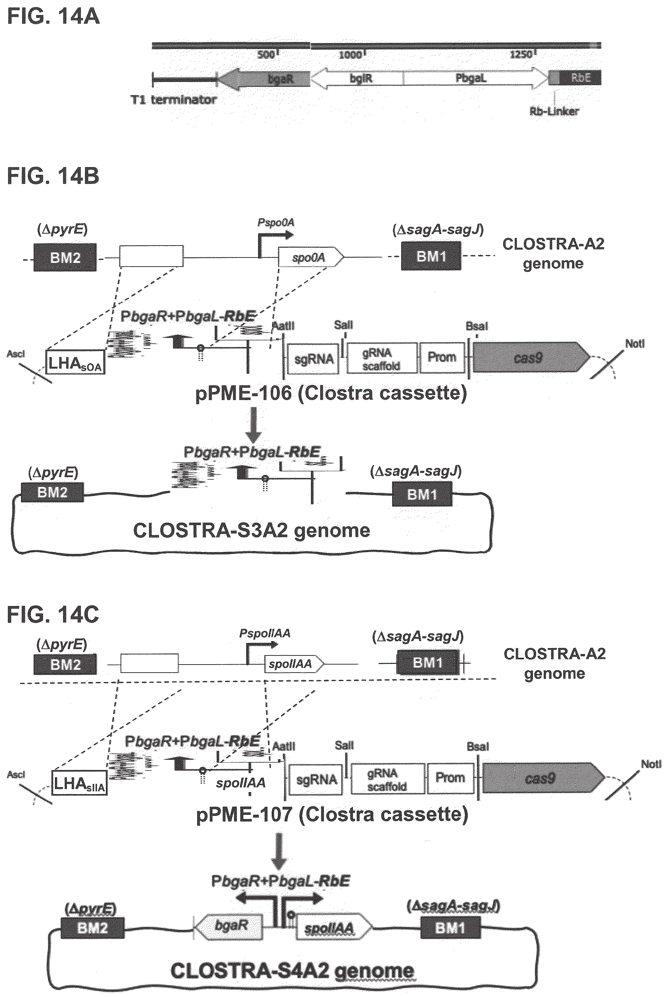

**FIG. 14**: Designing new CLOSTRA-Derived Strains based on the CLOSTRA-A2 strain (haemolysin and pyrE-negative) with two tag sequences (BM1 and BM2), subsequently modified to present *in vitro* control of the spore formation process. FIG. 14A: Schematic organization of a BgaR transcription regulator placed under control of a divergent promoter (P*bgaR*:P*bgaL*) where P*bgaL* has been altered to include riboswitch sequence (Rb-E) after the +1 initiation start. FIG. 14B: When compared to the simplified representation of pPME-102 in FIG 9A, F3 of the Clostra Cassette in the pPME-106 plasmid contains a *bgaR* transcription regulator that is placed under the control of a divergent promoter (P*bgaR*:P*bgaL*) containing two lactose operators, such as those described in the literature (Hartman AH *et al.,* 2011) and modified to contain theophylline inducible riboswitch sequence as those described in literature (Topp *et al.,* 2010). Left- and right-side homology arms ($LHA_{s0A}$ and *spo0A* as RHA) flanking the *spo0A* promoter (P*spo0A*) in pPME-106 plasmid are selected to trigger homologous recombination between the CLOSTRA-A2 genome and the pPME-106 plasmid. Upon heterologous recombination, the native *spo0A* promoter in CLOSTRA-A2 is targeted and replaced by a lactose- and theophylline-inducible system controlling sporulation at the level of *spo0A* and subsequently generating CLOSTRA-S1A2 with haemolysin- and uracil-negative phenotypes. FIG. 14C: A corresponding CLOSTRA-S2A2 strain has been generated using the pPME-107 plasmid, which differs from pPME-106 by including, as LHA and RHA sequences, the sequences $LHA_{sIIA}$ and *spoIIAA* as RHA) flanking the *spoIIAA* promoter (*PspoIIAA*), such that upon heterologous recombination, the native spoIIAA promoter in CLOSTRA-A2 are targeted and replaced by a lactose- and theophylline-inducible system controlling sporulation at the level of *spoIIAA,* yet still obtaining a CLOSTRA strain with haemolysin- and pyrE-negative phenotypes.

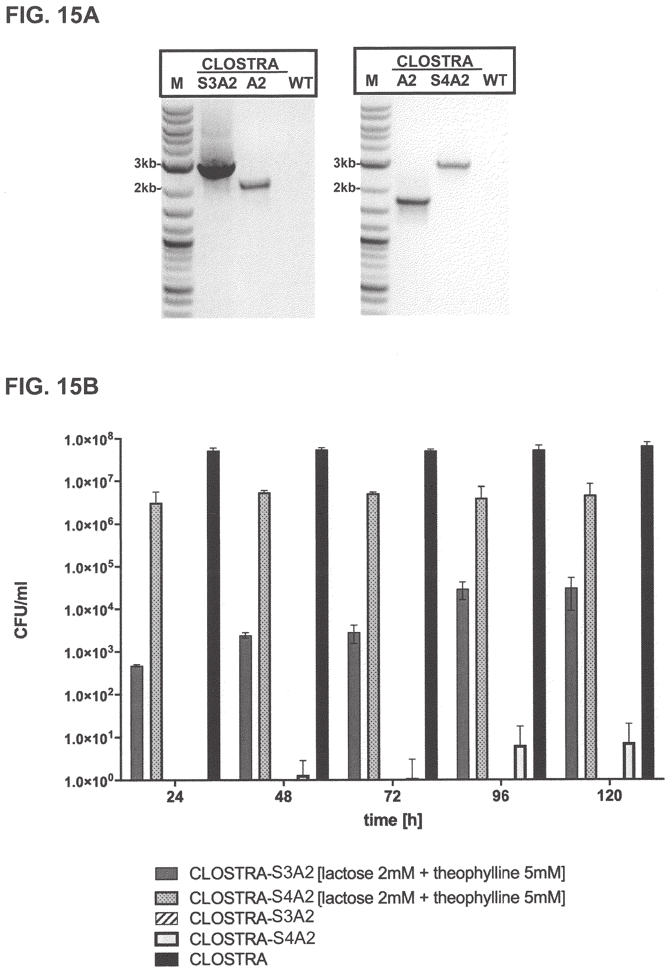

**FIG 15:** Validating new strains based on the CLOSTRA-A2 strain (haemolysin and pyrE-negative with two tag sequences (BM1 and BM2) that present *in vitro* control of spore formation process using two different *Clostridium* sporulation genes. FIG 15A: Confirmation of the CLOSTRA-S3A2 and CLOSTRA-S4A2 strains following PCR amplification using gene specific primers. CLOSTRA-S3A2 strain is characterized by a 422bp deletion and 1344bp insertion of the sequence comprising the inducible promoter (S3A2, with a PCR fragment of 3070 bp; CLOSTRA-A2, with a PCR fragment of 2151 bp; WT: water control). CLOSTRA-S4A2 strain shows a 145bp deletion and 1344bp insertion of the sequence comprising the inducible promoter (A2, with a PCR fragment of 1800 bp; S4A2, with a PCR fragment of 2999 bp; WT: water control). M: 1-kb marker ladder). FIG 15B: Testing the CLOSTRA-S3A2 and CLOSTRA-S4A2 strain in the context of inducible sporulation for developing heat resistant strains. CLOSTRA-S3A2 and CLOSTRA-S4A2 strains were cultured in BMF media with and without the addition of 2mM lactose and 5mM theophylline solutions (added four hours post-media inoculation). Heat-treated samples, taken at 24 hours intervals post-inoculation, for five subsequent days, were plated in anaerobic conditions in serial dilution on pre-reduced BFM agar plates. After a 24-48-hour incubation, formed colonies were evaluated. Bars represent the number of CFU (colony forming unit) per ml of listed CLOSTRA cultures. The data represent the average of three independent experiments and error bars indicate standard errors of means (detection limit :50 CFU/ml).

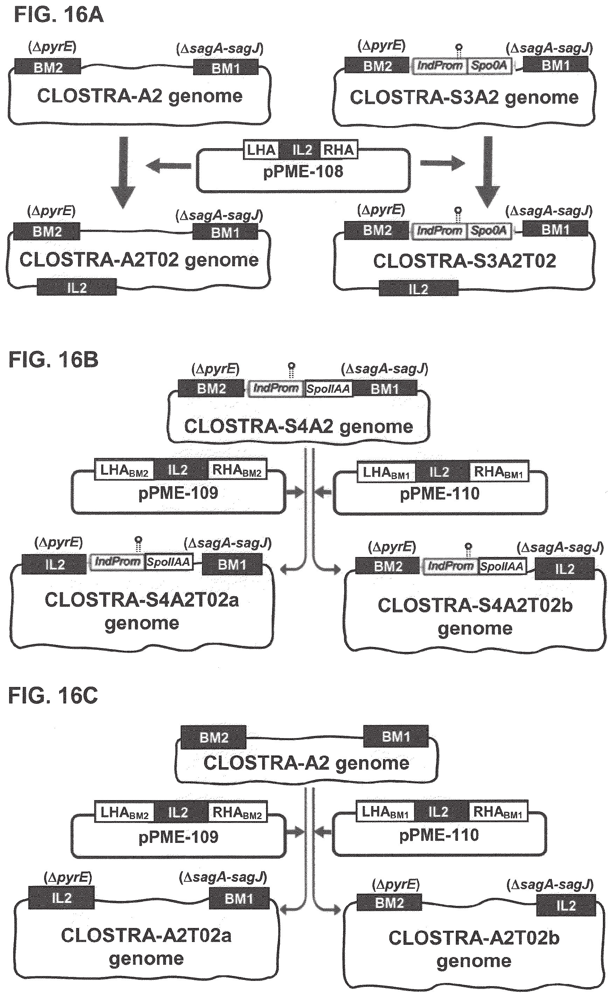

**FIG. 16:** Schematic representation of approaches for generating exemplary, alternative CLOSTRA-AT and CLOSTRA-SAT strains based on the integration of an exemplary therapeutic gene (IL2, interleukin 2I however, any disclosed therapeutic gene disclosed herein can be used, including those for a growth factor, an antibody, an antigen, or an enzyme) in different $Locus^n$ (natural or already modified) and different CLOSTRA-Derived strains (with an inducible or normal sporulation process). FIG 16A: The pPME-108 plasmid, a pPME-103 derivative plasmid, may contain a IL2 gene sequence with LHA and RHA sequences that are adapted to integrate this therapeutic gene in the same genomic position (*e.g.* for trehalose operon, $LHA_{tre}$ and $RHA_{tre}$) of two different CLOSTRA strains (CLOSTRA-A2 and CLOSTRA S3A2. Two additional pPME-103 derivative plasmids (pPME-109 and pPME-110) may be used targeting either a previously modified $Locus^n$ within a CLOSTRA-Derived Strain with inducible sporulation (CLOSTRA S4A2, generating CLOSTRA-S4A2T02a and CLOSTRA-S4A2T02b; FIG. 16B, with $LHA_{BM1}$ and $RHA_{BM1}$ in F3) or a CLOSTRA-Derived Strain with normal sporulation (CLOSTRA A2, generating CLOSTRA-A2T02a and CLOSTRA-A2T02b; FIG. 16C, , with $LHA_{BM2}$ and $RHA_{BM2}$ in F3). The resulting CLOSTRA strains can be used to generate cells, spore, and related compositions as described above.

## DETAILED DESCRIPTION OF THE INVENTION

*(i) General Definitions*

"CLOSTRA" refers to any species, strain, isolate, variant, and cells identified as belonging to the *Clostridium* genus that present sporulation and germination control in organisms, for example, being directly isolated from organisms and biological samples, or obtained from public sources, including repositories and cell banks. A non-exhaustive list of such specific *Clostridium* species includes *C. (Clostridium) butyricum, C. sporogenes, C. botulinum, C. difficile, C. acetobutylicum, C. autoethanogenum, C. beijerinckii, C. carboxidivorans, C. cellulolyticum, C. celerecrescens, C. cellobioparum, C. formicoaceticum, Clostridium josui, C. kluyveri, C. novyi, C. pasteurianum, C. papyrosolvens, C. perfringens, C. phytofermentans, C. polysaccharolyticum, C. propionicum, C. saccharoperbutylacetonicum, C. sticklandii, C. tetanii, C. thermocellum, C. thermobufyricum , C. thermoaceticum, C. populeti, Clostridium lentocellum, Clostridium chartatabidum, Clostridiumaldrichii, Clostridium herbivorans, C. madisonii., C. bifermentans, C. botulinum, C. brevifaciens, C. chauvoei, C. dissolvens, C. fallax, C. histolyticum, C. nigrificans, C. perfringens, C. putrificum, C. septicum, C. thermohyfrosulfuricum, C. thermosaccharolyticum, C. welchii, C. saccharolytieum, C. ljungdahlii,* and any isolate or strain found in a depositories and cell banks, described in the literature, and/or commonly used in industrial or clinical applications. Additional details and classifications pertaining to such *Clostridium* species to be used as CLOSTRA can be found in the literature (*e.g.,* Cruz-Morales P *et al.,* 2019).

Suitable CLOSTRA strains can be identified from those available in public collections such as American Type Culture Collection (ATCC), National Collections of Industrial, Food and Marine Bacteria (NCIMB), or National Collection of Type Cultures (NCTC), or from depositary Institutions that are International Depositary Authority Under the Budapest Treaty. The choice of *Clostridium* species to be used as CLOSTRA will depend on their biological features and later use of CLOSTRA-Derived Strains. For instance, *Clostridium* species known to colonize human tissues (such as internal organs or skin) will be preferred for generating CLOSTRA-Derived Strains to be used for medical applications (for instance, *C. butyricum* or *C. sporogenes*) directly, for being non-toxic, or after selecting strains missing already any toxic feature (as in the case of *C. novyi-NT* and other non-toxigenic variants of *C. botulinum, C. difficile, C. tetanii, or C. perfringens).* Any *Clostridium* species that specifically grows and metabolizes plant-based or other organic materials (for instance, *C. thermocellum, C. acetobutylicum, and C. cellulolyticum),* are preferred for generating CLOSTRA-Derived Strains useful for industrial applications. Indeed, a CLOSTRA genome may already include heterologous (non-Clostridial) sequences or present genomic deletions, rearrangements, mutations, duplications, or other genomic modifications from a reference genome sequence for a given *Clostridium* species unrelated to the use of sequences present in the Clostra Cassette as comprised in a plasmid or other vector that is designed, produced, and used according to the present invention.

"Locus^n" refers to any DNA sequence comprised in a CLOSTRA genome suitable for modification according to the methods of the present invention. Such DNA sequence in the CLOSTRA genome can be of any size, such as one or more full operons, one or more full genes, a replication site, or intergenic non-coding sequence, as well specific elements within such sequences including entire or partial coding sequences, promoters, or other sequence regulating gene expression or replication of any length and composition.

"HetSeq"" refers to any DNA sequence not comprised in a CLOSTRA genome, which is intended to be non-randomly integrated in a CLOSTRA genome according to the methods of the invention. This DNA sequence can be of any size and origin (including from the genome of a different CLOSTRA, bacteria, yeast, plant, mammal, human, or any man-made variant and artificial sequence) and may include as one or more full operons, one or more full genes, a replication site, or intergenic non-coding sequence, as well specific elements within them such as entire or partial coding sequences, tag sequences, DNA sequences that can be transcribed in specific RNA species, promoters, markers, or other sequence regulating gene expression or replication of any length and composition.

"Clostra Cassette" refers to a recombinant DNA sequence that is cloned in a plasmid or other vector comprising at least a first HetSeq" sequence to be integrated in a Locus" of CLOSTRA genome and, preferably, at least a second one HetSeq" sequence that allows the stable integration of the first HetSeq" in a Locus" of CLOSTRA genome. The first HetSeq" sequence may contain additional sequences at 5' and/or 3' end that are identical or at least highly homologous to the one within or surrounding the desired Locus", thereby triggering a precise integration of the first HetSeq" sequence into the CLOSTRA genome by homologous recombination (also defined as F3). The second HetSeq" sequence may include one or more genes coding for proteins that facilitate and/or guide the precise integration of DNA sequence in a CLOSTRA genome (*e.g.*, as shown in FIG. 3 and FIG. 4 for generic pPM-1nn, pPM-1nnH, and pPM-1nnS plasmids and also for the pPME-100 and pPME-100 derivative plasmids shown in e.g., FIG. 5, which feature elements suitable for the application of CRISPR/Cas9 technology within the F1/F2 elements. A Clostra Cassette may be constructed and used in any compatible plasmid or vector for DNA recombinant technologies, but preferably in a vector that can be maintained in Gram-positive and/or Gram-Negative bacteria.

"CLOSTRA-S" means a CLOSTRA strain, or CLOSTRA cells, presenting an inducible or repressible sporulation phenotype, in particular by means of compounds or conditions that can be used in cell culture conditions. Thus, the first HetSeq" present in the Clostra Cassette for generating CLOSTRA-S preferably contains non-Clostridial regulatory DNA suitably positioned within a Locus" being a CLOSTRA gene controlling or directly involved in a sporulation process, such as *spo0A, spoIIAA, spoIIE, sigH, sigF, sigE, sigG,* and *sigK.* Preferably, the sporulation genes are those that, according to each *Clostridium* species or strain, are identified in the literature (Shen S *et al.,* 2019; Al-Hinai M *et al.,* 2015) as being relevant in early phases of sporulation. As shown in the Examples, since the targeted, modified CLOSTRA gene is still present, conditional sporulation is possible and inducible in cell culture, *in vivo* and/or in any other condition, depending on the desired use, gene modification technology, and regulatory sequences included in the Clostra Cassette as the first (or subsequent) HetSeq^n.

"CLOSTRA-A" means a CLOSTRA strain, or CLOSTRA cells, presenting an inactivated, or at least attenuated, gene that normally provides CLOSTRA with biological activities unsuitable for subsequent medical or industrial uses, in particular where such use of the original CLOSTRA causes a decrease in activity, replication, and/or viability of non-CLOSTRA cells exposed to CLOSTRA in cell culture conditions, or *in vivo* (or even the properties of CLOSTRA itself when cultured at certain specific conditions and/or to a given density). Thus, the first (or subsequent) HetSeq" present in the Clostra Cassette for generating CLOSTRA-A preferably contains any DNA to be positioned within a Locus" being a CLOSTRA gene controlling or directly involved in undesired phenotype, disrupting, mutating, substituting, or otherwise mutating its regulatory and/or coding sequences, but with no or limited effects on CLOSTRA viability and other biological activities. For instance, the first HetSeq" can be a sequence negatively regulating the expression or Locus", introducing point mutagenesis, partial deletion, insertion or total deletion of the encoding region for the complete protein, or purely interrupting and substituting the entire, or a segment of, Locus" without providing any other activity. As shown in the Examples, substituting an operon involved in hemolytic CLOSTRA activities with a non-functional, reference, marker, or tag sequence substantially reduces such activity in CLOSTRA-A. The Locus" and related phenotype functionally inactivated or attenuated in CLOSTRA-A may be also related to antigenic sequences, enzymes, and in general by-products of Clostridial metabolism whose secretion and/or accumulation may be undesirable, for instance, including the use of specific molecules (as source of energy or for transcription and/or replication function) and/or the production of acids, alcohols, toxins, or other metabolic by-products. Moreover, CLOSTRA-A may combine the inactivation or deletion of two or more distinct Locus" following the integration at least multiple, distinct HetSeq" not related or not affecting the sporulation process, as shown in the examples with CLOSTRA-A2.

"CLOSTRA-T" refers to a CLOSTRA strain, or CLOSTRA cells, presenting a functional heterologous gene that is not present in CLOSTRA genome, providing CLOSTRA with biological activities beneficial for use in a therapeutic or industrial context, in particular where the use of the original CLOSTRA requires specifically contemplated activities directed to, for example, the physiological characteristics, replication, viability, and/or other properties of non-CLOSTRA cells exposed to CLOSTRA in cell culture conditions or *in vivo* (or even the CLOSTRA properties itself, when cultured at specific conditions and/or to a given density). Thus, the first (or subsequent) HetSeq" present in the Clostra Cassette for generating CLOSTRA-T preferably contains any gene (in its entirety or full coding sequences) to be positioned within a Locus" in CLOSTRA genome without affecting the normal (or desired) CLOSTRA phenotype, viability and other biological activities. For instance, the first HetSeq" can be a coding sequence for antigenic sequences (*e.g.* for vaccination applications), sequences coding for enzymes (targeting specific drugs or carbon sources), therapeutic proteins such as cytokines (*e.g.* IL-2, IL-10, IL-15), antigens raising an immunological response in humans, growth factors, toxins, or antibodies (*e.g.* anti-CTLA4, anti-PD-L1, or anti-PD1 antibody). The methods of treatment and regimens contemplated by the present invention can also involve the administration of CLOSTRA-T based, CLOSTRA-Derived PRODUCT that is preferably an antibody, including a monoclonal antibody, and other conventional antibody formats, or any other pharmaceutically available agent that binds a cell surface protein to control an immune response, thus acting as a checkpoint inhibitor (CPI), which can block, reduce and/or inhibit PD-1 and PD-L1 or PD-L2 and/or the binding of PD-1 with PD-L1 or PD-L2. Alternatively, the antibody may target cancer antigens for which therapeutic antibodies are presently used or developed in a clinical context. Examples of such antigens are PDGFRalpha, Her2, EGFR, VEGFR2, RANKL, CD38, EpCAM, VEGFA, CEA, CD40, and CD25 (Corraliza-Gorjón I *et al.,* 2017), and their corresponding protein/DNA coding sequences, that are available in databases and/or in the literature.

The DNA sequences that are introduced in CLOSTRA-T genome can be identical to those originally disclosed (or natural ones), but they can be modified and adapted to the Clostridia biology and/or the use of CLOSTRA-T strain. For example, the codon usage within the cloned sequence can be optimized to improve the transcription and translation in *Clostridium* strains of the corresponding protein, as described in the literature for a series of human or non-Clostridia genes that are expressed in *Clostridium* strain as recombinant proteins. Indeed, specific software can be used at this scope, such as JCat or Upgene (Gao W et *al.,* 2004; Grote A *et al.,* 2005; Alexaki A., *et al.,* 2019). Alternatively, when the natural sequence contains a signal sequence for secretion by eukaryotic cells, such signal sequence can be deleted or otherwise mutated to permit the correct transcription and translation in *Clostridium* strains of the corresponding protein. As a further alternative, the originally disclosed or natural DNA sequence is modified to include a tag sequence or other functional sequence within the recombinant protein that is later expressed by CLOSTRA-T strain.

As shown in the Examples, the CLOSTRA-T may contain more than one HetSeq[n], each having a different activity (*e.g.*, a prodrug converting enzyme and a cytokine), each integrated with a different plasmid and Clostra Cassette in sequential steps of CLOSTRA genome modification. The coding sequences that can be cloned in a Clostra Cassette for generating the generic pPM-1nn, pPM-1nnH, and pPM-1nnS plasmids (as well as pPME-100 derivative plasmids) and modify the genome of CLOSTRA and CLOSTRA-Derived Strains may express the human sequence (or the corresponding rodent sequence, when a validation in a rodent model is needed) for a cytokine of therapeutic interest, as a full sequence or only the mature sequence fused in at 5' end with codons coding for a starting methionine and/or other linker sequences. Examples of such cytokine sequences are Interleukin 2 (IL-2; mouse sequence UniProt code P04351; human sequence UniProt code P60568), Interleukin 10 (IL-10; mouse sequence UniProt code P18893; human sequence UniProt code P22301), or Interleukin 15 (IL-15; mouse sequence UniProt code P48346; human sequence UniProt code P40933). Alternatively, the sequence can be the one of therapeutic antibody (or any functional fragment thereof, such as ScFv, Fab, or nanobodies) that can be expressed and exert its biological activity in the same location where the CLOSTRA-Derived Strains replicate, as shown previously by oncolytic *Clostridium* strains expressing functional antibodies (Groot AJ *et al.,* 2007). Exemplary sequences are those coding for heavy and light chains of anti-PD-1 antibody Pembrolizumab (KEGG Drug code D10574 and other antibodies under DrugBank codes DB09037, DB09035, DB11595) of an anti-PD-L1 antibody (such as those under DrugBank codes DB11714, DB11495), or of anti-CTLA-4 antibody Ipilimumab (KEGG Drug code D04603).

"CLOSTRA-SA" refers to a CLOSTRA strain, or CLOSTRA cells, combining the functional properties and the genome modification of both CLOSTRA-S and CLOSTRA-A, which is produced by modifying CLOSTRA with at least two distinct Clostra Cassettes, whereby either CLOSTRA-S or CLOSTRA-A is first generated and later modified, in any order. The CLOSTRA-SA phenotype and genome can be used as a platform for subsequently adding one or more Clostra Cassettes for obtaining the corresponding CLOSTRA-SAT expressing (non-) Clostridial genes. In particular, the choice of the Locus" for integrating at least two distinct HetSeq" (or even three, if the CLOSTRA-A combines the inactivation or deletion of two distinct Clostridial genes not related to the sporulation process, such as in CLOSTRA-A2) may define and provide specifically improved CLOSTRA-SA strains to be used as a platform to insert into different HetSeq" according the desired use and features of a panel of CLOSTRA-SAT strains, sharing the same sporulation and biological features for the selection and manufacturing of CLOSTRA-Derived Strains and CLOSTRA-Derived Products.

"CLOSTRA-AT" refers to a CLOSTRA strain, or CLOSTRA cells, combining the functional properties and the genome modification of both CLOSTRA-T and CLOSTRA-A, produced by modifying CLOSTRA with at least two distinct Clostra Cassettes, whereby either CLOSTRA-T or CLOSTRA-A is first generated and later modified, in any order. CLOSTRA-AT phenotype and genome can be used as a platform for then adding one or more Clostra Cassettes for obtaining CLOSTRA-SAT with appropriate conditional sporulation features and induction systems.

"CLOSTRA-ST" refers to a CLOSTRA strain, or CLOSTRA cells, combining the functional properties and the genome modification of both CLOSTRA-T and CLOSTRA-S, which is produced by modifying CLOSTRA with at least two distinct Clostra Cassettes, whereby either CLOSTRA-T or CLOSTRA-S is first generated and subsequently modified, in any order. CLOSTRA-ST phenotype and genome can be used as a platform for then adding one or more Clostra Cassettes for obtaining CLOSTRA-SAT, with an appropriate attenuation or inhibition of a CLOSTRA gene.

"CLOSTRA-SAT" refers to a CLOSTRA strain, or CLOSTRA cells, presenting the functional properties and the genome modification of CLOSTRA-A, CLOSTRA-T and CLOSTRA-S, which is produced by modifying CLOSTRA with at least three distinct Clostra Cassettes, starting from any of CLOSTRA-SA, CLOSTRA-AT, and CLOSTRA-ST, in any order.

"CLOSTRA-Derived Strains" refers to a CLOSTRA strain, or CLOSTRA cells, presenting the functional properties and the genome modification of any of CLOSTRA-A, CLOSTRA-T CLOSTRA-S, CLOSTRA-SA, CLOSTRA-AT, CLOSTRA-ST, and CLOSTRA-SAT, in any order and independently from the Clostra Cassette(s) used to modify a CLOSTRA genome.

"CLOSTRA-Derived Spores" refers to any derived spore preparation obtained from the CLOSTRA-Derived Strains, in particular the spore-containing preparations indicated as CLOSTRA-SAS, CLOSTRA-STS, CLOSTRA-ATS, and CLOSTRA-SATS.

"CLOSTRA-Derived Formulations" refers to a cell or spore formulation amenable for industrial and/or therapeutic use, as obtained from CLOSTRA-Derived Strains or CLOSTRA-Derived Spores, especially the spore-containing preparations indicated as CLOSTRA-SAF, CLOSTRA-STF, CLOSTRA-ATF, and CLOSTRA-SATF.

"CLOSTRA-Derived Products" refers to CLOSTRA-Derived Strains, CLOSTRA-Derived Spores, CLOSTRA-Derived Formulations, as well as extracts, fractions, and the genetic elements isolated from them. The CLOSTRA-Derived Products can be provided as purified and/or concentrated preparations or formulations that can be stored or used directly. In particular, due to additional requirements related to use or storage, such preparations may further comprise, for example, pharmaceutically acceptable biological or chemical components such as drugs, additives, salts, or excipients. In addition, CLOSTRA-Derived Products can be provided in various alternative formats, such as liquid, solid, frozen, dried, and/or lyophilized formats, depending on the desired storage, use, or administration.

*(ii) Vectors and technologies for producing CLOSTRA-Derived Strains*

As described herein according to the Examples, the disclosed vectors and representative implementing technologies can be adapted for integrating DNA within the genome of an obligate anaerobic microorganism such as CLOSTRA in general (as described in the literature) and also CLOSTRA-Derived Strains. Preferably, such vectors and methods are selected from a CRISPR/Cas system, Cre/Lox system, TALEN system, and homologous recombinationi-based mechanisms in general. Additional details regarding the disclosed sequences, cloning technologies, and assembly of such vectors as a platform can be found in the literature (Nora L *et al.,* 2019).

These methods may optionally comprise use of an exogenous an antibiotic resistance gene or other nucleic acid encoding a selection marker conferring a selectable phenotype in CLOSTRA or CLOSTRA-Derived Strains. The modified selectable marker gene may comprise a region encoding a selectable marker and a promoter operably linked to said region, wherein the promoter causes the expression of the selectable marker encoded by a single copy of the modified selectable marker gene in an amount sufficient for the selectable marker to alter the phenotype in the CLOSTRA-Derived Strains such that it can be distinguished from CLOSTRA lacking the modified selectable (or counter-selectable) marker gene, including an antibiotic-resistance gene, a gene encoding a specific metabolic enzyme that utilizes a special nutrient substitute, a gene encoding an enzyme that catalyzes a chemical compound to form a distinctive color, a gene encoding a fluorescent protein, and a gene that encodes a protein with specific affinity for another molecule, heterologous toxin, or an antisense RNA. These markers allow the tracing and also the shuttling of the plasmid between the *Escherichia coli* cloning microorganism and CLOSTRA, or CLOSTRA-Derived Strains, via conjugative transfer from *Escherichia coli* to CLOSTRA.

The transformation, and genetic modification of CLOSTRA may involve the replacement of a target DNA sequence (*e.g.,* Locus[n]) by homologous recombination in the CLOSTRA genome, and comprise transforming this strain with a vector comprising an origin of replication(s) permitting its replication in CLOSTRA and preferably in other microorganism (such as *E. coli),* and a Clostra Cassette comprising sequences homologous to selected regions around the target DNA sequence. In particular, the CRISPR/Cas9 technology and double-crossover, homologous recombination-mediated chromosomal integration allows the recombination of HetSeq[n] within the Clostra Cassette and CLOSTRA genome, independent from any natural homologous recombination system in *Clostridium* species. This approach may be performed successively two or more times using appropriate plasmids in a specific or any order, as detailed in the Examples using the exemplary pPME-100 derived plasmids.

The sequence of promoters, coding sequences and other sequences in the plasmid may be also optimized for the specific CLOSTRA gene expression profile, metabolism and biology, for example, wherein the codon usage of the polynucleotide has been optimized. Moreover, CLOSTRA may also present specific features enhancing frequency and/or efficiency of homologous recombination, due to altered or missing genes involved in CLOSTRA homologous recombination. Typically, this process is possible due to sequences present in a Clostra Cassette region that is at least 70%; 80%, 90%,, 95%, 99% or more identical to the region downstream and upstream of Locus$^n$ within CLOSTRA or CLOSTRA-Derived Strain, and such homologous sequence is at least about 100, 250, 500, 750, 100, 1500 bases or more nucleotides.

The site-specific changes in the CLOSTRA strain may involve the use of the Cas9 enzyme (*e.g.,* as identified and cloned in *Streptococcus pyogenes* and other microorganisms) that may be introduced in the cells using the same plasmid containing the sequences to be introduced in the CLOSTRA genome *(*e.g., in the pPM-1nn plasmid, and in particular the pPME-100 derived plasmids shown the Examples) or by using two distinct plasmids. As used herein, the Cas9 enzyme may exploit one or more DNA sequences that are repetitive sequences associated with the endogenous C̲lustered R̲egularly I̲nterspersed S̲hort P̲alindromic R̲epeats (CRISPR) or one or more contiguous DNA sequences from the CLOSTRA genome or CLOSTRA-Derived Strains.

The present vector can be introduced into CLOSTRA and CLOSTRA-Derived Strains using a DNA delivery technique appropriate for *Clostridium* species, in particular selected from conjugation, DNA-calcium phosphate coprecipitation, general transduction, liposome fusion and protoplast transformation. In this manner, the Locus" can be modified, wherein the Locus" can be any of the following coding or non-coding sequences (or specific fragments comprised in them): promoters, operons, genes encoding a sporulation factor, a metabolic enzyme, a transcription regulatory protein, a cell growth factor, a toxin, a cell stress response protein, or cell replication factor.

In particular, a CLOSTRA-S Derived Strain or other CLOSTRA-Derived Strain that does not undergo spontaneous, natural sporulation, results from genome modifications that alter the structure, the function, and/or the control of gene expression for at least one sporulation gene, preferably *spo0A, spoIIE, spoIIAA, spoIIAB, spoIIR, spoIIGA* or as selected from *Sigma F, Sigma E* and *Sigma* G, and more preferably, *spo0A or spoIIAA.* The following codes can be used for identifying the sporulation gene common to *Clostridium* species to be modified and made inducible in CLOSTRA-S genome: *spoIIR* (stage II sporulation protein R), *spoIID* (stage II sporulation protein D), *spoIIGA* (stage II sporulation protein GA), *spoIIAB* (stage II sporulation protein AB), *spoIIAA* (stage II sporulation protein AA), *spoIIE* (stage II sporulation protein E), *spo0A* (stage 0 sporulation protein A), and any corresponding stage III sporulation protein.

The modification of the sporulation gene expression preferably leads to a conditional sporulation process either *in vivo* or *in vitro* that results from the induction or repression of the sporulation gene. Such control may result from the substitution and/or disruption of the sequences that control the expression of such gene directly, or indirectly (by altering the expression of one or more specific genes in signaling cascade leading to sporulation). The mutation or other genome modification induced in CLOSTRA-S derivative strains may be the disruption and/or the substitution of Clostridial gene promoters using conventional CRISPR/Cas9 techniques, homologous recombination, or any other technology compatible with the Clostridia biology and genome, with (non-) Clostridial sequences that are functionally active in CLOSTRA strain (*e.g.* by knocking-in, knocking-out, substituting, eliminating DNA sequences), and/or with or without modifying the sequences coding for such sporulation gene.

Preferably, the inducible or repressible sporulation phenotype results by exposing cells or spores of the CLOSTRA-S derivative strains to specific compounds (such as sugars, antibiotics, gases, or other chemicals) or conditions (such as temperature or pH). This deliberate control over the sporulation process can be performed according to manufacturing requirements, or generally accepted cell culture conditions, and more importantly, to the contemplated final use, for instance, after *in vivo* administration. CLOSTRA-S derivative strains are preferably unable to sporulate, or present limited sporulation activities following administration in humans, and are able to sporulate only in conditions reproducible in cell culture and in manufacturing the CLOSTRA strain. Among the known inducer or repression systems that are active in *Clostridium* strains and that can allow a proper control levels for a sporulation gene and consequently a conditional sporulation in any CLOSTRA-S derivative strain, two main mechanisms can be exploited. As a main approach, the regulatory sequence may be activated by compounds such as metals, chemicals, or sugars that can be added in cell culture media such as arabinose (Zhang *J et al.,* 2015), lactose (Hartman AH *et al.,* 2011), xylose (Nariya H *et al.,* 2011), or similar, non-native derivative RNA polymerase binding sequences. Alternative, more sophisticated approaches targeting gene transcription, post-transcriptional control or post translational control, may involve the cloning of silencing RNA, specific enzymes, and/or synthetic riboswitches that control gene expression in diverse bacterial species, including *Clostidium* species, and are based on the use of compounds such as Theophylline and/or lactose in cell culture (Topp S et al., 2010; Cui W *et al.,* 2016; Canadas I *et* al., 2019). Depending on the CLOSTRA genome and the later manufacturing and use of CLOSTRA-Derived Products, one or more of these systems can be combined for achieving the desired level of sporulation control, as shown in the Examples with the CLOSTRA strains identified as CLOSTRA-S3 and CLOSTRA-S4.

The pPM-1nn plasmids, the Clostra Cassettes, and the CLOSTRA-Derived Products (including any pPME-100 derivative plasmids) can be produced in accordance to the protocols applicable to *Clostridium* species in general, but more specifically to the strict requirements for using such biological products in a clinical or industrial context, for instance, using the equipment and protocols pertaining to biocontainment, storage, transport, elimination, experimental manipulation, and also uses of genetically modified microorganisms.

*(iii) Pharmaceutical Compositions, Uses, and Methods*

The pharmaceutical compositions comprising the CLOSTRA-Derived Products according to the present invention (such as CLOSTRA-SAS, CLOSTRA-ATS, CLOSTRA-STS, CLOSTRA-SATS, CLOSTRA-SAF, CLOSTRA-ATF, CLOSTRA-CLOSTRA-STF, and CLOSTRA-SATF) can be preferably used as a medicament, and in particular for the treatment of indications including cancer, and more preferably those solid cancer types in which CLOSTRA-Derived Products that are administered *in vivo* are accumulated due to the tropism towards hypoxic areas present within a tumor depending on its size, stage, location, and/or origin. In particular, the CLOSTRA strains presenting conditional sporulation and/or alternative gene expression controls can also express at least such prodrug converting enzymes, with or without expressing, in a inducible or basal expression system, another therapeutic agent (including an antibody, a cytokine, a cell growth factor, or a toxin), which also contributes to the overall therapeutic effect.

The pharmaceutical compositions of the invention can contain CLOSTRA cells or spores in an amount that is evaluated in terms of biological and/or therapeutic activity, and containing calculated concentration of CLOSTRA cells or spores per a given unit, for instance in a range between 10 to $10^{15}$ or more CLOSTRA spores or cells per dose, per ml, or per mg (and typically between $10^5$ to $10^9$ spores). The concentration of CLOSTRA cells or spores may be also defined as a ratio with respect of the concentration of with pharmaceutically acceptable carrier, vehicle, diluent, additives, excipients, solvents, adjuvants, or other compound and drug that is also included in the formulation (*e.g.* $10^5$-$10^8$ spores per mg of a drug such as Docetaxel), or alternatively in the format of colony-forming units (CFU) per dose or per kg.

The biological effects of therapeutic interest of CLOSTRA-Derived Products may be detected and defined using a variety of physiological criteria (such as changes in the combined secretion of chemokines, cytokines, interferons, etc., or in the expression of specific cell surface receptor or transcription factors). Such findings, when compared to clinical or therapeutic effects in cancer models or patients that are treated (or are a potential candidate of treatment) with a another *Clostridium*-based treatment in parallel or in comparable situations, advantageously permit the identification of novel clinical uses, methods of treatment, and drug regimens that may benefit from treatment using CLOSTRA-Derived Products, alone or in combination (simultaneously or separately, in any sequential order) with other drugs, including anti-cancer drugs (such as current standard-of-care agents, antibodies against cancer antigens, or cancer immunotherapies), or antigens (human or non-human ones, such as in vaccination), either during the course of treatment or prior to treatment with the CLOSTRA-Derived Products.

In CLOSTRA-Derived Products that also express a prodrug converting enzyme (PCE), use of such CLOSTRA-Derived Product may be beneficial in directed enzyme prodrug therapy (DEPT) in general, and specifically Clostridium-directed enzyme prodrug therapy (CDEPT). Exemplary PCEs, Prodrugs, and PCE-based strategies for treating cancers are described in the literature (Rautio J *et al.,* 2018; Hamada Y, 2017; Williams EM *et al.,* 2015). A reference PCE can be YfkO nitroreductase from *Bacillus licheniformis* (Emptage CD *et al.* 2009). A method of treating a solid tumor cancer in a subject may comprise the administration of an CLOSTRA-Derived Product expressing a polypeptide having nitroreductase activity and/or the spores thereof to the subject, wherein the CLOSTRA-Derived Product colonizes a tumor in the subject, wherein the prodrug is administered and activated within the tumor. CLOSTRA-Derived Products that proliferate in the hypoxic and/or necrotic environment can be used in methods for activating or targeting an anticancer drug (such as a chemotherapeutic agent) to a tumor in a tumor-bearing individual, comprising: (a) administering a CLOSTRA-Derived Product that expresses one or more enzymes effective in modifying a drug; and (b) systemically administering a prodrug that is converted at the tumor site into such anticancer agent by the enzyme produced by the microorganism. Such methods involve the administration of compositions comprising the CLOSTRA-Derived Product where the prodrug converting enzyme is either naturally expressed by CLOSTRA genome or, preferably, introduced by means of Clostra Cassette and recombinant vectors, including those described in the representative Examples.

These methods require suitably combining the prodrug to be administered and the prodrug converting enzymes *in vivo* by the administered CLOSTRA cells or spores. The prodrug can be selected from a glucuronide (such as a glucuronide conjugate of drugs such as epirubicin, doxorubicin), and original forms of chemical derivatives of drugs such as etoposide phosphate, doxorubicin phosphate, mitomycin C phosphate, derivatives of nucleotides (such as 5-fluorocytosine or 5-fluorouracil), hydroxyanaline mustard and other mustard-based drugs, and 4-hydroxycyclophosphamides. The corresponding prodrug converting enzyme can be a cytosine deaminase, a nitroreductase, a β-glucuronidase, a β-galactosidase, a carboxypeptidase, an alkaline phosphatase, or a β-lactamase, in particular targeting a nitro functional group in the prodrug.

The present invention further allows defining methods of treatment and medical regimens that may benefit from an administration of a CLOSTRA-Derived Product, such as the disclosed CLOSTRA-Derived Spores and formulations containing them, suitable for a given pathology (*i.e.* a specific type of cancer according its pathophysiology, metastatic properties, location, stage, etc., or a specific vaccination approach), populations of candidate patients (*i.e.* as defined by prior treatments, ongoing standard-of-care or other treatment paradigm, immune profile, altered copy number, type of infection, genetic mutations or activation of relevant genes), and/or combination with specific adjuvants (*e.g.* flagellin or other chemical or biological compound with comparable functional features) and other drugs such as checkpoint inhibitors, TLR agonists (*e.g.* TLR3 agonists, TLR8 agonists, or TLR9 agonists), antibodies targeting cancer antigens, small peptide inhibitors (*e.g.* for specific proteases), small molecules inhibiting kinases or signaling proteins, cancer vaccines, non-human antigens, or adoptive cell therapies. These alternative, novel therapeutic regimens may involve the administration of a CLOSTRA-Derived Product according to the invention, by injection into the blood stream (so that different tissues may be exposed to the formulation) or at specific physiological locations, such as directly in an organ, skin, and/or pathological altered tissues, such as cancer lesions. In the latter case, administration of a CLOSTRA-Derived Product may be performed in the same lesion (if still present) or another lesion if the original lesion is no longer present (through systemic immune activation or other mechanism), or as evaluated by clinicians in view of other clinical parameters such as a suitable clinical response defined by the analysis of biomarkers, gene expression signatures, cancer antigens, immune cells, or clinical criteria (*e.g.* tumor burden, stage, metastasis, and/or recurrence). The evaluation and/or control of clinical therapeutics according to criteria officially defined by various regulatory agencies (such as FDA, EMA, WHO and ICLIO), provide a series of criteria for evaluating a response to a drug treatment in cancer patients (such as stabilization, regression or elimination of a tumor), using any appropriate technologies (*e.g.* PET and Imaging), in general or for specific types of cancers (*e.g.* solid cancers), as described in the literature (Eleneen Y and Colen RR, 2017; Subbiah V et *al.,* 2017; Rossi S et *al.,* 2017).

Representative examples of a therapeutic combination according to the present invention may involve the administration of a CLOSTRA-Derived Product together with a cytokine (such as IL-2, IL-10, or IL-15) or an antibody anti-PD-1 and anti-PD-L1 antibodies (anti-PD1/PD-L1), whose efficacy as an immunosuppressive, pro-inflammatory, or otherwise cancer immunotherapeutic agent may be improved (or simply made possible) by modifying an existing regimen for such drugs (involving regular intravenous injections of the antibody), by including the administration of the instant CLOSTRA-Derived Product, preferably injected intravenously or intratumorally into one or more tumor lesions, one or multiple times. With respect to antibodies directed to cancer antigens (e.g. CD20, CD25, CD38, CD40, HER2, or EGFR), primary examples are those approved (or under validation and review) by regulatory authorities like EMA (in Europe) or FDA (in USA) and targeting cell surface proteins, in particular for treating solid tumors and whose therapeutic activity and/or clinical use may be improved by the combined administration with a CLOSTRA-Derived Product. Similar approaches can be applied when the additional drug to be administered is a cytokine, a chemotherapeutic agent, and/or other cell-based treatment (*e.g.,* adoptive cell transfer or CAR-T). The anti-cancer monoclonal antibody may be conjugated, with a radioisotope or a drug-activating enzyme, optionally wherein the radioisotope is rhenium-188, rhenium-186, fluorine-18, yttrium 90, or iodine-131.

The therapeutic or prophylactic effects observed following administration of the compositions disclosed herein results from exploiting the cell targeting and gene expression activities of obligate anaerobic CLOSTRA cells and spores defined as CLOSTRA-ATS, CLOSTRA-STS, CLOSTRA-SATS, CLOSTRA-ATF, CLOSTRA-CLOSTRA-STF, and CLOSTRA-SATF that proliferate, are biologically active, and germinate *in vivo* according to the genome modifications that contain at the level of sequences, coding for recombinant (non-)Clostridial genes and/or of sequences controlling the expression level of such (non-) Clostridial genes. The analysis of data obtained by exposing cell preparations, tissues, organs, animals, organoids, human or clinical samples (such as biopsies, blood or plasma preparations) or other biological samples or extracts indicative of a pathology (such as cancer or a viral infection) to the disclosed CLOSTRA-Derived Products advantageously permitted defining which distinct biological targets and effects are a direct result of, or associated with, the physiological activities that the disclosed CLOSTRA-Derived Products exerted on such cells (such as apoptosis, autophagy, activation, proliferation, or other) or that such cells later exerted in the human body (within the tumor, in lymph nodes, in blood, etc.). The present Examples demonstrate how representative CLOSTRA-Derived Products can be beneficially used in cell-based models (using cell lines, primary cell preparations or co-culture systems such as organoids), animal models (*e.g.* relevant studying cancer pathology and cancer treatments).

Exemplary biological features that can be determined and/or evaluated in these models by means of CLOSTRA-Derived Products include cytotoxicity, necrosis, differentiation, apoptosis, autophagy, (in)activation, cell migration outside or inside specific tissues or organs, proliferation, integrity of the extracellular matrix, proliferation, viability, differentiation, immunological response (as determined on the basis of the exposure to a specific infectious agent, or capacity to infect or immunize in response to such agent, and/or changes in the number or features of antigen-specific or non-specific immune cells such as B cells, T cells, or NK cells), effects on the microenvironment of the tumor, arresting growth, regressing or destroying a solid tumor, expression of enzymes (such as proteinases, lipases and collagenases), time of spore colonization, oncolysis, tumor volume, systemic toxicity, animal survival, number of spores, or other measurable effects that the CLOSTRA-Derived Products exert within the tumor, in blood, or elsewhere.

The present invention further provides medical methods and uses that may combine different routes of administration of a CLOSTRA-Derived Product, for instance, one or more intratumoral (or peritumoral) injections, followed by one or more sub-cutaneous, intravenous, intranodal, or intramuscular injections over a time period, such as one or more weeks. The composition dosage, in particular with respect to the content of CLOSTRA-Derived Spores, can be adapted for each type of administration, regimen (e.g. highest for intratumoral or intrahepatic injection, lower for subcutaneous or intramuscular injection), and/or with the administration of one or more additional drugs (*e.g.,* in a combination therapy).

Also provided herein are methods for making a pharmaceutical composition comprising a CLOSTRA-Derived Product, comprising mixing a CLOSTRA-Derived Product and one or more of a pharmaceutically acceptable adjuvant, diluent, carrier, or excipient thereof. Such components can be adapted according to the specific medical indication being treated (*e.g.* a solid cancer or a hematological cancer), and/or according to the administration means *e.g.* by injection (peritumoral, intratumoral, intraocular, intranodal, intrahepatic, intraspinal, intrapancreatic, intramuscular, or subcutaneous injection), by inhalation, topically, or by oral administration. In the latter case, the oral formulation may be intended to treat diseases of the gastrointestinal tract or depending from nutrition and/or intestinal microbiome (such as irritable bowel disorders, infections, cancer, neurodegenerative disorders, diabetes, amongst others).

Additional embodiments relating to salts, pharmaceutical compositions and doses for CLOSTRA-Derived Products that can be used according to the present invention are generally described in reference literature such as in Remington's Pharmaceutical Sciences (edited by Allen, Loyd V., Jr; 22nd edition, 2012). CLOSTRA-Derived Products may conveniently be presented in unit dosage forms, whereby such dosage forms can be prepared by any of the methods known in the art of pharmacy and of cell-based pharmaceutical preparations. Administration of the CLOSTRA-Derived Products as pharmaceutical formulation can be carried out continuously, or according to one or more discrete dosages within the maximum tolerated dose, adapting, as needed, any other drug or standard-of-care treatment, such as a radiotherapy, chemotherapy (*e.g.* mitomycin C, vinorelbine, or docetaxel), tumor hyperthermia, or chemicals such as paclitaxel, topotecan, DNA-altering drugs, carboplatin, antimetabolites, gemcitabine, drugs that prevent cell division, vincristine, anti-angiogenic or vascular targeting agents (*e.g.* dolastatin), and kinase or phosphatase inhibitors (*e.g.* pazopanib, olaparib).

Optimal administration rates for a given set of conditions can be ascertained by those skilled in the art using conventional dosage administration protocols. Individual dosages of the agents described herein and/or pharmaceutical compositions of the present invention can be administered in a unit dosage form including pre-filled syringes or vials that contain CLOSTRA-Derived Products at a predetermined concentration and in a therapeutically useful amount. For instance, each unit dosage form may contain from 10 to $10^{15}$ CLOSTRA cells or spores, or an alternative amount, for example, from about 0.001 mg to about 1,000 mg of a CLOSTRA-Derived Product, *e.g.* preferably about 0.1 mg to about 100 mg, inclusive of all values and ranges therebetween. In some embodiments, the agents and/or pharmaceutical compositions described herein may be administered more than once daily, about once per day, about every other day, about every third day, about once a week, about once every two weeks, or about once every three weeks, to be repeated for two or more cycles of administration, as described in the literature (Theys J *et al.,* 2006). Each cycle comprises two or more administrations and/or associated with other regular, standardized, or cyclic therapeutic regimens (such as radiotherapy, chemotherapy, immunotherapies, or other treatments, in particular state-of-art cancer therapies). When a CLOSTRA-Derived Product is administered by intravenous, intrahepatic, intracutaneous or intratumoral injection, the total amount of composition can be adapted consequently.

The methods of treatment and uses of CLOSTRA-Derived Products according to the present invention relate to treating or preventing a cell growth disorder characterized by an abnormal growth of human or animal cells, preferably cancer, and most preferably solid cancers or lymphomas. In a further preferred embodiment, the methods of treatment and uses of CLOSTRA-Derived Products as a pharmaceutical composition formulated as an injectable, aqueous composition (optionally comprising a pharmaceutically acceptable carrier, excipient and/or adjuvant) to an organ or a tissue in a healthy state, presenting a disease related to a exogenous pathogenic agent (such a bacteria, a virus, and infections in general), or presenting an alteration due to a cell growth disorder characterized by an abnormal growth of human or animal cells for instance, due to cancer (that is, involving tumorigenic transformation, metastasis, toxic compound), or a gynecological disorder characterized by abnormal growth of cells of the female mammal reproductive organs). Preferably, the methods of treatment and uses of the CLOSTRA-Derived Products according to the invention are intended for inducing (directly or indirectly) the death of one or more tumor cells, or suppressing growth of the one or more tumor cells, and further including treating, reducing, ameliorating, or preventing cancer growth, survival, metastasis, epithelial-mesenchymal transition, immunologic escape or recurrence. In some embodiments, the CLOSTRA-Derived Product is used to treat cancers at various stages, *e.g.,* Stage I, or Stage II, or Stage III, or Stage IV. By way of non-limiting example, using the overall stage grouping, Stage I cancers are localized to one part of the body; Stage II cancers are locally advanced, as are Stage III cancers. Whether a cancer is designated as Stage II or Stage III usually depends on the specific type of cancer. Otherwise the cancer can be defined according to the tissue and location suitable for being targeted and treated by direct, intratumoral or peritumoral injection.

According to the present invention, the cancer treated by the disclosed compositions, combinations, regimens, and related methods of administration is one or more of basal cell carcinoma, biliary tract cancer; bladder cancer; bone cancer; brain and central nervous system cancer; breast cancer; cancer of the peritoneum; choriocarcinoma; connective tissue cancer; cancer of the digestive system (including esophageal, stomach, colon, rectal or other gastrointestinal cancer); eye cancer; cancer of the head and neck; glioblastoma; hepatic carcinoma; hepatoma; intra-epithelial neoplasm; kidney, adrenal, or renal cancer; leukemia; liver cancer; lung cancer (*e.g.* small-cell lung cancer, non-small cell lung cancer, lung adenocarcinoma, and lung squamous carcinoma); melanoma; renal cell carcinoma; myeloma; neuroblastoma; oral cavity cancer (lip, larynx, tongue, mouth, and pharynx); pancreatic cancer; prostate cancer; retinoblastoma; rhabdomyosarcoma; cancer of the respiratory system; salivary gland carcinoma; skin cancer; squamous cell cancer; testicular cancer; thyroid cancer; uterine, endometrial, cervical, vulval, ovarian or other gynecological cancer; cancer of the urinary system; lymphoma including B-cell lymphoma, Hodgkin's and non-Hodgkin's lymphoma (NHL; including specific types such as low grade/follicular, small lymphocytic, intermediate grade/follicular, intermediate grade diffuse, high grade immunoblastic, high grade lymphoblastic, high grade small non-cleaved cell, or bulky disease NHL), mantle cell and AIDS-related lymphoma; chronic lymphocytic leukemia; acute lymphoblastic leukemia; Hairy cell leukemia; chronic myeloblastic leukemia; as well as other carcinomas and sarcomas; post-transplant lymphoproliferative disorder (PTLD), as well as abnormal vascular proliferation associated with phakomatoses or edema (such as those that associated with brain tumors). In some embodiments, the cancer is a biliary tract cancer. In some embodiments, the biliary tract cancer is selected from pancreatic cancer, gallbladder cancer, bile duct cancer, and cancer of the ampulla of Vater. In some embodiments, the cancer is liver cancer. In some embodiments, the cancer is colon cancer. In some embodiments, the biliary tract cancer is cholangiocarcinoma and/or an adenocarcinoma. Alternatively, the cancer may be listed among the list of rare diseases, being defined according to the criteria of incidence and/or prevalence as defined in Europe or in the USA, and indicated in the regularly updated lists made available in the website of organizations such as the International Rare Cancers Initiative (http://www.irci.info) or RareCare (http://www.rarecare.eu).

More preferably, the CLOSTRA-Derived Products according to the invention are used in methods for treating solid tumors, such as carcinomas, gliomas, melanomas, or sarcomas. In particular, the CLOSTRA-Derived Products are administered either systemically, or directly within or at a location site near to or at the tumor, such as at the margin of the tumor mass, in the surrounding epithelial cells, lymphatic or blood vessels (*e.g.* by intratumoral or peritumoral injection), or in the abnormally growing cells of female mammal reproductive organs. The cancer may be a dormant tumor, which may result from the metastasis of a cancer. The dormant tumor may also a residual tumor following a surgical removal of a tumor. The cancer recurrence may, for example, be tumor regrowth, a lung metastasis, or a liver metastasis, wherein the methods of treatment and uses according to the invention provides for reducing or blocking metastasis in distant sites that the treated cancer may otherwise have caused.

Additionally, macroscopic examination of organs and skin and microscopic, pathological analysis in either immune-deficient fully immune competent animal models may further indicate the efficacy of the methods and uses according to the present invention. The quantitative data generated in similar studies can be readily compared among the different experimental groups described herein by using the appropriate statistical tests, with and without corrections for multiple testing, to evaluate which therapeutic (in particular anti-tumor) effects are observed following the administration of a CLOSTRA-Derived Product described herein, alone or in combination with another drug.

Furthermore, the disclosed compositions can be administered for supporting vaccines (or by itself as a vaccine product), cytokines, antigens, antibodies, chemical compounds, and other compounds having immunomodulatory activities, for treating and/or preventing cancers (solid or not) or infection, for instance as an adjuvant and/or for rescuing patients poorly responding or resistant to a drug, including agents for cancer immunotherapy, for altering cell metabolism and/or functions (preferably, in immune and/or cancer cells), for modulating DNA expression, replication and/or repair (including drugs that target epigenetic mechanisms), or for standard-of-care therapies (such as chemo- or radiotherapy, or vaccine-based therapies involving cancer or viral antigens). Such additional agent that is co-administered (subsequently, in any order) with a CLOSTRA-Derived Product may improve a method of treatment with respect to the bioavailability, efficacy, pharmacokinetic/pharmacodynamic profiles, stability, metabolization, or other property of pharmaceutical interest not observed when treatment with each compound of pharmaceutical interest is administered alone.

Exemplary cancer indications wherein the methods of treatment and regimens according to the present invention can be pursued by appropriately combining the administration of an agent inhibiting PD-1 (such an anti-PD-1 antibody) and a CLOSTRA-Derived Product, with or without a standard-of care treatment (for instance, radiotherapy, as adjuvant), include, but are not limited to, melanoma, triple negative breast cancer, sarcoma, head-and-neck cancer, colorectal cancer, bladder cancer, renal cell carcinoma, liver metastasis, gastric cancer, prostate cancer, and hepatocellular carcinoma. Such indications may be also treated by administering a CLOSTRA-Derived Product in combination with an anti-PD-L1, an anti-CTLA4, or an anti-OX-40 antibody (or standard-of-care such as radiotherapy) in appropriate regimens.

In some embodiments, the CLOSTRA-Derived Product can be administered with an immune-modulating agent that targets one or more of PD-1, PD-L1, and PD-L2. Preferably, the immune-modulating agent is a PD-1 inhibitor. In some embodiments, the immune-modulating agent is an antibody specific for one or more of PD-1, PD-L1, and PD-L2. Such immune-modulating agent is an antibody, including the non-limiting examples of nivolumab, (ONO-4538/BMS-936558, MDX1106, OPDIVO), pembrolizumab (KEYTRUDA), pidilizumab (CT-011), MK-3475, BMS 936559, MPDL328OA.

A CLOSTRA-Derived Product can be included in kit, as a pharmaceutical composition that may be provided as a liquid solution or a freeze-dried powder for injection. In particular, when containing CLOSTRA-Derived Spores, the kit may also contain a solvent to be mixed with the spores prior to use, wherein the solvent is selected from Ringer's solution, phosphate buffer saline, or other solution compatible with injection in humans, including for treating cancer. The kit may also comprise another drug or prodrug to be co-administered or separately administered.

*(iv) Industrial Compositions, Uses, and Methods*

In addition to the pharmaceutical products, uses, and methods described herein, CLOSTRA-Derived Products may be also advantageously applied in a number of industrial or commercial contexts, in particular where the presently disclosed CLOSTRA-Derived Strains are desired at a high density and/or in connection with efficient, tightly controlled cell culture conditions for producing chemical compounds, acids, and other raw materials offering an improved purity and/or yield.

CLOSTRA-Derived Strains may be genetically modified to express one or more heterologous genes to enhance or inhibit the activity of one or more endogenous enzymes (or adding a further enzymatic property), starting from CLOSTRA already being used in industrial, non-clinical processes, or making a further CLOSTRA suitable for such industrial process. A CLOSTRA-Derived Strain may be modified to associate conditional sporulation to the inhibition or attenuation of activity and/or expression for an endogenous CLOSTRA gene, with or without adding one or more non-Clostridial biological or enzymatic properties. Examples of such properties include the hydrolysis of specific sugars, cellulosic and/or lignocellulosic materials. These materials and a CLOSTRA-Derived Strain may be applied in methods and processes for producing bulk chemical preparations from a variety of raw, organic biomass that are used as a main carbon source. Such carbonaceous biomass source may be (non-)woody plant matter, cellulosic material, lignocellulosic material, hemicellulosic material, carbohydrates, pectin, starch, inulin, fructans, glucans, corn and corn-derived products, sugar cane, grasses, switch grass, sorghum, bamboo, distillers grains, Distillers Dried Solubles (DDS), Distillers Dried Grains (DDG), Condensed Distillers Solubles (CDS), Distillers Wet Grains (DWG), Distillers Dried Grains with Solubles (DDGS), peels, citrus peels, bagasse, poplar, or algae. Prior to, or following, exposure to a CLOSTRA-Derived Strain, the carbonaceous biomass may be treated by an acid, steam explosion, hot water treatment, alkali, catalase, or a detoxifying or chelating agent in order to facilitate and/or improve the fermentation process or the isolation of a chemical, end-product such as, for example, a gas, a biofuel, an alcohol, a solvent, a nutritional element or other factor, and acids of interest relevant to the food, plastic, or other industry. The CLOSTRA-Derived Strain may thus be used in a fermentation vessel where the carbonaceous biomass is introduced and by-products are extracted after a given period of time in order to eliminate irrelevant or toxic compounds, and isolating desired by-products, for example, n-butanol, acetone, isopropanol, lactate, or ethanol.

**EXAMPLES**

**EXAMPLE 1: Preparation of a Clostra Cassette and pPM-1nn Plasmids (as pPME-100 Derivative Plasmids) for Generating and Validating Exemplary Non-Haemolytic *Clostridium* Strains (CLOSTRA-A1)**

*Materials & Methods*

*Bacterial strains cell culture conditions*

**TABLE 1**

| ***Clostridium* species** | **Culture collection number** | **Growth requirements** | **Reference/Purpose** |
|---|---|---|---|
| *C. sporogenes* | NCIMB 10696 | Anaerobic, 37°C | Kubiak AM *et al.,* 2015 |
| | Wild type | | Cooksley CM *et al.,* 2010 |
| *C. butyricum* | DSM 10702 | | Tanner R et al., 1981 |
| | Wild type | | |
| *Escherichia coli* | TOP10 | Aerobic, 37°C | Invitrogen™ (Expression/ plasmid storage strain) |
| | NEB ® Stable competent cells | | NEB Product: C3040 (Expression/plasmid storage strain) |
| | S17-1™ | Aerobic, 30°C | ATCC 47055 (Conjugative donor strain) |

*E. coli* was cultured in LB medium, supplemented where appropriate with chloramphenicol (25 μg/ml), at 37°C with horizontal shaking at 200 rpm. All anaerobic *Clostridium* strains were cultured at 37°C under anaerobic conditions (80 % $N_2$, 10 % $CO_2$, 10 % $H_2$) in a MACS1000 workstation (Don Whitely, Yorkshire, UK) in BFM medium, a solid or liquid medium developed for culturing and obtaining *Clostridia* cells and spores without making use material of animal origin.

**TABLE 2**

| Main components | Concentration Range |
|---|---|
| Peptone, peptidase digest for microbiology from vegetable sources (for example, soybean) | 1-25 g/l |
| Certified animal-free yeast extract (for example, Yeast extract, Novagen® Veggie™) | 1-25 g/l |
| Media component that consumes oxygen and allows growing obligate anaerobes like *Clostridia* (for example, sodium thioglycolate) | 0.1-1.5 g/l |
| Sugar source (for example, dextrose) | 0.01-0.5% |
| Antibiotics, when needed (e.g., thiamphenicol or d-cycloserine) | 0 - 250 μg/ml |

Induction of spore formation for the CLOSTRA-S1A1 strain was induced by the addition of 1-50 mM isopropyl-1-thio-β-D-galactopyranosine (IPTG) to growth media. All strains were stored as frozen stocks in their culture media with 10% glycerol (filter sterilized) in a -80°C freezer. All media, buffers and solutions were prepared in distilled water and autoclaved at 121°C for 15 min or filter sterilized. Except where otherwise specified, media components, antibiotics and other chemicals and biochemicals were supplied by Oxoid, Sigma-Aldrich or Fisher Scientific. Agar plates were prepared by addition of 1% (w/v) Number 1 Bacteriological Agar (Oxoid).

*Preparation of high titre pure spore stocks of CLOSTRA strains.* The CLOSTRA wild type strain was streaked from a frozen spore stock on BMF agar plate in an anaerobic cabinet (model MG1000 Mark II, Don Whitley Scientific Limited) under the following conditions: 80% $N_2$, 10% $CO_2$, 10% $H_2$, 37°C. After 24 hours, 10 ml of anaerobically reduced BFM broth were inoculated with a heavy loop of *Clostridium* cells. After a 48-hour incubation, 10 ml of culture was spun down (5000 g for 10 min), re-suspended in 1 ml of PBS and heat-treated (80°C for 20 min). The 1 ml mixture was inoculated, under anaerobic conditions, into 40 ml of fresh and anaerobically reduced BFM and left for a 48-hour incubation. After 48 hours, the entire 40 ml culture was spun down for 10 min at 5000 g at room temperature, re-suspended in a 2 ml of PBS buffer subjected to heat-treatment at 80°C for 20 min. 2 ml of the heat-treated culture was inoculated into 1 liter of pre-reduced fresh BMF medium in a Schott-Duran bottle.

The CLOSTRA 1-liter culture was left for 72 hours under anaerobic conditions at 37°C, followed by a subsequent 72-hour incubation at sub-optimal 30°C with daily agitation, under previously described anaerobic conditions. Following the incubation process, 1 liter of spore culture was removed from the anaerobic chamber and exposed to environmental oxygen for 24 hours and room temperature. Subsequently, the spore/debris mixture was heat-treated (80°C for 20 min) and cooled to room temperature. An aliquot (300 □l) of the spore samples was plated on pre-reduced BFM plate to examine spore formation titer. Spore mixture was distributed into 250-ml centrifuge bottles (NALGENE 3120-0250) filled to 80% of total capacity (that is 200 ml in each). The spore mixture was then spun down (5000 g, 30 min) and the supernatant discarded. Pellet from each bottle was resuspended in 20 ml PBS and pooled together. Final 100 ml spore/debris mixture was incubated at 4°C for 24 hours and 100 rpm.

The high titer spore suspension, containing spores, dead cells and cell debris was pelleted by centrifugation at 3000 g for 30 minutes at room temperature and then resuspended in 10 ml of a 20% sterile Histodenz solution. Each 1 ml of the 10 ml spore suspension was carefully layered on top of the 30 ml Histodenz gradient (50%) in falcon tubes, taking care not to mix the spores with the gradient medium. The gradient was centrifuged at 4000 g for 20 mins. Vegetative cells and cell debris settled at the interface between the applied suspension and the 50% gradient medium solution, while the spores moved down the gradient medium to form a pellet at the bottom of the tube. The cell debris at the interface was gently discarded alongside the rest of the solution, leaving the spore pellet untouched. Each pore pellet was washed in 10 ml sterile water and finally pelleted and resuspended in 1 ml PBS and pooled together. Resultant 10 ml spore suspension was examined using a bright phase microscope to confirm the purity.

*Amplifying and cloning DNA sequences for exemplary Clostra Cassettes and pPME-100 plasmids.* The main primer sequences that were used for PCR amplification, screening, and sequencing of DNA fragments, according to standard protocols and as described in the Examples, are listed in Table 3.

**TABLE 3**

| Name | Sequence | Description |
|---|---|---|
| Cas9-F (SEQ ID NO:1) | TATAGCGGCCGCTCAGTCACCTCCTAGCTGACTC | Amplification of Cas9 gene from *Streptococcus pyogenes* (Dep. No. DSM 20565); in all pPME-100 derivative plasmids |
| Cas9-R (SEQ ID NO:2) | CCGGTCTCACAAGATGGATAAGAAATACTCAATAGGCTTAGATATCGG | |
| Prom-F (SEQ ID NO:3) | CCGGTCTCATGGTCGTACACTCCCTTTTACTATTTAATTATCTATG | Amplification of module F2 with sgRNA s; in all pPME-100 derivative plasmids (full F2 sequence, SEQ ID NO: 61) |
| sgRNA-R (SEQ ID NO:4) | TATAGACGTCATAAAAATAAGAAGCCTGCAAATGCAGGCTTCTTATTTTTATAAAAAAAGCACCGACTCGGTGCCACTTTTTCAAGTTG | |
| Sag-LHA-F (SEQ ID NO:5) | TATATAGGTCTCGGGAAAATAAAGAAGGACAAGTATTGCTAAATTCTTATGATGTAG | Amplification of LHA$_{sag}$ and RHA$_{sag}$ to generate F3 element targeting Sag operon within CLOSTRA; in pPME-101 |
| Sag-LHA-R (SEQ ID NO:6) | TATAGGTCTCGTCTCTGTTAACCCTCAAATTTATTATACCTAATAATCCTTTTTAATTATTTTCATGTACAC | |
| Sag-RHA-F (SEQ ID NO:7) | TATAGGTCTCGGAGATTTACACAGAGGCCCTTAGTATAGTAATTTTATTTGCAGTAAGTTTCTTTATTATAGG | |
| Sag-RHA-R (SEQ ID NO:8) | TATATAGGTCTCGTGGCCCAAAGTTCACTAATTTTAACTTTAACAAGAAACCTG | |
| Sag-SG-R (SEQ ID NO:9) | TATAGACGTCATAAAAATAAGAAGCCTGCAAATGCAGGCTTCTTATTTTTATAAAAAAAGCACCGACTCGGTGCCACTTTTTCAAGTTG | Insertion of Sag targeting sgRNA, into pPME-101 vector via Sall, AatII restriction sites |
| Sag-SG-F (SEQ ID NO:10) | TATAGTCGACGAGTTAATCCATCTGCAGGAGTTTTAGAGCTAGAAATAGCAAGTTAAAATAAGGCTAGTCCGTTATCAACTTGAAAAAGTGGCACCGAGTCGGTGCTTTTTTTATAAAAA | |
| Upstream-F (SEQ ID NO:11) | TAGCATCAGGAGGAACGAAGATAAAGGC | Colony PCR screen for sag operon knockout mutants in *C. sporogenes* (validation of CLOSTRA-A1 clones) |
| Junction-R (SEQ ID NO:12) | ACCGCCACCTACACTAACACTAACGC | |
| Downstream-R (SEQ ID NO:13) | CCATAAATCTCTCAATATGTCAAAGCCATCAAGTCC | |
| TreO-LHA-F (SEQ ID NO:14) | TATAGACGTCGATAAGTCCTATTATTATTAAAATTTCCTATAAGAAAATATTAATG | Amplification of LHA$_{tre}$ and RHA$_{tre}$ to generate the F3 element targeting Trehalose operon within CLOSTRA; in pPME-101 |
| TreO-LHA-R (SEQ ID NO:15) | CCGGTCTCGGCAGCATTTTCAACTAATTTAATATCTCCAATAATATTACGG | |
| TreO-RHA-F (SEQ ID NO:16) | CCGGTCTCAGATTTAAATTTGTAGAATTTGCACGAAGAAAATAATACTG | |
| TreO-RHA-R (SEQ ID NO:17) | TATAGGCGCGCCCAATAATTTACAAGGTATTTAAGATAAG | |

(continued)

| Name | Sequence | Description |
|---|---|---|
| TreO-SG-R (SEQ ID NO:18) | TATAGACGTCATAAAAATAAGAAGCCTGCAAA TGCAGGCTTCTTATTTTTATAAAAAAAGCACCG ACTCGGTGCCACTTTTTCAAGTTG | Insertion of Trehalose targeting sgRNA, into pPME-102 vector via *SalI, AatII* restriction sites |
| TreO-SG-F (SEQ ID NO:19) | CCGTCGACATACCTCCACAGAACATAAGGTTT TAGAGCTAGAAATAGCAAGTTAAAATAAGGCT AGTCCGTTATCAACTTGAAAAAGTGGCACCGA GTCGGTGCTTTTTTTATAAAAATA | |
| Thl14YfkO-F (SEQ ID NO:20) | CCGGTCTCACTGCCAATAGATAAAATAAAG | Amplification of pthl14-YfkO sequence; in pPME-102 (full Thl14 yfkO sequence, SEQ ID NO: 62) |
| Thl14YfkO-R (SEQ ID NO:21) | CCGGTCTCAAATCATAAAAATAAGAAGCCTGC AAATGCAGGC | |
| CR2-TreO-F (SEQ ID NO:22) | GACTTATTTGGGTTTATTTAAACTACCCCG | Colony PCR screen confirming CLOSTRA-T01 clones |
| CR2-TreO-R (SEQ ID NO:23) | TAGCATTGGACATTATTGTTAAGCATAAGAC | |
| pyrE-LHA-F (SEQ ID NO:24) | TATAGACGTCCCTAAAGAAGGAAATAATGGCA TAAGAAT | Amplification of LHA_{pyr} and RHA_{pyr} to generate the F3 element targeting CLOSTRA pyrE gene to insert mIL2; for pPME-103 and pPME-105 |
| pyrE-LHA-R (SEQ ID NO:25) | CCGGTCTCAATTATTAAATAATTCCCCTTATTT CTTCTAAAGTTTGAATACC | |
| pyrE-RHA-F (SEQ ID NO:26) | CCGGTCTCAATTTAAAAATAAGGAGTGTCTCA AAATAGATTTAATTTTG | |
| pyrE-RHA-R (SEQ ID NO:27) | TATAGGCGCGCCAGTTGTTCCAGATGTTGATG TATACTGCTTATTTG | |
| mIL2-F (SEQ ID NO:28) | CCGGTCTCATAATTTATAAATAAAAATCACCTT TTAGAGGTGG | Amplification of pfdx-nprM3-mIL2 sequence ; for pPME-103 |
| mIL2-R (SEQ ID NO:29) | CCGGTCTCAAAATTTATTATTTATCATCATCAT CTTTATAATCTTGAGGACTTG | |
| pyrE-SG-R (SEQ ID NO:30) | TATAGACGTCATAAAAATAAGAAGCCTGCAAA TGCAGGCTTCTTATTTTTATAAAAAAAGCACCG ACTCGGTGCCACTTTTTCAAGTTG | Insertion of pyrE targeting sgRNA, into pPME-103 vector via *SalI, AatII* restriction sites |
| pyrE-SG-F (SEQ ID NO:31) | TAGTCGACATAGTAGGACCAGCTATGGGGTTT TAGAGCTAGAAATAGCAAGTTAAAATAAGGCT AGTCCGTTATCAACTTGAAAAAGTGGCACCGA GTCGGTGCTTTTTTTATAAAAATA | |
| pyrE-ChR-R (SEQ ID NO:32) | GGAGATATATAATGCTTCAAGTAAATTTATGTG GTAAG | Colony PCR screening confirming CLOSTRA-T02 clones and CLOSTRA-A2 clones |
| pyrE-ChR-F (SEQ ID NO:33) | CTCCCCATAAATAATCTTGTTTTGACGCTAATA TAGC | |

(continued)

| Name | Sequence | Description |
|------|----------|-------------|
| Lspo-LHA-F (SEQ ID NO:34) | CCGACGTCGAAATTGTGAAGGAAAAGACTTAA AAATAGTGGCTTATAG | Amplification of LHA and RHA to generate the F3 element targeting CLOSTRA-A1 spo0A promoter to insert inducible bgaR-bgaL module; for pPME-104 |
| Lspo-LHA-R (SEQ ID NO:35) | CCGGTCTCAGGTCTTAATTATTATCTAATATTC CAGCATCCTTTAGCATC | |
| Lspo-RHA-F (SEQ ID NO:36) | CCGGTCTCAATGGAAGAAACAAAGATCAATGT TATCATTGC | |
| Lspo-RHA-R (SEQ ID NO:37) | TAGGCGCGCCCTTTCCATTATTTATAGTATACC CAAATATATTGTTTATAG | |
| bgaRL-F (SEQ ID NO:38) | CCGGTCTCACTGCCAATAGATAAAATAAAGTC TGCC | Amplification of lactose inducible bgaR-PbgaR-PbgaL.; for pPME-104 |
| bgaRL-R (SEQ ID NO:39) | CCGGTCTCACCATTTTACCCTCCCAATACATTT AAAATAATTATGTATTCATG | |
| LSpo0A-SG-R (SEQ ID NO:40) | TATAGACGTCATAAAAATAAGAAGCCTGCAAA TGCAGGCTTCTTATTTTTATAAAAAAAGCACCG ACTCGGTGCCACTTTTTCAAGTTG | Insertion of spo0A promoter targeting sgRNA, into pPME-104 vector via *SalI, AatII* restriction sites |
| LSpo0A-SG-F (SEQ ID NO:41) | TAGTCGACGTACAACTCCTTTGTCACTGGTTTT AGAGCTAGAAATAGCAAGTTAAAATAAGGCTA GTCCGTTATCAACTTGAAAAAGTGGCACCGAG TCGGTGCTTTTTTTATAAAAATA | |
| spo0A-Chr-R2 (SEQ ID NO:42) | CCTGCAGCTGTATCCGGTATACAAATAGGAGA TAG | Colony PCR screening confirming CLOSTRA-S1A1 clones |
| spo0A-Chr-F2 (SEQ ID NO:43) | GGTCATTTTTAGGTTTATTCCCGTAGACC | |
| pCB102-F (SEQ ID NO:44) | GCAAAATACATTCGTTGATG | Colony PCR screening confirming the loss of pPME-100 derivative plasmids |
| pCB102-R (SEQ ID NO:45) | CTTTATTTATGATTTCATACTTGAC | |
| pyrE-BM2-LHA (SEQ ID NO:48) | CCGGTCTCACTGCTTAAATAATTCCCCTTATTT CTTCTAAAGTTTG | Amplification of LHA$_{pyr}$ and RHA$_{pyr}$ to generate the F3 element targeting CLOSTRA-A1 pyrE gene to insert Tag sequence BM2 for pPME-105 plasmid. The other primers to amplify and build this F3 element are Sag-LHA-F and Sag-LHA-R. |
| pyrE-BM2-RHA (SEQ ID NO:49) | CCGGTCTCAGCAGCCCTCCACTCCCATGGAG GATTTAAAAATAAGGAGTGTCTCAAAATAGATT | |
| bgaL-RbEspo0A-R (SEQ ID NO:50) | CCGGTCTCACCATCTTGTTGTTACCTCCTTAG CAGGGTGCTGCCAAGGGCATC | Primer used with Thl14YfkO-F to amplify modified bgaL-RbE sequence for pPME-106 |
| PSpoIIAA-SG1-F (SEQ ID NO:51) | TAGTCGACGGATTTGGATGAGTAAAAAGGTTT TAGAGCTAGAAATAGCAAGTTAAAATAAGG | Insertion of PspoIIAA targeting sgRNA, into pPME-107 via *SalI, AatII* restriction sites |
| sgRNA-Uni-R (SEQ ID NO:52) | TATAGACGTCATAAAAATAAGAAGCCTGCAAA TGCAGGCTTCTTATTTTTATAAAAAAAGCACCG ACTCGGTGCCACTTTTTCAAGTTG | |

(continued)

| Name | Sequence | Description |
|------|----------|-------------|
| spoIIAA-LHA-F (SEQ ID NO:53) | TATAGACGTCGAGTCTATAAATTTATGAATTTT AAAATATTTTTTAGGTAAG | Amplification of LHA and RHA to generate the F3 element targeting CLOSTRA-S4A2 spoIIAA promoter to insert inducible bgaL-RbE module for pPME-107 |
| spoIIAA-LHA-R (SEQ ID NO:54) | CCGGTCTCAGCAGGTAAAAATTTTTTATATTGA TTATTCTAGAACAC | |
| spoIIAA-RHA-F (SEQ ID NO:55) | CCGGTCTCAATGTATTTAAAATTTGATAAAAAA GCAGACAAAC | |
| spoIIAA-RHA-R (SEQ ID NO:56) | GGCGCGCCTTTAGTTCCCTTCCCCTTTTCTG | |
| bgaL-RbEspoIIAA-R (SEQ ID NO:57) | CCGGTCTCAACATCTTGTTGTTACCTCCTTAG CAGGGTGCTGCCAAGGGCATC | Primer used with ThI14YfkO-F to amplify modified bgaL-RbE sequence for pPME-107 plasmid |
| Chr-spoIIAA-F (SEQ ID NO:58) | CCACTTGGGTATGTTGGAATAGCTGCC | Colony PCR screening confirming CLOSTRA-S4A2 |
| Chr-spoIIAA-R (SEQ ID NO:59) | CCAGATCTTGCCTCTTCCATAAGTTCC | |

The pPM-1nn vectors were designed and assembled as schematically presented in FIGs. 3-5. The Cas9 gene was amplified from *Streptococcus pyogenes* DSM 20565 using Cas9-F and Cas9-R primers, with the resulting PCR product digested with *BsaI* and *NotI*, generating F1 fragment. F2 module, containing promoter driving Cas9, gRNA scaffold and 20 nt sgRNA targeting region was synthesized (Thermo Fisher Scientific), amplified by PCR (using Prom-F and sgRNA-R primers) and digested with *BsaI* and *AatII* restriction enzymes. Left and Right Homology arms represent sequences flanking the chromosomal locus subjected to CRISPR-Cas9 mutagenesis that are cloned in F3 element of pPME-100 derivative plasmids and were amplified using CLOSTRA wild type genomic DNA as the template and the relevant primers for PCR amplification listed in Table 3.

*Locus[n] and HetSeq[n] that are targeted by Clostra Cassettes and Other Sequences Included in pPME-100 derivative plasmids.* Main CLOSTRA sequences targeted for constructing the CLOSTRA-Derived Strains as described in Examples are shown in Table 4.

**TABLE 4**

| Locus[n] name | Location in the *C. sporogenes* NCIMB 10696 genome (CP009225.1) | Example no. |
|---------------|----------------------------------------------------------------|-------------|
| *Sag operon* | From AKC61242.1 to AKC61250.1 | Ex. 1 (CLOSTRA-A1) |
| *Trehalose operon* | From AKC62714.1 to AKC62716.1 | Ex. 2 (CLOSTRA-T01) |
| *pyrE* | AKC64051.1 | Ex. 2 (CLOSTRA-T02) Ex. 3 (CLOSTRA-A2) |
| P*spo0A* | Range: 2,072,794 to 2,073,215 | Ex. 3 (CLOSTRA-S1A1) Ex. 4 (CLOSTRA-S3A2) |
| PspoIIAA | Range: 3,473,550 to 3,473,694 | Ex. 3 (CLOSTRA-S2A1) Ex. 4 (CLOSTRA-S4A2) |

The HetSeq" sequences were designed, codon-optimized where necessary, and synthesized (Thermo Fisher Scientific) with their specific promoters and/or signal sequences. The DNA sequences for HetSeq" were subject to PCR using specific primers (Table 3) using standard protocols; the amplification products were digested and linearly ligated with appropriate LHA and RHA fragments using Golden Gate assembly cloning system (ThermoFisher Scientific) according to the manufacturer's instructions. The resulting DNA sequences were amplified with specific primers and digested using *AscI* and *AatII* restriction enzymes making up the F3 fragment. F1, F2 and F3 fragments were linearly ligated, amplified by PCR and digested with *AscI* and *NotI* restriction enzymes and cloned into a pMTL82151 backbone (Heap J et al., 2009), linearized using the same enzymes before further cloning. F3 is a Homologous Recombination Cassette that is designed to facilitate recombination with CLOSTRA genome in Locus" and comprises sequence homologous to CLOSTRA sequences up- and down-stream of the HetSeq[n] target site. The cassette may or may not contain cargo between the homology arms and consists of two arms. Left homology arm (LHA): the most upstream/5-prime part of the cassette (between 100 bp and 1000bp, such as 750 bp, or more), defines the 5-prime locus of DNA recombination. Right homology arm (RHA): the most downstream/3-prime part of the cassette (between 100 bp and 1000bp, such as 750 bp, or more), defines the 3-prime locus of DNA recombination.

The standard and variable genetic elements present in pPME-100 derivative plasmids are summarized in Table 5, and further include a Cas9 and guide RNA scaffold and a conserved DNA sequence that is transcribed into RNA (but not translated), and is recognized and bound by Cas9 (Jinek M et al., 2012). The variable genetic elements in pPME-100 derivative plasmids are designed and generated based on desired CLOSTRA-Derived Strains as described in the Examples. *AatII* and *SalI* restriction sites allow the modular exchange of the 20nt retargeting sequence (sgRNA) depending on chromosomal targeting region (Locus"). These guide sequences are designed using the CRISPR guide design tool per Benchling (www.benchling.com). The sequence of the sgRNA (single guide RNA), a variable sequence located immediately upstream of, and co-transcribed with, the gRNA scaffold, determines the target site for Cas9 cutting of CLOSTRA genome in Locus".

### TABLE 5

| Main Activity | Name/ symbol | Specific Function and main reference |
|---|---|---|
| For replication/maintenance in microorganisms | pCB102 | Gram-positive replicon, derived from the native pCB102 plasmid of *C. butyricum* NCIMB 7423 (Minton N and Morris J 1981; Collins ME et al., 1985) |
| | pBP1 | Gram-positive replicon, derived from C. botulinum (Minton N et al., 2016) |
| | catP | Antibiotic selection marker. Cloned from the *C. perfringens* transposon element (Bannam TL et al., 1995); catP provides chloramphenicol resistance in *E. coli* and thiamphenicol *Clostridium* strains, respectively, for selecting plasmid-carrying cells |
| | colE1 | Gram-negative replicon, for the replication of the plasmid and segregation into daughter cells in *E. coli* cloning strains |
| | traJ | Transfer gene, for the expression of the genes in the *E. coli* plasmid host required for conjugation |
| | p15a | Gram negative replicon from pACYC184 plasmid, available from ATCC (Cat. No. 37033) |
| Promoters | *araE* | CP002660.1 range: 1480078 to 1480346 |
| | *thl-s* | CP002660.1 range: 3008857 to 3008980 and 3008769 to 3008788 |
| | *fdx* | CP009225.1 range: 86395 to 86475 |
| Main type of HetSeq[n] that are introduced using F3/HRC[t] | Tag | (Non)coding sequences that allow identifying the CLOSTRA genome where the Locus[n] endogenous sequence is deleted and substituted with marker or other non-biologically functional sequence (then identified by PCR, antibody, or any other *in vitro/in vivo* assay) |
| | Enzyme (PCE) | Prodrug converting element consisting of an exogenous gene (with its own regulatory and coding sequences) that, once inserted in CLOSTRA genome and expressed, allows for the modification of a chemical or biological compound into a drug, in particular when CLOSTRA strain replicates in subjects exposed to such a compound and the drug would be useful for subject in the body location where CLOSTRA strain replicates |
| | Drug | Drug-coding element consisting of an exogenous gene (with its own regulatory and coding sequences) that, once inserted in CLOSTRA genome and expressed, allows the production of a biological drug, in particular when the drug would be useful for subjects at the location in the organism where CLOSTRA strain replicates |

**EP 4 775 665 A2**

Representative HetSeq" heterologous, coding sequences that can be inserted in pPME-100 derivative plasmids (SEQ ID NO:55) and integrated in the genome of CLOSTRA-Derived Strains are summarized in Table 6.

**TABLE 6**

| HetSeqⁿ Type | Gene/ Insert Name | Reference, Examples |
|---|---|---|
| gene | *bgaR* | CPE0770 UniProtKB - Q8XMB9_CLOPE (Example 3: Creation of CLOSTRA-S1A1) |
| Enzyme | *yfkO* | GeneBank: TWK17509.1 (Example 2: Creation of CLOSTRA-T01) |
| Drugs | mIL-2 | Immunotherapeutics, UniProtKB - P04351 (IL2_MOUSE) position [21-169] (Example 2: Creation of CLOSTRA-T02)<br>Alternatively the corresponding human sequence IL2 UniProtKB - P60568 (IL2_HUMAN) position [21-153] (Example 4: pPME-108) |

Representative HetSeq" heterologous, non-coding, and regulatory sequences that can be inserted in pPM-1nn vectors for controlling HetSeq" or Locus" expression are summarized in Table 7.

**TABLE 7**

| HetSeqⁿ Type | Gene/ Insert Name | Reference, Examples |
|---|---|---|
| Tag (PM-CB) | BM1 (SEQ ID NO:46) | GTTAACAGAGATTTACACAGAGG (Example 1: creation of CLOSTRA-A1) |
| Tag (PM-CB) | BM2 (SEQ ID NO:47) | GCAGCCCTCCACTCCCATGGAGG (Example 4: creation of CLOSTRA-A2 |
| Promoter | thl-s | CP002660.1 range: 3008857 to 3008980 and 3008769 to 3008788 |
| Promoter | thl13 | CP002660.1 range: 3008857 to 3008980. RBS and spacer region (16 bp from the ATG start codon) were selected experimentally |
| Promoter | thl14 | CP002660.1 range: 3008857 to 3008980. RBS and spacer region (16 bp from the ATG start codon) were selected experimentally |
| Promoter | ptb | CP002660.1 Range: 3228725 to 3228872 |
| Promoter | ptb13 | CP002660.1 range: 3228743 to 3228872, RBS and spacer region (16 bp from the ATG start codon) were selected experimentally |
| Promoter | ptb14 | CP002660.1 range: 3228743 to 3228872 RBS and spacer region (16 bp from the ATG start codon) were selected experimentally |
| Promoter | fdx | CP009225.1 range: 86,395 to 86,475 |
| Promoter | fdx-RsE (RbE) | CP009225.1 range: 86,395 to 86,458 and riboswitch E GGTGATACCAGCATCGTCTTGATGCCCTTGGCAGCACCCTGCTAA GGAGGCAACAA (Topp S et al., 2010; SEQ ID NO: 64) |
| Promoter | fdx13 | CP009225.1 range: 86395 to 86458. RBS and spacer region (16 bp from the ATG start codon) were selected experimentally |
| Promoter | fdx14 | CP009225.1 range: 86395 to 86458. RBS and spacer region (16 bp from the ATG start codon) were selected experimentally |
| Promoter | bgaR-bgaL | BA000016.3 range: 952242 to 952604 |
| Signal sequence | nprM3 | CP009225.1 range: 1,611,069 to 1,611,143 |
| Signal sequence | nprM4 | CP009225.1 range: 1,612,928 to 1,6113,002 |
| | cstA1 | CP009225.1 range: 647,832 to 647,900 |
| | nprM2 | CP009225.1 range: 1,614,791 to 1,614,865 |
| | SH3 | CP009225.1 range: 599,142 to 599,234 |
| Terminator | T1 | CP035785.1 range: 1029153 to 1029229 |
| Terminator | T2 | AE015927.1 range: 1499755 to 1499840 |
| Terminator | T3 | CP042184.1 range: 3601746 to 3601788 |
| Terminator | T4 | M11214.1 range: 354 to 395 |

List of pPM-1nn plasmids and genes cloned to generate different CLOSTRA genotypes is shown in Table 8.

**TABLE 8**

| Name of plasmid | HetSeq[n] | CLOSTRA strain [genotype] | Feature | Example |
|---|---|---|---|---|
| pPME-101 | PM-CB Sequence Tag (BM1) | CLOSTRA-A1 [*C.sporogenes*-Δ (sagA-sagJ)::PM-CB] | Deletion of Sag operon Δ *(sagA-sagJ)* and insertion of PM-CB sequence tag (BM1) at Sag operon | Ex. 1 |
| pPME-102 | P*thl14-yfkO* | CLOSTRA-T01 [*C.sporogenes*-Δ *(treB-treR)*::(P*thl14-yfkO*)] | Deletion of Trehalose operon Δ *(treB-treR)* and insertion of *yfkO* gene controlled by *thl14* promoter | Ex. 2 |
| pPME-103 | P*fdx*-nprM3-mIL2 | CLOSTRA-T02 [*C.sporogenes*-Δ*pyrE*::(P*fdx*-nprM3-mIL2)] | Deletion of pyrE gene (Δ*pyrE*) and insertion of mIL2 (interleukin 2) controlled by P*fdx*-nprM3 (promoter-signal sequence) | Ex. 2 |
| pPME-104 | *(PbgaR-PbgaL)-bgaR* | CLOSTRA-S1A1 [*C.sporogenes*--Δ (sagA-sagJ)::PM-CB-ΔP*spo0A*::(P*bgaR*-P*bgaL*-BgaR)] | Deletion of Sag operon Δ *(sagA-sagJ)* and insertion of PM-CB sequence tag (BM1 at Sag operon and deletion of spo0A promoter (Δ*Pspo0A*) and insertion of BgaR gene under the control of a *PbgaR-PbgaL* divergent promoter | Ex. 3 |
| pPME-105 | BM2 sequence tag | CLOSTRA-A2 | Deletion of pyrE gene Δ*pyrE* and insertion of BM2 sequence tag at pyrE locus in CLOSTRA-A1 (haemolysin deficient) strain | Ex. 4 |
| pPME-106 | *(PbgaR-PbgaL-RbE)-bgaR* | CLOSTRA-S2A2 [*C.sporogenes*--Δ (sagA-sagJ)::PM-CB-ΔP*spo0A*::(P*bgaR*-P*bgaL*-BgaR)] (Full BgaR-bglA-RbE sequence SEQ ID NO:60) | Deletion of constitutive spo0A promoter (Δ*Pspo0A*) and insertion of BgaR gene under the control of a *PbgaR-PbgaL-RbE* promoter with theophylline inducible riboswitch in CLOSTRA-A2 strain (pyrE and Sag-negative) | Ex. 4 |

*Transformation of plasmids of pPME-100 derivative plasmids from an E. coli host into CLOSTRA strains.* Plasmid DNA was transformed into commercially obtained (Invitrogen) and chemically competent One shot TOP10 *E. coli* cells as well as conjugative donor *E. coli* S17-1, according to manufacturer's instructions. Subsequently, the exemplary pPM-1nn plasmids (such as pPME-100 derivative plasmids, being either pPM-1nnH or pPM-1nnS) were introduced into CLOSTRA using a modification of the protocol previously described (Purdy et. al., 2002). Briefly, cells harvested from a 1 ml overnight culture of the *E. coli* S17-1 donor were washed in 1 ml of PBS and centrifuged for 1 min at 5000 rpm. The pellet was carefully re-suspended in 200 μl CLOSTRA strain (previously grown overnight in an anaerobic cabinet). The mixture of donor and recipient cells was spotted onto plain BFM plate and incubated at 37°C for 6-7 hours under anaerobic conditions. Finally, conjugated cells were harvested with a sterile loop and re-suspended in 0.5 ml of sterile, anaerobic PBS and plated in suitable dilutions onto appropriate selective media (containing thiamphenicol). *E. coli* donors were counter-selected by the addition of D-cycloserine (250 μl/ml).

*CRISPR-Cas9 mutagenesis - selection of CLOSTRA-Derived Strains.* CRISPR-Cas9 mutagenesis utilizing pPM-1nn vectors occurs via mechanisms schematically presented in the disclosed Figures. pPM-1nn vectors were transferred to CLOSTRA strains via conjugation from *E. coli* S17-1 donors as described above. Thiamphenicol resistant transconjugant colonies appeared within 24-48 hours and were harvested into 1ml BFM media with 10% glycerol. The 100 □l of harvested transconjugant library was inoculated into 5 ml fresh BFM broth and incubated for 48 hours to facilitate the loss of plasmid. In addition, the 48-hour culture was serially diluted in PBS and plated on non-selective plates to obtain single colonies. After a 24-hour incubation, resultant single colonies were used as the template in colony PCR screens. 2xDreamTaq Green PCR Master Mix was used according to manufacturer's instructions (ThermoScientific, K1081). Desired mutations were confirmed using primer pairs annealing to chromosomal regions flanking the editing template sequences (Table 3). Plasmid specific primers were also used to confirm the loss of pPME-100 derivative plasmids from the mutated strains. Colony PCR products were separated using agarose gel electrophoresis.

*Quantitative analysis of red blood cell breakdown.* The CLOSTRA-A1 strain was subjected to a whole blood assay as published (Totten PA et al., 1995). Briefly, 200 □l PBS-washed cultures were incubated in a 96-well plate with 20 □l whole human blood for one hour. The plates were then centrifuged (200 x g, 10 min) and the supernatants transferred to a new sterile 96-well plate. Haemoglobin release was measured at OD540. *C. sporogenes* NCIMB10696 wild type (CLOSTRA) and group B streptococcus strains were used as controls.

*In vivo animal colonisation study.* Mouse colorectal CT26 cancerous cells were injected subcutaneously into adult immune-competent BALB/c mice. Tumor volumes were determined three times per week using a Vernier caliper. 100 $\mu$l of $1\times10^7$ pure spore suspensions or vehicle (PBS) were administered when tumors reached a volume of ~300 mm$^3$. At the end of the follow-up period, colonization levels were determined in tumors, whole blood and normal tissues (lymph nodes and spleen). In order to determine the levels of vegetative cells (V), all samples were serially diluted in PBS in an anaerobic chamber and plated on BFM agar plates, with the addition of D-cycloserine. To evaluate levels of endospores (S), prior to dilution and plating, all tissue samples were briefly spun down and subjected to heat treatment (80°C, 20 min). All plates were incubated for 24 hours followed by colony enumeration (CFU/ml values).

*Results*

The general strategy presented herein, *i.e.,* to genetically modify the exemplary CLOSTRA strain (as summarized in FIG. 2) was pursued by constructing, introducing in CLOSTRA, and biologically validating a series of plasmids based on the template vector pPM-1nn as described in FIG. 3A. This vector is characterized by a DNA cassette described herein as Clostra Cassette formed by three main elements (F1, F2, and F3), in which the DNA sequence elements to be introduced within the CLOSTRA genome by homologous recombination (*i.e.,* a non-coding sequence to be used as marker or for driving further modifications, or a sequence coding an enzyme, a drug, tag sequence or regulatory sequence such as a constitutive or inducible promoter) are cloned together with the DNA encoding the regulatory and transcribed DNA sequences needed to perform CRISPR/Cas9 genome modification at a specific CLOSTRA Locus" (FIG. 3B). Among the elements cloned in the Clostra Cassette, F3 contains the most variable sequences among pPM-1nn plasmids since it is formed by the heterologous sequence to be integrated in the CLOSTRA genome (HetSeq") that is cloned between the sequences that guide the integration of HetSeq$^n$ at a specific Locus$^n$ (LHA and RHA). The nature and use of HetSeq$^n$ may distinguish between two main categories of pPM-1nn plasmids (FIG. 4A): those intended to modify the regulatory region for a sporulation gene to make it inducible according to the specific use and requirements (pPM-1nnS) and those intended to modify, substitute, or delete any other type of Locus", being a gene (or intergenic sequence), totally or partially, at the level of coding or non-coding region (pPM-1nnH). The combined selection of HetSeq", Locus", and LHA/RHA sequence, and the sequential use of pPM-1nnH and pPM-1nnS plasmids (where Clostra Cassette is assembled) to modify CLOSTRA genome define the relevant phenotypes and functional features of CLOSTRA-Derived Strains as required for future selection, production, and use of the CLOSTRA-Derived Products (FIG. 4B).

As a first step, the exemplary pPME-100 plasmid template was designed and generated by sequentially cloning the Clostra Cassette elements in an appropriate order and orientation, using unique restriction sites, and suitable to generate the desired pPM-1nnS and pPM-1nnH plasmids (FIG. 5A). In order to generate an exemplary CLOSTRA-A1, a tag sequence (generally referred to as PM-CB and exemplified with the sequences BM1 and, in other plasmids, BM2) was cloned into the pPME-101 plasmid (a pPM-1nnH plasmid) between a sequence allowing its integration by homologous recombination using a CRISPR/Cas9 approach within the Sag operon, such that the operon is inactivated in CLOSTRA-A1 (FIG. 5B). When using the pPME-101 plasmid for modifying the *C. sporogenes,* strain NCIMB 10696 as an exemplary CLOSTRA, the resulting clones can be screened using different PCR amplification strategies to identify those presenting the desired DNA modification, i.e. the deletion of sag operon and the consequent functional loss of hemolytic activity (FIG. 6A and FIG. 6B). One of these CLOSTRA-A1 clones was then subsequently validated using a standard in vitro assay for hemolysis, confirming that, when compared to positive and negative controls, CLOSTRA-A1 does not present such activity (FIG. 7A and FIG. 7B). CLOSTRA-A1 was also tested for sporulation and in vivo localization properties, confirming that the spores obtained from CLOSTRA-A1 can be produced and injected intravenously in an animal model presenting a solid cancer colonized by CLOSTRA-A1, where it is found as vegetative cells or spores (FIG. 8A and FIG. 8B).

Thus, the genome of the exemplary CLOSTRA-A1 can be further modified by using pPM-1nn-based plasmids in which another HetSeq" sequence is cloned and is then transferred to be specifically functional and/or regulated in combination with a non- hemolytic phenotype of CLOSTRA-A1, in particular with respect to conditional sporulation (using a pPM-1nnS plasmid) and/or expression of an exogenous coding sequence (using a further pPM-1nnH plasmid).

Moreover, the choice of *C. sporogenes,* strain may help combining other features, for instance on the basis of the metabolites that are released, as those having neuroprotective, or immunoglobulin A-modulatory activity.

**EXAMPLE 2: Preparation of Clostra Cassettes and pPME-100 Derivative Plasmids for Generating Exemplary *Clostridium* Strains Expressing an Exogenous Enzyme (CLOSTRA-T01) or a Cytokine (CLOSTRA-T02) and the Preliminary Validation of CLOSTRA-T01 and CLOSTRA-T02 Clones**

*Materials & Methods*

*Plasmid construction and validation.* The cloning strategy, the primers, the sequences that have been included in the pPME-102 and pPME-103 plasmids, and their use to generate and confirm the correct sequence integration in the CLOSTRA-Derived Strains CLOSTRA-T01 and CLOSTRA-T02 are summarized in the Materials & Methods of Example 1.

*Poly-acrylamide gel electrophoresis (PAGE).* Polyacrylamide gel. Polyacrylamide gel electrophoresis was performed using Invitrogen NuPAGE® buffers and reagents. The protein samples (cell lysates) were mixed with 4x concentrated Invitrogen Loading Buffer mixed with 400 mM dithiothreitol (DTT). Samples were subjected to 5-min denaturation in a heating block set at 100°C and then loaded onto an Invitrogen 4-12% NuPage gel alongside a Precision Plus Protein Standard™ All Blue (Bio-Rad). Protein gels were electrophorized at 150 V for 60 min in 1x MES Invitrogen Buffer. The gel was thoroughly rinsed with dH2O and stained with Coomassie Blue Stain (50 % MeOH, 10 % Acetic Acid, 0.05 % Brilliant Blue R-250) in a shaking tray for 1 hour at room temperature and washed with de-stain solution (50% MeOH, 40% dH$_2$O, 10% Acetic Acid) for 1-3 hours.

*Quantification of nitroreductase enzyme activity* 7-hour cell lysates of CLOSTRA and CLOSTRA-T01 strains were prepared in triplicate using BugBuster reagents (Novagen Milipore, 70584) in accordance with manufacturer's recommendations (Novagen, User Protocol TB245 Rev. F 1108). Menadione reductase activity was determined as described (Knox R et I., 1988). Enzymes belonging to the nitroreductase family are known to possess menadione reductase activity. Nitroreductase catalyzes the reduction of menadione to menadiol by NADPH, and cytochrome C is reduced non-enzymatically by menadiol, thus resulting in the formation of a 'red-pink' color that is quantified at $\lambda$=550 nm. The assay was performed on a spectrophotometer set at 37°C. To the quartz cuvette containing 780 $\mu$l of pre-warmed assay buffer (10 mM Tris-HCl, pH 7.5), the following solutions were added: 10 $\mu$l of 1 mM menadione in DMSO, 100 $\mu$l of 10 mM NADH and 100 $\mu$l of 700 $\mu$M bovine cytochrome C. The cuvette content was mixed immediately, 10 $\mu$l of cell lysates were added and mixed again. The enzymatic reaction was recorded by measuring an increase in the absorbance at $\lambda$=550 nm for 30 s. Units per ml of nitroreductase activity were calculated using the following equation:

$$U/ml = \frac{((\Delta A550/min))/\text{ml}}{14.79}$$

where ($\Delta A550/min$) indicates the increase in A$_{550}$ per minute.

Statistical analyses on the menadione assays were carried out in GraphPad Prism using either one-way or two-way RM ANOVA analysis of variance.

*CTLL-2 cell proliferation assay.* The CTLL-2 indicator cell line (Mouse C57b1/6 T cell) was obtained from ECACC cell culture collection (Cat. No. 93042610). Cells were cultured in RPMI medium with supplemented with10% FBS (fetal bovine serum) and 10% T-STIM (T-Cell Culture Supplement). After 10-14 days of undisturbed growth, the cell line was maintained and incubated under 5% CO$_2$, 37°C. Finally, a cell sample was stained with 0.4% trypan blue to evaluate cellular viability. When satisfactory viability was achieved, the cell culture was harvested by centrifugation (200 x g) and washed three times in RPMI-1640. The cell viability was again evaluated using 0.4% trypan blue staining, and cells were counted in the hemocytometric chamber under the microscope. Finally, the cells were re-suspended in culture medium at a density of 3x10$^5$ cells/ml.

CLOSTRA-T02 and a CLOSTRA (*C. sporogenes* control strains were streaked and inoculated in 10ml fresh BFM media in triplicate. After a 7-hour growth phase, 1 ml samples were obtained from all strains. Samples were spun down and supernatants were filter sterilized using a 0.22$\mu$m filter and a syringe. To perform the assay, 100 $\mu$l of culture medium (RPMI-1640 + 10% FBS) was added to each well of a 96-well plate. Samples and standards were added to 96-well plate, in a 2-fold serial dilution from row 2 to 12, leaving row 1 empty. Recombinant mouse Interleukin-2 was used (#IL031 Sigma) as a standard. Working concentration of a IL-2 standard was prepared (40 ng/ml), and the assay range included 10 ng/ml in row 2 to 0.0097 ng/ml in row 12. 100 $\mu$l of washed CTLL-2 cells (3x10$^5$ cells/ml) was added to each well. Plate was incubated for 48 hours at 5% CO2; 37°C in a humidified incubator. 10 $\mu$l of 5 mg/ml MTT solution was added to each well, and the plate was incubated for a further 4 hours. 50 $\mu$l of Lysing Solution (20% SDS in 50% DMF) was then added to the plate and incubated overnight. The assay plate was spectrophotometrically measured at 570 nm the following day. The final results of the assay were calculated based on the generated mouse IL2 standard curve.

*Western blotting:* The secretion of IL-2 protein in CLOSTRA-T02 was confirmed by Western blotting in DOC-TCA precipitated fractions of culture supernatants using anti-FLAG antibody according to the literature (Schwarz K et al.,2007) and manufacturer's instructions. The levels of IL-2 secreted from CLOSTRA-T02 in 7-hour culture supernatants were determined by cytokine specific ELISA assay (Invitrogen, cat. No. BMS601) according to manufacturer's instructions. The results were recorded in a microplate reader (BMG Labtech SPECTROstar Omega) and calculated based on recombinant IL2 standard.

*Results*

The modular structure of the Clostra Cassette within pPME-100 derived plasmids allows for producing alternative pPM-1nn plasmids (being either pPM-1nnH or pPM-1nnS plasmids) that can be adapted to integrate HetSeq" from human or other genome sources within a CLOSTRA Locus", such that the modifications can be then used as a marker for screening and selecting viable CLOSTRA-T clones (or further derivatives including CLOSTRA-AT, CLOSTRA-ST, or CLOSTRA-SAT) that are correctly modified for any applicable use or manufacturing requirement.

As a first representative CLOSTRA-T model, the DNA coding for a nitroreductase, an enzyme that can activate prodrugs into anti-cancer drugs, was cloned into a pPME-102 plasmid (*i.e.,* a pPM-1nnH plasmid) together with a functional promoter between the LHA and RHA sequences, thereby allowing integration of this sequence module within the CLOSTRA Trehalose operon using the pPM-1nnH plasmid (FIG. 9A). This substitution renders the resulting, positive clones also Trehalose-negative, so that they can be consequently screened and selected, in parallel with the non-clostridial enzymatic sequence and activity that is introduced. A panel of CLOSTRA clones for this CLOSTRA-Derived Strain, referred to herein as CLOSTRA-T01, was obtained and validated at both the DNA (FIG. 9B) and protein (FIG. 9C) level, in addition to confirming the nitroreductase activity that is introduced (FIG. 9D).

As a second representative CLOSTRA-T model, the DNA coding for a cytokine, a protein with immunomodulatory and anti-cancer activities, was cloned into a pPME-103 plasmid, together with a functional promoter between the LHA and RHA sequences, thereby allowing integration of this sequence module within the CLOSTRA Orotate phosphoribosyltransferase gene (FIG. 10A). This substitution renders the resulting, positive clones unable to transfer a ribosyl phosphate group from 5-phosphoribose 1- diphosphate to orotate and to produce orotidine monophosphate (OMP), so that they can be consequently screened and selected, in parallel with the cytokine sequence and activity, being an uracil auxotroph mutant. A panel of clones for the CLOSTRA-Derived Strain referred to as CLOSTRA-T02, was obtained and validated at both the DNA (FIG. 10B) and protein (FIG. 10B) level, in addition to confirming IL2-specific cytokine activity (FIG. 10C) and secretion activity (FIG. 10D).

Thus, the genomes of these exemplary respective CLOSTRA-T strains can be additionally modified by using pPM-1nn-based plasmids (for instance, pPM-1nnH plasmids based on the pPME-100 plasmid format), in which another HetSeq" sequence is cloned, for instance, by using the pPME-101 plasmid so that a protein having clinical and/or industrial use can be advantageously specifically expressed in combination with a non-hemolytic phenotype of CLOSTRA-A1 (generating a CLOSTRA-A1T strain). Alternatively, such CLOSTRA-A1T strain can be also generated by using a pPM-1nnH designed on the basis of either a pPME-102 or pPME-103 plasmid for modifying a CLOSTRA-A strain, in the manner described in Example 1.

**EXAMPLE 3: Preparation of a Clostra Cassette for Generating Exemplary *Clostridium* Strains Combining Non-Haemolytic and Conditional Sporulation properties (CLOSTRA-S1A1) and Preliminary Validation of CLOSTRA-S1A1 Clones**

*Materials & Methods*

*Generation of a CLOSTRA-S1A1 strain.* The lactose inducible promoter, bgaR-bgaL, was synthesised (Thermo Fisher Scientific), amplified using specified oligonucleotides, and ligated alongside a HC (Homologous cassette), to generate pPME-103 plasmid. The CLOSTRA-A1 strain was used as a recipient for the S17-1 *E. Coli* strain carrying the pPME-103 plasmid and introduce the inducible promoter controlling *spo0A* gene. The CRISPR/Cas9 and sequence validations protocols were followed as described in Materials and Methods presented with representative Example 1. This approach can be adapted to generate not only the CLOSTRA-S1 strain (starting from unmodified CLOSTRA) but also the corresponding CLOSTRA-S2 and CLOSTRA-S2A1 in which inducible promoter controls *spoIIAA* gene, using the cloning strategy, the primers, the sequences that are also summarized in the Materials & Methods of Example 1.

*Development of heat-resistant CFU.* Sporulation assays designed to evaluate the development of heat resistant CFU over 5 days were performed as described in previous Examples. Briefly, sporulation cultures (CLOSTRA-S1A1) were grown in triplicate in BFM broth (with and without the addition of 1mM IPTG) for 5 days. A CLOSTRA control strain was incubated in BFM broth without the addition of 1mM IPTG. During the course of the assay, 300 $\mu$l samples were assayed

after 0, 24, 48, 72, 96 and 120 hours of sample incubation. At each of these time-points, a sample was briefly spun down and heat-treated (80°C, 20 minutes). Following incubation, each respective sample was serially diluted from 100 to $10^{-7}$ in PBS; three 20 $\mu$l aliquots of each dilution were spotted onto an BFM agar plate. After 24-48 hours, colonies were observed and counted, and the CFU/ml values determined. The purity and quality of these high-titer spore preparation was confirmed also by light microscopy and according to the literature (Yang WW *et al.,* 2009).

*Results*

Additional evidence on how the modular structure of Clostra Cassette allows producing alternative pPM-1nn (pPM-1nnH and pPM-1nnS) plasmids that can be adapted to integrate HetSeq" derived from human or other genomes within a CLOSTRA Locus" was generated by introducing sequences that convert CLOSTRA-A1 with normal sporulation activity into a CLOSTRA strain having a conditional sporulation phenotype, a property that can be exploited to advantageously modulate and control the use of the resulting CLOSTRA-S1A1 cells and CLOSTRA-S1A1S spores.

Accordingly, a DNA sequence containing a bgaR transcription regulator under the control of a divergent promoter (PbgaR:PbgaL) containing two lactose operators, was cloned into a pPME-104 plasmid between LHA and RHA sequences, which allow substituting endogenous the s*po0A* promoter (Pspo0A) with a sequence that renders *spo0A* expression (and thus sporulation) inducible by a compound such as lactose (FIG. 11A). This system beneficially makes the replication of the resulting, positive clones more controllable, as this feature may be required according to a specific use. A panel of CLOSTRA-A1 clones thus modified by using pPME-104, referred to herein as CLOSTRA-S1A1, was obtained and validated at both the DNA and functional level, in particular by using IPTG as an inducer of sporulation in CLOSTRA-S1A1, which shows sporulation properties comparable to the original CLOSTRA (FIG. 11B). This approach can be applied using alternative cloning strategies where other combinations of CLOSTRA genes are inactivated or deleted in a coordinated, consistent manner in the genome of a CLOSTRA-SA strain, thereby improving their safety and providing an ease of use, while maintaining key biological and replication features needed for manufacturing, administering, or otherwise using CLOSTRA-Derived Products.

This and previous Examples described herein effectively demonstrate how the presently disclosed CLOSTRA and CLOSTRA-Derived Strains and Clostra Cassette can be produced and used separately or alternatively in specifically adapted combinations for both industrial and clinical use. For instance, the CLOSTRA-SA genome herein can be used as a platform for integrating the HetSeq" sequence coding for a protein providing with additional activities of clinical and/or industrial use, such as the proteins that were expressed used by disclosed CLOSTRA-T strains described above. The panels of the CLOSTRA-SA strains where the non-hemolytic phenotype is already combined with a conditional, inducible sporulation feature can be generated, and a corresponding CLOSTRA-SATS spore preparation and a CLOSTRA-SATF formulation can be manufactured and used in a highly controlled manner with respect to the intended objectives, avoiding any undesired biological property otherwise attributable to *Clostridium* strains and respective spores, as commonly available and described in the literature.

As illustrated in FIG. 12, the instant CLOSTRA-S1A1 strain can be modified or otherwise adapted to an industrial or clinical aim through use of either a pPME-102 or a pPME-103 plasmid construct, such that the prodrug-converting enzyme or the cytokine results, respectively, combined with a conditional sporulation phenotype in a corresponding cell culture (with sporulation to be induced, or repressed, for manufacturing or other suitable purposes), spore preparations, and spore formulations. Indeed, whenever an administration of a CLOSTRA-SATF preparation to a subject, including a human subject, is contemplated, the lack of hemolytic activity of such preparations offers the possibility of evaluating alternative administration routes, dosages, and regimens, in particular, when intravenous administration is required or preferred, and/or additional pharmaceutical composition or medical treatments are also co-administered or otherwise part of a combination therapy regimen. Scientific literature provides additional details regarding protocols useful for evaluating the safety, tolerance, any adverse effect relating to CLOSTRA-SATF and other CLOSTRA-Derived Products for treating cancer *e.g.,* by testing pre-clinical models and adapting the design of prior clinical studies involving by genetic engineering bacteria, and in particular the potential of clostridial spores in tumor therapy (Torres *W et al.,* 2018; Kubiak AM and Minton NP, 2015).

The disclosed strategies according to the present invention can be effectively applied to *C. sporogenes* and other *Clostridium* species where the elimination of the hemolytic activity is beneficial. Indeed, other *Clostridium* species not presenting such activity (such as *C. butyricum*) can be exploited as CLOSTRA, whose genome is modified using a pPM-1nnS plasmid to present conditional sporulation under the control of riboswitches, RNA polymerases-inducible and/or sugar inducible system. The resulting CLOSTRA-S strains may be then directly modified using a pPM-1nnH plasmid into CLOSTRA-ST strains to advantageously deliver therapeutic agents after systemic administration and colonization of a tumor or, after oral administration, locally in the gastrointestinal tract. The administration of the CLOSTRA-Derived Products, in particular those allowing an in vivo expression and administration of enzymes, cytokines, and other protein-based therapeutic products (*e.g.,* IL2 and yfkO nitroreductase shown in FIG. 12), with or without adding other feature related to auxotrophic features consequent to the use of pPM-1nnH plasmids for constructing the CLOSTRA-Derived Strains that delete or inactivate metabolic or enzymatic activities, for example, PyrE or Trehalose operon).

**EXAMPLE 4: Preparation of Clostra Cassette for Generating Exemplary *Clostridium* Strains Combining Non-Haemolytic, uracil auxotrophic and Conditional Sporulation properties (CLOSTRA-S3A2 and CLOSTRA-S4A2) and Preliminary Validation of CLOSTRA-S2A2 and CLOSTRA-S3A2 Clones**

*Materials & Methods*

*Generation and validation of CLOSTRA-A2, CLOSTRA-S3A2, and CLOSTRA-S4A2 strains.* The CRISPR/Cas9 and sequence validations protocols were followed as described in Materials and Methods presented with representative Example 1. CLOSTRA-A1 has been modified with pPME-105 plasmid to create a strain which is auxotrophic for uracil. The 576 bp, corresponding to orotate phosphoribosyltransferase PyrE gene together 33 bp of upstream region have been amplified using specified oligonucleotides, and ligated alongside in F3 element to generate pPME-105. The CLOSTRA-A1 strain was used as a recipient for the S17-1 strain carrying the pPME-105 vector have been replaced with BM2 sequence to generate CLOSTRA-A2 strain and that can be used for the unique identification and detection of CLOSTRA-A2 strain and any further derived strain. CLOSTRA-S2A2 and CLOSTRA-S3A2 were cultivated with or without the addition of 2mM lactose and 5mM theophylline. CLOSTRA-A2 was supplemented only in defined medium with 20ul/ml of uracil solution. Defined Clostridium growth media has been prepared as stated in the literature (Lovitt RW *et al.,* 1987.). Agar plates with and without uracil supplementation (at the final concentration of 20 $\mu$g/ml) were pre-reduced in anaerobic cabinet. Strains: CLOSTRA-A1 and CLOSTRA-A2 were streaked and incubated for 48 hours under anaerobic conditions for the detection of growth.

*Results*

The modular structure of Clostra Cassette in the series of pPME-100 derived plasmids can be exploited for combining the deletion, substitution, and/or addition of sequences within Locus" of CLOSTRA-Derived Strains according to specific requirements. For instance, the pPME-103 plasmid can be modified by substituting the mIL2 sequence used as HetSeq$^n$ with a marker sequence (or a sequence that can facilitate later modification) cloned between the LHA and RHA sequences used for targeting *PyrE* gene in CLOSTRA. The resulting pPM-1nnH (pPME-105 plasmid) can b used to modify CLOSTRA-A1, and the consequently obtained CLOSTRA-Derived Strain (CLOSTRA-A2) can be used as a platform for introducing any further HetSeq$^n$ from a further pPM-1nnH and/or pPM-1nnS plasmids (FIG. 13A). The construction of this CLOSTRA-A2 has been pursued and validated both at the DNA (FIG. 13B) and at a biological, functional (FIG. 13C) level, confirming that the CLOSTRA-Derived Strain is, in addition of being a non-haematolytic mutant, an uracil auxotrophic mutant as well.

Another exemplified approach for obtaining a tighter regulation of sporulation in CLOSTRA-Derived Strains is based on the integration of a riboswitch based on *Theophylline* into the inducible promoter system previously described and cloned as HetSeq" in the pPME-104 plasmid (FIG. 14A). This sequence can be used to generate two derivatives of pPME-104 plasmids: pPME-106 plasmid triggers the integration of the improved sporulation regulatory sequence for controlling *Spo0A* expression, while the pPME-107 plasmid triggers such integration for controlling another important sporulation gene, *SpoIIAA.* When these two plasmids are used to modify CLOSTRA-A2, the resulting CLOSTRA-S3A2 (FIG. 14B) and CLOSTRA-S4A2 (FIG. 14C) advantageously combine selection and sporulation control features before introducing any further HetSeq", for instance, those encoding heterologous proteins such as antibodies, antigens, cytokines, enzymes, or growth factors. The exemplary CLOSTRA-S3A2 and CLOSTRA-S4A2 were generated and validated at the level of correct DNA integration (FIG. 15A) and control of sporulation that appears particularly well repressed in absence of the combined induction by lactose and theophylline (FIG. 15B). Indeed, an auxotrophic feature such as the one in CLOSTRA-S3A2 and CLOSTRA-S4A2, when combined to the expression of a therapeutic (e.g. anti-cancer) protein in further modified CLOSTRA-S3A2T and CLOSTRA-S4A2T strains provide CLOSTRA-Derived Strains and CLOSTRA-Derived Products that can be administered in tissues where the auxotrophic feature is favorable for localized, *in vivo* proliferation of CLOSTRA-Derived Strains without colonizing or dispersing spores in other tissues. In the case of the loss or substitution of the *PyrE* gene, the spores derived from CLOSTRA-S3A2T and CLOSTRA-S4A2T that are administered by intratumoral or other appropriate injection, not only can be specifically driven and proliferate into tumor masses following exposure to a hypoxic condition (without further sporulation events), but also provide an advantage of a much higher uracil concentration in these locations compared to the uracil concentration present in other tissues.

Exemplary approaches for exploiting the features of CLOSTRA-A2 for generating CLOSTRA-Derived Strains expressing one or more therapeutic proteins (with or without control of the sporulation process) can be exemplified through the integration of a IL2 (Interleukin 2) gene into a further Locus$^n$ such as the Trehalose operon, using the pPME-108 plasmid, which is a derivative of pPME-102 (FIG. 16A). Alternatively, the CLOSTRA-A2 may be modified by introducing IL2 within the already modified Locus" using the pPME-109 and pPME-110 plasmids, with (FIG. 16B) or without (FIG. 16C) the control of the sporulation process by making use of genes *spo0A* or *spoIIAA.*

**REFERENCES**

Agrawal S and Kandimalla E, 2019. Immuno-Oncol Tech. 3: 15-23.
Al-Hinai M et al., 2015. Microbiol Mol Biol Rev. 79:19-37.
Alexaki A., et al., 2019. J Mol Biol, 431:2434-2441
Atmadjaja A et al. 2019. FEMS Microbiol Lett. 366:fnz059.
Banjeree A et al., 2014. Appl Environ Microbiol. 80:2410-2416.
Bannam TL et al., 1995. Mol Microbiol. 16:535-51.
Bruder M et al., 2016. Appl Environ Microbiol. 82:6109-6119.
Canadas I et al., 2019. ACS Synth Biol. 8:1379-1390.
Collins ME et al., 1985. J Gen Microbiol. 131:2097-105.
Cooksley CM et al., 2010. Appl Environ Microbiol. 76:4448-60.
Corraliza-Gorjón I et al., 2017. Front Immunol. 8:1804.
Cruz-Morales P et al., 2019. Genome Biol. Evol. 11(7):2035.
Cui W et al., 2016. Microb Cell Fact. 15:199.
Dembek M et al., 2017. Front Microbiol. 8:1793.
Diallo M et al., 2019. Methods. S1046-2023: 30090-8.
Dong H et al., 2012. Metab Engin. 14:59-67.
Ehsaan M et al, 2016. Biotechnol Biofuel. 9:4.
Eleneen Y and Colen RR, 2017. Adv Exp Med Biol. 995:141-153.
Emptage CD et al. 2009. Biochem Pharmacol. 77(1):21-9.
Foulquier C et al., 2019. Biotechnol Biofuel. 12:31.
Gao W et al., 2004. Biotechnol Prog. 20:443-8.
Groot AJ et al., 2007. Biochem Biophys Res Commun. 364:985-9.
Grote A et al., 2005. Nucleic Acids Res. 33:W526-31.
Hamada Y, 2017. Bioorg Med Chem Lett. 27:1627-1632.
Hartman AH et al., 2011. Appl Environ Microbiol. 77:471-8.
Heap J et al., 2009. J Microbiol Methods. 78:79-85.
Heap J et al., 2014. Oncotarget. 5:1761-9.
Huang H et al., 2016. ACS Synth. Biol. 12 :1355-1361
Ingle P et al., 2019. Sci Rep. 9:8123.
Jinek M et al., 2012. Science. 2012 337: 816-21.
Joseph R et al., 2018. Front. Microbiol. 9:154.
Knox R et I., 1988. Biochem Pharmacol. 37:4671-7.
Kubiak AM and Minton NP, 2015. Res Microbiol. 66:244-54.
Kubiak AM et al., 2015. Genome Announc. 3:e00942-15.
Kwon SW et al., 2020. Front Bioeng Biotechnol. 8:282.
Lovitt RW et al., 1987. J Appl Bacteriol. 62:81-92.
McAllister KN, and Sorg JA, 2019. J Bacteriol. 201:e00219-19.
Minton N and Morris J ,1981. J Gen Microbiol. 127:325-331.
Minton N et al., 2016. Anaerobe 41:104-112.
Mowday A et al. 2016. Cancers. 8:63.
Nariya H et al., 2011. Appl Environ Microbiol. 77:8439-41.
Ni G et al., 2019. Biomed Res Int. 2019:1395138.
Nora L et al., 2019. Biotechnol Adv. 3:107433.
Purdy D et al., 2002. Mol Microbiol. 46:439-52.
Pyne M et al., 2016. Sci Rep. 6:25666.
Rautio J et al., 2018. Nat Rev Drug Discov. 17:559-587.
Rossi S et al., 2017.Eur J Nucl Med Mol Imaging. 44: 2310-2325.
Schwarz K et al.,2007. J Microbiol Methods. 68:396-402.
Shen S et al., 2019 Microbiol Spectr. 7:10.1128/microbiolspec.GPP3-0017-2018.
Subbiah V et al., 2017. Diagnostics. 7: E10.
Tanner R et al., 1981. Current Microbiol. 5 :35-38.
Theys J et al., 2006. British Journal of Cancer. 95:1212-1219.

Topp S et al., 2010. Appl Environ Microbiol. 76:7881-4.
Torres W et al., 2018. J Cancer Metastasis Treat. 4:4.
Totten PA et al., 1995. Infect Immun. 63:4409-16.
Wang S et al., 2018. Clin Microbiol Infec. 24:1095-1099.
Wasels F et al., 2017. J Microbiol Methods. 140:5-11.
Wen Z et al., 2017. Metab Eng. 39:38-48.
Williams EM et al., 2015. Biochem J. 471:131-53.
Xin Y et al., 2019. Nat Rev Drug Discov. 18: 899-90.
Yang WW et al., 2009. J Appl Microbiol. 106:27-33.
Zhang J et al., 2015. Biotechnol Biofuels. 8:36.
Zhang YL et al. 2014. Lett Appl Microbiol. 59:580-6.
Zhong S et al., 2019. Transl Oncol. 13:57-69.

## Disclosed Items

1. A genetically modified *Clostridium* strain presenting an inducible or repressible sporulation phenotype, and one or more additional genome modifications for expressing a heterologous sequence and/or to inactivate a second *Clostridium* gene.

2. The genetically modified *Clostridium* strain according to item 1, wherein the inducible or repressible sporulation phenotype results from deletion, substitution, or other genetic modification of the sequence that controls the expression of one or more *Clostridium* genes involved in *Clostridium* strain sporulation.

3. The genetically modified *Clostridium* strain according to item 2, wherein the genetic modification is the insertion of an inducible or repressible promoter.

4. The genetically modified *Clostridium* strain according to any of the preceding items, wherein the *Clostridium* gene involved in *Clostridium* strain sporulation is selected from *Spo0A, SpoIIE, SpoIIAA, and SpoIIAB*.

4. The genetically modified *Clostridium* strain according to any of the preceding items, wherein the *Clostridium* strain is derived from the group of *Clostridium* species comprising *C. butyricum, C. sporogenes,* and *C. novyi.*

5. The genetically modified *Clostridium* strain according to any of the preceding items, wherein the *Clostridium* strain expresses a heterologous gene encoding at least one protein selected from enzymes, antibodies, antigens, toxins, and cytokines.

6. The genetically modified *Clostridium* strain according to item 6, wherein the *Clostridium* strain expresses a heterologous gene encoding a prodrug converting enzyme, an antibody directed to a cancer antigen, and/or a cytokine.

7. The genetically modified *Clostridium* strain according to any of the preceding items wherein the *Clostridium* strain is modified to inactivate or attenuate at least one *Clostridium* gene in haemolysis.

8. The genetically modified *Clostridium* strain according to any of the preceding items, wherein the *Clostridium* strain is modified in order to inactivate or attenuate at least one *Clostridium* gene in metabolism.

9. The genetically modified *Clostridium* strain according to item 10, wherein the *Clostridium* strain is a uracil auxotroph mutant.

10. *Clostridium* spores obtained from a genetically modified *Clostridium* strain according to any of the preceding items.

11. The *Clostridium* spore according to item 10, wherein the spores are produced in the presence of a compound inducing or repressing the genetically modified sequence that controls the expression of one or more *Clostridium* genes involved in sporulation.

12. A composition comprising cells from the genetically modified *Clostridium* strain according to any of items 1-10 or from the spores of the genetically modified *Clostridium* strain according to item 11 or 12.

13. The composition according to item 12, wherein the composition is for use as a medicament, and optionally comprising an additive, carrier, vehicle, diluent, salts, and/or excipient.

15. The composition according to any of items 12 to 14, wherein the composition is a liquid suspension or lyophilized preparation.

16. The composition according to any of items 12 to 15, wherein the composition is for use in the treatment of cancer, and optionally comprising an additional compound having pharmacological or drug-like properties.

17. The composition according to any of items 12 to 16, wherein the composition is formulated for systemic, intratumoral, or oral administration.

18. A method for treating cancer comprising administering a composition according to any of items 12 to 17 to a subject in need thereof.

19. The method according to item 18, wherein the additional compound is administered separately, simultaneously, or sequentially with the composition.

20. The method according to item 18, wherein the additional compound is a prodrug.

**Claims**

1. A genetically modified Clostridium strain derived from Clostridium butyricum or Clostridium sporogenes, exhibiting an inducible sporulation phenotype and one or more additional genomic modifications for inactivating at least one Clostridium gene in hemolysis, said inducible sporulation phenotype being caused by deletion or substitution of sequences controlling the expression of one or more Clostridium genes involved in sporulation of the Clostridium strain selected from Spo0A and SpoIIAB wherein the Clostridium strain is further modified to inactivate or weaken at least one Clostridium gene in its metabolism for establishing auxtrophy, wherein the Clostridium strain expresses a heterologous gene encoding at least one protein selected from enzymes, antibodies, antigens, toxins, and cytokines and contains a signal sequence for the secretion of a corresponding expressed protein in said genetically modified Clostridium strain, and wherein the genetic modification controlling the expression of one or more Clostridium genes involved in Clostridium spore formation selected from Spo0A and SpoIIAB, is the insertion of an inducible promoter that is induced or repressed by exposing the genetically modified Clostridium strain to a sugar, gas, or chemical substance, a specific temperature or pH range.

2. The genetically modified Clostridium strain according to claim 1, wherein the inducible promoter for controlling the expression of one or more Clostridium genes involved in sporulation of the Clostridium strain is a lactose-dependent promoter or a theophylline-dependent promoter or a combination thereof, with or without a riboswitch.

3. The genetically modified Clostridium strain according to any of the preceding claims, wherein the heterologous gene is expressed under the control of a promoter selected from Thl-S promoter, Thl13 promoter, Thl14 promoter, or a Ferredoxin promoter.

4. The genetically modified Clostridium strain described in any of the preceding claims, wherein the heterologous gene is inserted in the pyrE gene, trehalose operon or Sag operon .

5. The genetically modified Clostridium strain according to any of the preceding claims, wherein a tag sequence is inserted in the pyrE gene, trehalose operon or Sag operon.

6. The genetically modified Clostridium strain according to any of the preceding claims, wherein the Clostridium strain expresses a heterologous gene encoding an antigen, wherein the antigen is a non-human antigen, preferably an antigen derived from a viral or bacterial protein.

7. The genetically modified Clostridium strain according to any one of the preceding claims, wherein the codons of the heterologous gene are optimized for the transcription and translation in Clostridium strains.

8. The genetically modified Clostridium strain according to any of the preceding claims, wherein the Clostridium strain is modified to inactivate or attenuate at least one Clostridium gene in hemolysis, wherein the Sag operon is deleted.

9. The genetically modified Clostridium strain according to any one of the preceding claims, wherein the Clostridium strain is a uracil auxotrophic mutant due to the deletion of pyrE gene.

10. Clostridium spores obtained from a genetically modified Clostridium strain according to any one of the preceding claims, wherein the spores are produced in the presence of a compound that induces or inhibits a gene-modifying sequence that controls the expression of one or more Clostridium genes involved in spore formation, the compound preferably being a sugar, gas, chemical substance, and/or exposure to specific conditions, preferably a specific temperature or pH range.

11. A composition comprising cells from a genetically modified Clostridium strain according to any one of claims 1-9 or spores of a genetically modified Clostridium strain according to claim 10.

12. The composition according to claim 11, wherein the composition is used as a pharmaceutical and optionally comprises an additive, a carrier, a medium, a diluent, a salt and/or an excipient.

13. The composition of claim 11 or 12, wherein the composition is a liquid suspension or a lyophilized formulation.

14. The composition according to any one of claim 11-13, wherein the composition is formulated for oral administration.

15. The composition according to any one of claim 11-14, wherein the composition is used as a vaccine or for human immunization against viral or bacterial antigens.

## FIG. 1

Selecting an appropriate clostridium strain (CLOSTRA)

Modifying CLOSTRA by modifying and/or deleting genetic element(s) controlling sporulation gene(s) (obtaining CLOSTRA-S)

Modifying CLOSTRA by including and/or deleting genetic element(s) functional for medical or industrial uses (obtaining CLOSTRA-T, CLOSTRA-A, or CLOSTRA-AT)

Culturing, selecting, and optimizing CLOSTRA that combine genetic and functional features that have been modified in CLOSTRA-T, CLOSTRA-A, or CLOSTRA-AT with those that have been modified in CLOSTRA-S (obtaining CLOSTRA-ST, CLOSTRA-SA, or CLOSTRA-SAT)

Producing corresponding spore preparations (CLOSTRA-STS, CLOSTRA-SAS, OR CLOSTRA-SATS)

Formulating corresponding spore (or cell) preparations for industrial or medical uses (CLOSTRA-STF, CLOSTRA-SAF, OR CLOSTRA-SATF)

# FIG. 2

## FIG. 3A

## FIG. 3B

## FIG. 4A

## FIG. 4B

# FIG. 5A

pPME-100

# FIG. 5B

pPME-101 (Clostra cassette)

CLOSTRA-A1 genome

**FIG. 6A**

**FIG. 6B**

**FIG. 7A**

**FIG. 7B**

**FIG. 8A**

Step 1     Step 2     Administration     Step 3     Step 4

**FIG. 8B**

## FIG. 9A

pPME-102 (Clostra cassette)

CLOSTRA-T01 genome

## FIG. 9B

## FIG. 9C

## FIG. 9D

## FIG. 10A

## FIG. 10B

## FIG.10C

## FIG. 10D

## FIG. 10E

## FIG. 11A

## FIG. 11B

# FIG. 12

# FIG. 13A

pPME-105 (Clostra cassette)

CLOSTRA-A2 genome

# FIG. 13B

# FIG. 13C

## FIG. 14A

## FIG. 14B

## FIG. 14C

## FIG. 15A

## FIG. 15B

CLOSTRA-S3A2 [lactose 2mM + theophylline 5mM]
CLOSTRA-S4A2 [lactose 2mM + theophylline 5mM]
CLOSTRA-S3A2
CLOSTRA-S4A2
CLOSTRA

FIG. 16A

FIG. 16B

FIG. 16C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003074681 A
- WO 2017064439 A
- WO 2010005722 A
- WO 2015075475 A
- WO 2017190257 A
- WO 2007149433 A
- WO 2016095503 A
- WO 2009111177 A
- WO 2003045153 A
- WO 2013159155 A

### Non-patent literature cited in the description

- **AGRAWAL S** ; **KANDIMALLA E**. *Immuno-Oncol Tech.*, 2019, vol. 3, 15-23
- **AL-HINAI M et al.** *Microbiol Mol Biol Rev.*, 2015, vol. 79, 19-37
- **ALEXAKI A. et al.** *J Mol Biol*, 2019, vol. 431, 2434-2441
- **ATMADJAJA A et al.** *FEMS Microbiol Lett.*, 2019, vol. 366, fnz059
- **BANJEREE A et al.** *Appl Environ Microbiol.*, 2014, vol. 80, 2410-2416
- **BANNAM TL et al.** *Mol Microbiol.*, 1995, vol. 16, 535-51
- **BRUDER M et al.** *Appl Environ Microbiol.*, 2016, vol. 82, 6109-6119
- **CANADAS I et al.** *ACS Synth Biol.*, 2019, vol. 8, 1379-1390
- **COLLINS ME et al.** *J Gen Microbiol.*, 1985, vol. 131, 2097-105
- **COOKSLEY CM et al.** *Appl Environ Microbiol.*, 2010, vol. 76, 4448-60
- **CORRALIZA-GORJÓN I et al.** *Front Immunol.*, 2017, vol. 8, 1804
- **CRUZ-MORALES P et al.** *Genome Biol. Evol.*, 2019, vol. 11 (7), 2035
- **CUI W et al.** *Microb Cell Fact.*, 2016, vol. 15, 199
- **DEMBEK M et al.** *Front Microbiol.*, 2017, vol. 8, 1793
- **DIALLO M et al.** *Methods*, 2019, vol. S1046-2023, 30090-8
- **DONG H et al.** *Metab Engin.*, 2012, vol. 14, 59-67
- **EHSAAN M et al.** *Biotechnol Biofuel.*, 2016, vol. 9, 4
- **ELENEEN Y** ; **COLEN RR**. *Adv Exp Med Biol.*, 2017, vol. 995, 141-153
- **EMPTAGE CD et al.** *Biochem Pharmacol.*, 2009, vol. 77 (1), 21-9
- **FOULQUIER C et al.** *Biotechnol Biofuel.*, 2019, vol. 12, 31
- **GAO W et al.** *Biotechnol Prog.*, 2004, vol. 20, 443-8
- **GROOT AJ et al.** *Biochem Biophys Res Commun.*, 2007, vol. 364, 985-9
- **GROTE A et al.** *Nucleic Acids Res.*, 2005, vol. 33, W526-31
- **HAMADA Y**. *Bioorg Med Chem Lett.*, 2017, vol. 27, 1627-1632
- **HARTMAN AH et al.** *Appl Environ Microbiol.*, 2011, vol. 77, 471-8
- **HEAP J et al.** *J Microbiol Methods.*, 2009, vol. 78, 79-85
- **HEAP J et al.** *Oncotarget.*, 2014, vol. 5, 1761-9
- **HUANG H et al.** *ACS Synth. Biol.*, 2016, vol. 12, 1355-1361
- **INGLE P et al.** *Sci Rep.*, 2019, vol. 9, 8123
- **JINEK M et al.** *Science*, 2012, vol. 337, 816-21
- **JOSEPH R et al.** *Front. Microbiol.*, 2018, vol. 9, 154
- **KNOX R**. *Biochem Pharmacol.*, 1988, vol. 37, 4671-7
- **KUBIAK AM** ; **MINTON NP**. *Res Microbiol.*, 2015, vol. 66, 244-54
- **KUBIAK AM et al.** *Genome Announc.*, 2015, vol. 3, e00942-15
- **KWON SW et al.** *Front Bioeng Biotechnol.*, 2020, vol. 8, 282
- **LOVITT RW et al.** *J Appl Bacteriol.*, 1987, vol. 62, 81-92
- **MCALLISTER KN** ; **SORG JA**. *J Bacteriol.*, 2019, vol. 201, e00219-19
- **MINTON N** ; **MORRIS J**. *J Gen Microbiol.*, 1981, vol. 127, 325-331
- **MINTON N et al.** *Anaerobe*, 2016, vol. 41, 104-112
- **MOWDAY A et al.** *Cancers*, 2016, vol. 8, 63
- **NARIYA H et al.** *Appl Environ Microbiol.*, 2011, vol. 77, 8439-41

- **NI G et al.** *Biomed Res Int.*, 2019, vol. 2019, 1395138
- **NORA L et al.** *Biotechnol Adv.*, 2019, vol. 3, 107433
- **PURDY D et al.** *Mol Microbiol.*, 2002, vol. 46, 439-52
- **PYNE M et al.** *Sci Rep.*, 2016, vol. 6, 25666
- **RAUTIO J et al.** *Nat Rev Drug Discov.*, 2018, vol. 17, 559-587
- **ROSSI S et al.** *Eur J Nucl Med Mol Imaging.*, 2017, vol. 44, 2310-2325
- **SCHWARZ K et al.** *J Microbiol Methods.*, 2007, vol. 68, 396-402
- **SHEN S et al.** *Microbiol Spectr.*, 2019, vol. 7
- **SUBBIAH V et al.** *Diagnostics*, 2017, vol. 7, E10
- **TANNER R et al.** *Current Microbiol.*, 1981, vol. 5, 35-38
- **THEYS J et al.** *British Journal of Cancer.*, 2006, vol. 95, 1212-1219
- **TOPP S et al.** *Appl Environ Microbiol.*, 2010, vol. 76, 7881-4
- **TORRES W et al.** *J Cancer Metastasis Treat.*, 2018, vol. 4, 4
- **TOTTEN PA et al.** *Infect Immun.*, 1995, vol. 63, 4409-16
- **WANG S et al.** *Clin Microbiol Infec.*, 2018, vol. 24, 1095-1099
- **WASELS F et al.** *J Microbiol Methods.*, 2017, vol. 140, 5-11
- **WEN Z et al.** *Metab Eng.*, 2017, vol. 39, 38-48
- **WILLIAMS EM et al.** *Biochem J.*, 2015, vol. 471, 131-53
- **XIN Y et al.** *Nat Rev Drug Discov.*, 2019, vol. 18, 899-90
- **YANG WW et al.** *J Appl Microbiol.*, 2009, vol. 106, 27-33
- **ZHANG J et al.** *Biotechnol Biofuels*, 2015, vol. 8, 36
- **ZHANG YL et al.** *Lett Appl Microbiol.*, 2014, vol. 59, 580-6
- **ZHONG S et al.** *Transl Oncol.*, 2019, vol. 13, 57-69